# EUROPEAN PATENT SPECIFICATION

(11) **EP 1 954 699 B1**
(45) Date of publication and mention of the grant of the patent: **19.09.2012**
(21) Application number: 06808609.9
(22) Date of filing: 20.11.2006
(51) Int. Cl.: C07D 475/06, C07D 475/08, C07D 487/04, A61K 31/495, A61P 35/00

(54) **PYRIDO-, PYRAZO- AND PYRIMIDOPYRIMIDINE DERIVATIVES AS mTOR INHIBITORS**
PYRIDO-, PYRAZO- UND PYRIMIDOPYRIMIDINDERIVATE ALS mTOR-INHIBITOREN
DÉRIVÉS DE PYRIDO-, PYRAZO- ET PYRIMIDOPYRIMIDINE EN TANT QU'INHIBITEURS DE mTOR

(30) Priority: 22.11.2005 US 738902 P; 25.11.2005 GB 0524047; 23.08.2006 US 823308 P; 23.08.2006 US 823309 P
(43) Date of publication of application: 13.08.2008
(73) Proprietor: Kudos Pharmaceuticals Ltd, London W1K ILN (GB)
(72) Inventor: HUMMERSONE, Marc, Geoffrey, Horsham Sussex RH13 5PX (GB); GOMEZ, Sylvie, Horsham Sussex RH13 5PX (GB); MENEAR, Keith, Allan, Horsham Sussex RH13 5PX (GB); SMITH, Graeme, Cameron, Murray, Cambridge Cambridgeshire CB4 0WG (GB); MALAGU, Karine, Horsham Sussex RH13 5PX (GB); DUGGAN, Heather, Mary, Ellen, Horsham Sussex RH13 5PX (GB); COCKCROFT, Xiao-Ling Fan, Horsham Sussex RH13 5PX (GB); HERMANN, Gesine, Johanna, Horsham Sussex RH13 5PX (GB)
(74) Representative: Gairns, Raymond Stevenson
(86) International application number: PCT/GB2006/004327
(87) International publication number: WO 2007/060404

(56) References cited:
- EP-A- 0 185 259
- WO-A-2006/069805
- NISHIKAWA ET AL.: "Structure-Activity Relationships of the DiureticActivity of Triaza- and Tetraazanaphthalene Compounds" CHEM. PHARM. BULL., vol. 24, no. 9, 1976, pages 2057-2077, XP008073154

## Description

The present invention relates to compounds, pyrido-, pyrazo- and pyrimido-pyrimidine derivatives, which act as mTOR inhibitors.

### Background

Growth factor/mitogenic activation of the phosphatidylinositol 3-kinase (PI3K)/AKT signalling pathway ultimately leads to the key cell cycle and growth control regulator mTOR, the mammalian target of rapamycin (alternatively referred to as FRAP (FKBP12 and rapamycin associated protein), RAFT1 (Rapamycin and FKBP12 target 1), RAPT1 (rapamycin target 1) - all derived from the interaction with the FK-506-binding protein FKBP12, and SEP (sirolimus effector protein)). mTOR is a mammalian serine/threonine kinase of approximately 289 kDa in size and a member of the evolutionary conserved eukaryotic TOR kinases (refs. 1-4). The mTOR protein is a member of the PI3-kinase like kinase (PIKK) family of proteins due to its C-terminal homology (catalytic domain) with PI3-kinase and the other family members, e.g. DNA-PKcs (DNA dependent protein kinase), ATM (Ataxia-telangiectasia mutated). In addition to a catalytic domain in the C-terminus, mTOR contains a FKBP12/rapamycin complex binding domain (FRB). At the N-terminus up to 20 HEAT (Huntingtin, EF3, alpha regulatory subunit of PP2A and TOR) motifs are found whilst more C-terminal is a FAT (FRAP-ATM-TRRAP) domain, and at the extreme C-terminus of the protein an additional FAT domain is found (FAT-C) (refs. 5,6).

TOR has been identified as a central regulator of both cell growth (size) and proliferation, which is in part governed by translation initiation. TOR dependant phosphorylation of S6-kinase (S6K1) allows translation of ribosomal proteins involved in cell cycle progression (refs. 7-9). Cap-dependant translation is regulated by the phosphorylation of the eukaryotic translation initiation factor 4E (eIF4E)-binding protein 1 (4E-BP (PHAS-1)). This modification prevents PHAS-1 binding eIF4E, thereby permitting formation of an active eIF4F translation complex (reviewed in refs. 10,11,12). Activation of these signalling elements is dependant on insulin, other growth factors and nutrients suggesting a gatekeeper role for mTOR in the control of cell cycle progression only under favourable environmental conditions. The PI3K/AKT signalling cascade lies upstream of mTOR and this has been shown to be deregulated in certain cancers and results in growth factor independent activation in, for example, PTEN deficient cells. mTOR lies at the axis of control for this pathway and inhibitors of this kinase (e.g. sirolimus (rapamycin or Rapamune^{™}) and everolimus (RAD001 or Certican^{™})) are already approved for immunosuppression and drug eluting stents (reviewed in refs. 13, 14), and are now receiving particular interest as novel agents for cancer treatment.

Tumour cell growth arises from the deregulation of normal growth control mechanisms such as the loss of tumour suppressor function(s). One such tumour suppressor is the phosphatase and tensin homologue deleted from chromosome ten (PTEN). This gene, also known as mutated in multiple advanced cancers (MMAC), has been shown to play a significant role in cell cycle arrest and is the most highly mutated tumour suppressor after p53. Up to 30% of glioblastoma, endometrial and prostate cancers have somatic mutations or deletions of this locus (refs. 15,16).

PI3K converts phosphatidylinositol 4,5, bisphosphate (PIP2) to phosphatidylinositol 3,4,5, triphosphate (PIP3) whilst PTEN is responsible for removing the 3' phosphate from PIP3 producing PIP2. PI3-K and PTEN act to maintain an appropriate level of PIP3 which recruits and thus activates AKT (also known as PKB) and the downstream signalling cascade that is then initiated. In the absence of PTEN, there is inappropriate regulation of this cascade, AKT becomes effectively constitutively activated and cell growth is deregulated. An alternative mechanism for the deregulation of this cell signalling process is the recent identification of a mutant form of the PI3K isoform, p110alpha (ref. 17). The apparent increased activity of this mutant is thought to result in increased PIP3 production, presumably in excess of that which the function of PTEN can counteract. Increased signalling from PI3K, thus results in increased signalling to mTOR and consequently, its downstream activators.

In addition to the evidence linking mTOR with cell cycle regulation (from G1 to S-phase) and that inhibition of mTOR results in inhibition of these regulatory events it has been shown that down regulation of mTOR activity results in cell growth inhibition (Reviewed in refs. 7,18,19). The known inhibitor of mTOR, rapamycin, potently inhibits proliferation or growth of cells derived from a range of tissue types such as smooth muscle, T-cells as well as cells derived from a diverse range of tumour types including rhabdomyosarcoma, neuroblastoma, glioblastoma and medulloblastoma, small cell lung cancer, osteosarcoma, pancreatic carcinoma and breast and prostate carcinoma (reviewed in ref. 20). Rapamycin has been approved and is in clinical use as an immunosuppressant, its prevention of organ rejection being successful and with fewer side effects than previous therapies (refs. 20, 21). Inhibition of mTOR by rapamycin and its analogues (RAD001, CCI-779) is brought about by the prior interaction of the drug with the FK506 binding protein, FKBP12. Subsequently, the complex of FKBP12/rapamycin then binds to the FRB domain of mTOR and inhibits the downstream signalling from mTOR.

The potent but non-specific inhibitors of PI3K, LY294002 and wortmannin, also have been shown to inhibit the kinase function of mTOR but act through targeting the catalytic domain of the protein (ref. 21). Further to the inhibition of mTOR function by small molecules targeted to the kinase domain, it has been demonstrated that kinase dead mTOR cannot transmit the upstream activating signals to the downstream effectors of mTOR, PHAS-1 or p70S6 kinase (ref. 22). It is also shown that not all functions of mTOR are rapamycin sensitive and this may be related to the observation that rapamycin alters the substrate profile of mTOR rather than inhibiting its activity *per se* (ref. 23). Analysis of the interactions of mTOR with other cellular factors has revealed that in addition to the mTOR-Raptor complex, there is also an mTOR-Rictor complex representing a rapamycin insensitive activity of mTOR (B) (Sarbassov et al. Current Biology, 2004, 14, 1296-1302). This activity likely accounts for the discrepancy between kinase dead mTOR and the alteration of mTOR signalling by rapamycin and its derivatives. The discrepancy also identifies the possibility of a therapeutic advantage in inhibiting directly the catalytic activity of mTOR It has been suggested that a catalytic inhibitor of mTOR may be a more effective antagonist of cancer cell proliferation and survival and that rapamycin may be more useful in combination with agents that can compensate for its failure to completely disrupt pathway signalling (Choo and Blenis, Cancer Cell, 2006, 9, 77-79; Hay, Cancer Cell, 2005, 8, 179-183). Therefore, it is proposed that a kinase domain directed inhibitor of mTOR may be a more effective inhibitor of mTOR.

In addition to rapamycin's ability to induce growth inhibition (cytostasis) in its own right, rapamycin and its derivatives have been shown to potentiate the cytotoxicity of a number of chemotherapies including cisplatin, camptothecin and doxorubicin (reviewed in ref. 20). Potentiation of ionising radiation induced cell killing has also been observed following inhibition of mTOR (ref. 24). Experimental and clinical evidence has shown that rapamycin analogues are showing evidence of efficacy in treating cancer, either alone or in combination with other therapies (see refs. 10, 18, 20). These findings suggest that pharmacological inhibitors of mTOR kinase should be of therapeutic value for treatment of the various forms of cancer comprising solid tumours such as carcinomas and sarcomas and the leukaemias and lymphoid malignancies. In particular, inhibitors of mTOR kinase should be of therapeutic value for treatment of, for example, cancer of the breast, colorectum, lung (including small cell lung cancer, non-small cell lung cancer and bronchioalveolar cancer) and prostate, and of cancer of the bile duct, bone, bladder, head and neck, kidney, liver, gastrointestinal tissue, oesophagus, ovary, pancreas, skin, testes, thyroid, uterus, cervix and vulva, and of leukaemias (including ALL and CML), multiple myeloma and lymphomas.

Renal cell carcinoma in particular, has been identified as sensitive to the rapamycin derivative CCI-779, resulting from a loss of VHL expression (Thomas et al. Nature Medicine, 2006, 12, 122-127). Tumours that have lost the promyelocytic leukaemia (PML) tumour suppressor, have also been shown to be sensitive to inhibition of mTOR by rapamycin as a consequence of disruption of the regulation of the mTOR signalling pathway (Bemadi, Nature, 2006, 442, 779-785) and the use of an mTOR kinase inhibitor in these diseases should be of therapeutic value. These latter examples in addition to those of PTEN deficiency or PI3K mutation indicate where a targeted approach to the use of mTOR inihibitors may prove particularly effective due to an underlying genetic profile, but are not considered to be exclusive targets.

Recent studies have revealed a role for mTOR kinase in other diseases (Easton & Houghton, Expert Opinion on Therapeutic Targets, 2004, 8, 551-564). Rapamycin has been demonstrated to be a potent immunosuppressant by inhibiting antigen-induced proliferation of T cells, B cells and antibody production (Sehgal, Transplantation Proceedings, 2003, 35, 7S-14S) and thus mTOR kinase inhibitors may also be useful immunosuppressives. Inhibition of the kinase activity of mTOR may also be useful in the prevention of restenosis, that is the control of undesired proliferation of normal cells in the vasculature in response to the introduction of stents in the treatment of vasculature disease (Morice et al., New England Journal of Medicine, 2002, 346, 1773-1780). Furthermore, the Rapamycin analogue, everolimus, can reduce the severity and incidence of cardiac allograft vasculopathy (Eisen et al., New England Journal of Medicine, 2003, 349, 847-858). Elevated mTOR kinase activity has been associated with cardiac hypertrophy, which is of clinical importance as a major risk factor for heart failure and is a consequence of increased cellular size of cardiomyocytes (Tee & Blenis, Seminars in Cell and Developmental Biology, 2005, 16, 29-37). Thus mTOR kinase inhibitors are expected to be of value in the prevention and treatment of a wide variety of diseases in addition to cancer.

The vast majority of mTOR pharmacology to date has focused on inhibition of mTOR via rapamycin or its analogues. However, as noted above, the only non-rapamycin agents that have been reported to inhibit mTOR's activity via a kinase domain targetted mechanism are the small molecule LY294002 and the natural product wortmannin (ref. 21).

EP0185259 (Dr Karl Thomae GMBH) discloses certain pteridines with valuable pharmacological properties, more particularly antithrombotic and metastasis-inhibiting effects and inhibiting anti tumour activity.

### Summary of the Invention

The present inventors have identified compounds which are ATP-competitive inhibitors of mTOR, and hence are non-rapamycin like in their mechanism of action.

Accordingly, the first aspect of the present invention provides a compound of formula I: 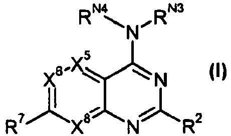 wherein:
X⁸ is N, and X⁵ and X⁶ are CH;;
R⁷ is a C₅₋₂₀ aryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino);
R^{N3} and R^{N4}, together with the nitrogen to which they are bound, form a heterocyclic ring containing between 3 and 8 ring atoms optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyi, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino);
R² is selected from NR^{N5}R^{N6}, a C₅₋₂₀ heteroaryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyi, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), and a C₅₋₂₀ aryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cydoalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
wherein R^{N5} and R^{N6} are independently selected from H, a C₁₋₇ alkyl group, a C₅₋₂₀ heteroaryl group, and a C₅₋₂₀ aryl group, or R^{N5} and R^{N6} together with the nitrogen to which they are bound form a heterocyclic ring containing between 3 and 8 ring atoms where each C₁₋₇alkyl, C₅₋₂₀heteroaryl, C₅₋₂₀aryl or heterocyclic ring is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
or a pharmaceutically acceptable salt thereof,
and wherein
"C₅₋₂₀ aryl" as used herein, pertains to a monovalent moiety obtained by removing a hydrogen atom from an aromatic ring atom of a C₅₋₂₀ aromatic compound, said compound having one ring, or two or more rings (e.g., fused), and having from 5 to 20 ring atoms, and wherein at least one of said ring(s) is an aromatic ring and where the ring atoms may include one or more heteroatoms, including but not limited to oxygen, nitrogen, and sulphur;
"alkyl" as used herein, pertains to a monovalent moiety obtained by removing a hydrogen atom from a carbon atom of a hydrocarbon compound having from 1 to 20 carbon atoms (unless otherwise specified), which may be aliphatic or alicyclic, and which may be saturated or unsaturated (e.g. partially unsaturated, fully unsaturated);
"alkenyl", as used herein, pertains to an alkyl group having one or more carbon-carbon double bonds;
"alkynyl", as used herein, pertains to an alkyl group having one or more carbon-carbon triple bonds;
"cycloalkyl", as used herein, pertains to an alkyl group which is also a cyclyl group; that is, a monovalent moiety obtained by removing a hydrogen atom from an alicyclic ring atom of a carbocyclic ring of a carbocyclic compound, which carbocyclic ring may be saturated or unsaturated (e.g. partially unsaturated, fully unsaturated), which moiety has from 3 to 20 carbon atoms (unless otherwise specified), including from 3 to 20 ring atoms;
"ether", as used herein, pertains to a -OR group, wherein R is a C₁₋₇ alkyl group, a C₃₋₂₀ heterocyclyl group, or a C₅₋₂₀ aryl group;
"acyl", as used herein, pertains to a -C(=O)R group, wherein R is H, a C₁₋₇ alkyl group, a C₃₋₂₀ heterocyclyl group, or a C₅₋₂₀ aryl group;
"ester", as used herein, pertains to a -C(=O)OR group, wherein R is a C₁₋₇ alkyl group, a C₃₋₂₀ heterocyclyl group, or a C₅₋₂₀ aryl group;
"amido", as used herein, pertains to a -C(=O)NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"amino", as used herein, pertains to a -NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"acylamido", as used herein, pertains to a -NR¹C(=O)R² group, wherein R¹ hydrogen, a C₁₋₇ alkyl group, a C₃₋₂₀ heterocyclyl group, or a C₅₋₂₀ aryl group, R² is a C₁₋₇ alkyl group, a C₃₋₂₀ heterocyclyl group, or a C₅₋₂₀ aryl group, or R¹ and R² may together with the atoms to which they are attached form a succinimidyl, maleimidyl, and phthalimidyl group;
"ureido", as used herein, pertains to a -N(R¹)CONR²R³ group, wherein R² and R³ are independently hydrogen, a C₁₋₇ alkyl group, a C₃₋₂₀ heterocyclyl group, or a C₅₋₂₀ aryl group, or R² and R³, taken together with the nitrogen atom to which they are attached, form a heterocyclic ring having from 4 to 8 ring atoms, and R¹ is hydrogen, a C₁₋₇alkyl group, a C₃₋₂₀heterocyclyl group, or a C₅₋₂₀aryl group;
"acyloxy", as used herein, pertains to a -OC(=O)R group, wherein R is a C₁₋₇ alkyl group, a C₃₋₂₀ heterocyclyl group, or a C₅₋₂₀ aryl group;
"thioether", as used herein, pertains to a -SR group, wherein R is a C₁₋₇ alkyl group, a C₃₋₂₀ heterocyclyl group, or a C₅₋₂₀ aryl group;
"sulfoxide", as used herein, pertains to a -S(=O)R group, wherein R is a C₁₋₇ alkyl group, a C₃₋₂₀ heterocyclyl group, or a C₅₋₂₀ aryl group;
"sulfonyl", as used herein, pertains to a -S(=O)₂R group, wherein R is a C₁₋₇ alkyl group, a C₃₋₂₀ heterocyclyl group, or a C₅₋₂₀ aryl group;
"thioamido", as used herein, pertains to a -C(=S)NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"sulfonamino", as used herein, pertains to a -NR¹S(=O)₂R group, wherein R¹ is hydrogen, a C₁₋₇ alkyl group, a C₃₋₂₀ heterocyclyl group, or a C₅₋₂₀ aryl group, and R is a C₁₋₇alkyl group, a C₃₋₂₀heterocyclyl group, or a C₅₋₂₀aryl group; and
with the proviso that when R² is unsubstituted morpholino, R^{N3} and R^{N4} together with the nitrogen atom to which they are attached form an unsubstituted morpholino, R⁷ is not unsubstituted phenyl, and
when R² is unsubstituted piperidinyl, R^{N3} and R^{N4} together with the nitrogen atom to which they are attached form an unsubstituted piperidinyl, R⁷ is not unsubstituted phenyl.

According to a second aspect of the present invention there is provided a compound of formula I(A): 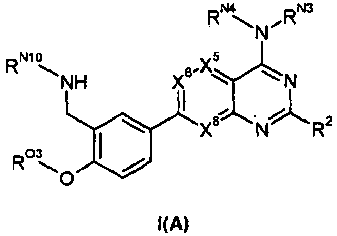 wherein:
X⁸ is N, and X⁵ and X⁶ are CH;;
R^{N3} and R^{N4}, together with the nitrogen to which they are bound, form a heterocyclic ring containing between 3 and 8 ring atoms optionally substituted by one or more groups selected from halo, hydroxyl, nitro, cyano, carboxy, and thiol, or C₁₋₇halkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino);
R² is selected from NR^{N5}R^{N6}, a C₅₋₂₀ heteroaryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), and a C₅₋₂₀ aryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
wherein R^{N5} and R^{N6} are independently selected from H, a C₁₋₇ alkyl group, a C₅₋₂₀ heteroaryl group, and a C₅₋₂₀ aryl group, or R^{N5} and R^{N6} together with the nitrogen to which they are bound form a heterocyclic ring containing between 3 and 8 ring atoms where each C₁₋₇alkyl, C₅₋₂₀heteroaryl, C₅₋₂₀aryl or heterocyclic ring is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroatyl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino);
R^{O3} is selected from hydrogen or a C₁₋₆ alkyl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino); and
R^{N10} is selected from C(=O)R^{C2}, C(=S)R^{C3}, SO₂R^{S3}, a C₅₋₂₀ heteroaryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), a C₅₋₂₀ aryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), or a C₁₋₁₀ alkyl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino) where R^{C2} and R^{C3} are selected from H, a C₅₋₂₀ aryl group, a C₅₋₂₀ heteroaryl group, a C₁₋₇ alkyl group or NRN¹¹RN¹², where R^{N11} and R^{N12} are independently selected from H, a C₁₋₇ alkyl group, a C₅₋₂₀ heteroaryl group, a C₅₋₂₀ aryl group or R^{N11} and R^{N12} together with the nitrogen to which they are bound form a heterocyclic ring containing between 3 and 8 ring atoms, where each C₁₋₇alkyl, C₅₋₂₀heteroaryl, C₅₋₂₀aryl or heterocyclic ring is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cydoalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino); and R^{S3} is selected from H, a C₅₋₂₀ aryl group, a C₅₋₂₀ heteroaryl group, or a C₁₋₇ alkyl group where each C₁₋₇alkyl, C₅₋₂₀heteroaryl or C₅₋₂₀aryl is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
or a pharmaceutically acceptable salt thereof,
and wherein "C₅₋₂₀ aryl" as used herein, pertains to a monovalent moiety obtained by removing a hydrogen atom from an aromatic ring atom of a C₅₋₂₀ aromatic compound, said compound having one ring, or two or more rings (e.g., fused), and having from 5 to 20 ring atoms, and wherein at least one of said ring(s) is an aromatic ring and where the ring atoms may include one or more heteroatoms, including but not limited to oxygen, nitrogen, and sulphur;
"alkyl" as used herein, pertains to a monovalent moiety obtained by removing a hydrogen atom from a carbon atom of a hydrocarbon compound having from 1 to 20 carbon atoms (unless otherwise specified), which may be aliphatic or alicyclic, and which may be saturated or unsaturated (e.g. partially unsaturated, fully unsaturated);
"alkenyl", as used herein, pertains to an alkyl group having one or more carbon-carbon double bonds;
"alkynyl", as used herein, pertains to an alkyl group having one or more carbon-carbon triple bonds;
"cycloalkyl", as used herein, pertains to an alkyl group which is also a cyclyl group; that is, a monovalent moiety obtained by removing a hydrogen atom from an alicyclic ring atom of a carbocyclic ring of a carbocyclic compound, which carbocyclic ring may be saturated or unsaturated (e.g. partially unsaturated, fully unsaturated), which moiety has from 3 to 20 carbon atoms (unless otherwise specified), including from 3 to 20 ring atoms;
"ether", as used herein, pertains to a -OR group, wherein R is a C₁₋₇ alkyl group, a C₃₋₂₀ heterocyclyl group, or a C₅₋₂₀ aryl group;
"acyl", as used herein, pertains to a -C(=O)R group, wherein R is H, a C₁₋₇ alkyl group, a C₃₋₂₀ heterocyclyl group, or a C₅₋₂₀ aryl group;
"ester", as used herein, pertains to a -C(=O)OR group, wherein R is a C₁₋₇ alkyl group, a C₃₋₂₀ heterocyclyl group, or a C₅₋₂₀ aryl group;
"amido", as used herein, pertains to a -C(=O)NR¹R² group, wherein R¹ and R² are independently hydrogen, a C₁₋₇ alkyl group, a C₃₋₂₀ heterocyclyl group, or a C₅₋₂₀ aryl group, or
R¹ and R², taken together with the nitrogen atom to which they are attached, form a heterocyclic ring having from 4 to 8 ring atoms;
"amino", as used herein, pertains to a -NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"acylamido", as used herein, pertains to a -NR¹C(=O)R² group, wherein R¹ hydrogen, a C₁₋₇ alkyl group, a C₃₋₂₀ heterocyclyl group, or a C₅₋₂₀ aryl group, R² is a C₁₋₇ alkyl group, a C₃₋₂₀ heterocyclyl group, or a C₅₋₂₀ aryl group, or R¹ and R² may together with the atoms to which they are attached form a succinimidyl, maleimidyl, and phthalimidyl group;
"ureido", as used herein, pertains to a -N(R¹)CONR²R³ group, wherein R² and R³ are independently hydrogen, a C₁₋₇ alkyl group, a C₃₋₂₀ heterocyclyl group, or a C₅₋₂₀ aryl group, or R² and R³, taken together with the nitrogen atom to which they are attached, form a heterocyclic ring having from 4 to 8 ring atoms, and R¹ is hydrogen, a C₁₋₇alkyl group, a C₃₋₂₀heterocyclyl group, or a C₅₋₂₀aryl group;
"acyloxy", as used herein, pertains to a -OC(=O)R group, wherein R is a C₁₋₇ alkyl group, a C₃₋₂₀ heterocyclyl group, or a C₅₋₂₀ aryl group;
"thioether", as used herein, pertains to a -SR group, wherein R is a C₁₋₇ alkyl group, a C₃₋₂₀ heterocyclyl group, or a C₅₋₂₀ aryl group;
"sulfoxide", as used herein, pertains to a -S(=O)R group, wherein R is a C₁₋₇alkyl group, a C₃₋₂₀ heterocyclyl group, or a C₅₋₂₀ aryl group;
"sulfonyl", as used herein, pertains to a -S(=O)₂R group, wherein R is a C₁₋₇ alkyl group, a C₃₋₂₀ heterocyclyl group, or a C₅₋₂₀ aryl group;
"thioamido", as used herein, pertains to a -C(=S)NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"sulfonamino", as used herein, pertains to a -NR¹S(=O)₂R group, wherein R¹ is hydrogen, a C₁₋₇ alkyl group, a C₃₋₂₀ heterocyclyl group, or a C₅₋₂₀ aryl group, and R is a C₁₋₇alkyl group, a C₃₋₂₀heterocyclyl group, or a C₅₋₂₀aryl group;.

According to a further aspect of the present invention there is provided a pharmaceutical composition comprising a compound of formula 1 or 1(A), or a pharmaceutically acceptable salt thereof, and a pharmaceutically acceptable carrier or diluent.

According to a further aspect of the present invention there is provided of formula 1 or 1(A), or a pharmaceutically acceptable salt thereof, for use in a method of treatment of the human or animal body.

According to a further aspect of the present invention there is provided the use of a compound of formula 1 or 1(A), or a pharmaceutically acceptable salt thereof, in the preparation of a medicament for treating a disease ameliorated by the inhibition of mTOR.

Further aspects of the invention provide the use of a compound of formula 1 or 1(A), or a pharmaceutically acceptable salt thereof, in the preparation of a medicament for the treatment of: cancer, immuno-suppression, immune tolerance, autoimmune disease, inflammation, bone loss, bowel disorders, hepatic fibrosis, hepatic necrosis, rheumatoid arthritis, restenosis, cardiac allograft vasculopathy, psoriasis, beta-thalassaemia, and ocular conditions such as dry eye. mTOR inhibitors may also be effective as antifungal agents.

We have found that the compounds defined in the present invention, or a pharmaceutically acceptable salt thereof, are effective anti-cancer agents which property is believed to arise from their mTOR inhibitory properties. Accordingly the compounds of the present invention are expected to be useful in the treatment of diseases or medical conditions mediated alone or in part by mTOR, i.e. the compounds may be used to produce a mTOR inhibitory effect in a warm-blooded animal in need of such treatment.

Thus the compounds of the present invention provide a method for treating cancer characterised by inhibition of mTOR, i.e. the compounds may be used to produce an anti-cancer effect mediated alone or in part by the inhibition of mTOR.
Such a compound of the invention is expected to possess a wide range of anti-cancer properties as activating mutations in mTOR have been observed in many human cancers, including but not limited to, melanoma, papillary thyroid tumours, cholangiocarcinomas, colon, ovarian and lung cancers. Thus it is expected that a compound of the invention will possess anti-cancer activity against these cancers. It is in addition expected that a compound of the present invention will possess activity against a range of leukaemias, lymphoid malignancies and solid tumours such as carcinomas and sarcomas in tissues such as the liver, kidney, bladder, prostate, breast and pancreas. In particular such compounds of the invention are expected to slow advantageously the growth of primary and recurrent solid tumours of, for example, the skin, colon, thyroid, lungs and ovaries. More particularly such compounds of the invention, or a pharmaceutically acceptable salt thereof, are expected to inhibit the growth of those primary and recurrent solid tumours which are associated with mTOR, especially those tumours which are significantly dependent on mTOR for their growth and spread, including for example, certain tumours of the skin, colon, thyroid, lungs and ovaries. Particularly the compounds of the present invention are useful in the treatment of melanomas.

Thus according to this aspect of the invention there is provided a compound of the formula I or 1 (A), or a pharmaceutically acceptable salt thereof, as defined herein for use as a medicament.

According to a further aspect of the invention there is provided the use of a compound of the formula I or I(A), or a pharmaceutically acceptable salt thereof, as defined herein in the manufacture of a medicament for use in the production of a mTOR inhibitory effect in a warm-blooded animal such as man.

According to this aspect of the invention there is provided the use of a compound of the formula I or I(A), or a pharmaceutically acceptable salt thereof, as defined herein in the manufacture of a medicament for use in the production of an anti-cancer effect in a warm-blooded animal such as man.

According to a further feature of the invention, there is provided the use of a compound of the formula I or I(A), or a pharmaceutically acceptable salt thereof, as defined herein in the manufacture of a medicament for use in the treatment of melanoma, papillary thyroid tumours, cholangiocarcinomas, colon cancer, ovarian cancer, lung cancer, leukaemias, lymphoid malignancies, carcinomas and sarcomas in the liver, kidney, bladder, prostate, breast and pancreas, and primary and recurrent solid tumours of the skin, colon, thyroid, lungs and ovaries.

In a further aspect of the invention there is provided a pharmaceutical composition which comprises a compound of the formula I or I(A), or a pharmaceutically acceptable salt thereof, as defined herein in association with a pharmaceutically-acceptable diluent or carrier for use in the production of a mTOR inhibitory effect in a warm-blooded animal such as man.

In a further aspect of the invention there is provided a pharmaceutical composition which comprises a compound of the formula I or I(A), or a pharmaceutically acceptable salt thereof, as defined herein in association with a pharmaceutically-acceptable diluent or carrier for use in the production of an anti-cancer effect in a warm-blooded animal such as man.

In a further aspect of the invention there is provided a pharmaceutical composition which comprises a compound of the formula I or I(A), or a pharmaceutically acceptable salt thereof, as defined herein in association with a pharmaceutically-acceptable diluent or carrier for use in the treatment of melanoma, papillary thyroid tumours, cholangiocarcinomas, colon cancer, ovarian cancer, lung cancer, leukaemias, lymphoid malignancies, carcinomas and sarcomas in the liver, kidney, bladder, prostate, breast and pancreas, and primary and recurrent solid tumors of the skin, colon, thyroïd, lungs and ovaries in a wram-blooded animal such as man.

Another further aspect of the invention provides for the use of a compound of formula 1 or 1 (A), or a pharmaceutically acceptable salt thereof, in the preparation of a medicament for use as an adjunct in cancer therapy or for potentiating tumour cells for treatment with ionizing radiation or chemotherapeutic agents.

The compounds of the invention can be used for the treatment of disease ameliorated by the inhibition of mTOR, comprising administering to a subject in need of treatment a therapeutically-effective amount of a compound of formula 1 or 1(A), or a pharmaceutically acceptable salt thereof, preferably in the form of a pharmaceutical composition and the treatment of cancer, comprising administering to a subject in need of treatment a therapeutically-effective amount of a compound as defined in the first or second aspect in combination, preferably in the form of a pharmaceutical composition, simultaneously or sequentially with ionizing radiation or chemotherapeutic agents.

### Definitions

The term "aromatic ring" is used herein in the conventional sense to refer to a cyclic aromatic structure, that is, a structure having delocalised π-electron orbitals.

Nitrogen-coritaining heterocyclic ring having from 3 to 8 ring atoms: The term "Nitrogen-containing heterocyclic ring having from 3 to 8 ring atoms" as used herein refers to a 3 to 8 membered heterocylic ring containing at least one nitrogen ring atom. The term "together with the nitrogen to which they are bound, form a heterocyclic ring containing between 3 and 8 ring atoms" as used herein refers to a 3 to 8 membered heterocylic ring containing at least one nitrogen ring atom. Examples of these groups include, but are not limited to:
N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆), tetrahydropyridine (C₆), azepine (C₇);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂O₁: oxadiazine (C₆);
N₁O₁S₁: oxathiazine (C₆).
Alkyl: The term "alkyl" as used herein, pertains to a monovalent moiety obtained by removing a hydrogen atom from a carbon atom of a hydrocarbon compound having from 1 to 20 carbon atoms (unless otherwise specified), which may be aliphatic or alicyclic, and which may be saturated or unsaturated (e.g. partially unsaturated, fully unsaturated). Thus, the term "alkyl" includes the sub-classes alkenyl, alkynyl, cycloalkyl, cycloalkyenyl, cylcoalkynyl, etc., discussed below.

In the context of alkyl groups, the prefixes (e.g. C₁₋₄, C₁₋₇, C₁₋₂₀, C₂₋₇, C₃₋₇, etc.) denote the number of carbon atoms, or range of number of carbon atoms. For example, the term "C₁₋₄ alkyl", as used herein, pertains to an alkyl group having from 1 to 4 carbon atoms. Examples of groups of alkyl groups include C₁₋₄ alkyl ("lower alkyl,"), C₁₋₇ alkyl, and C₁₋₂₀ alkyl. Note that the first prefix may vary according to other limitations; for example, for unsaturated alkyl groups, the first prefix must be at least 2; for cyclic alkyl groups, the first prefix must be at least 3; etc.

Examples of (unsubstituted) saturated alkyl groups include, but are not limited to, methyl (C₁), ethyl (C₂), propyl (C₃), butyl (C₄), pentyl (C₅), hexyl (C₆), heptyl (C₇), octyl (C₈), nonyl (C₉), decyl (C₁₀), undecyl (C₁₁), dodecyl (C₁₂), tridecyl (C₁₃), tetradecyl (C₁₄), pentadecyl (C₁₅), and eicodecyl (C₂₀).

Examples of (unsubstituted) saturated linear alkyl groups include, but are not limited to, methyl (C₁), ethyl (C₂), n-propyl (C₃), n-butyl (C₄), n-pentyl (amyl) (C₅), n-hexyl (C₆), and n-heptyl (C₇).

Examples of (unsubstituted) saturated branched alkyl groups include iso-propyl (C₃), iso-butyl (C₄), see-butyl (C₄), tert-butyl (C₄), iso-pentyl (C₅), and neo-pentyl (C₅).

Alkenyl: The term "alkenyl", as used herein, pertains to an alkyl group having one or more carbon-carbon double bonds. Examples of groups of alkenyl groups include C₂₋₄ alkenyl, C₂₋₇ alkenyl, C₂₋₂₀ alkenyl.

Examples of (unsubstituted) unsaturated alkenyl groups include, but are not limited to, ethenyl (vinyl, -CH=CH₂), 1-propenyl (-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

Alkynyl: The term "alkynyl", as used herein, pertains to an alkyl group having one or more carbon-carbon triple bonds. Examples of groups of alkynyl groups include C₂₋₄ alkynyl, C₂₋₇ alkynyl, C₂₋₂₀ alkynyl.

Examples of (unsubstituted) unsaturated alkynyl groups include, but are not limited to, ethynyl (ethinyl, -C=CH) and 2-propynyl (propargyl, -CH₂-C=CH).

Cycloalkyl: The term "cycloalkyl", as used herein, pertains to an alkyl group which is also a cyclyl group; that is, a monovalent moiety obtained by removing a hydrogen atom from an alicyclic ring atom of a carbocyclic ring of a carbocyclic compound, which carbocyclic ring may be saturated or unsaturated (e.g. partially unsaturated, fully unsaturated), which moiety has from 3 to 20 carbon atoms (unless otherwise specified), including from 3 to 20 ring atoms. Thus, the term "cycloalkyl" includes the sub-classes cycloalkenyl and cycloalkynyl. Preferably, each ring has from 3 to 7 ring atoms. Examples of groups of cycloalkyl groups include C₃₋₂₀ cycloalkyl, C₃₋₁₅ cycloalkyl, C₃₋₁₀ cycloalkyl, C₃₋₇ cycloalkyl. Examples of cycloalkyl groups include, but are not limited to, those derived from:
saturated monocyclic hydrocarbon compounds: cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅), dimethylcyclobutane (C₆), methylcyclopentane (C₆), dimethylcyclopentane (C₇), methylcyclohexane (C₇), dimethylcyclohexane (C₈), menthane (C₁₀);
unsaturated monocyclic hydrocarbon compounds: cyclopropene (C₃), cyclobutene (C₄), cyclopentene (C₅), cyclohexene (C₆), methylcyclopropene (C₄), dimethylcyclopropene (C₅), methylcyclobutene (C₅), dimethylcyclobutene (C₆), methylcyclopentene (C₆), dimethylcyclopentene (C₇), methylcyclohexene (C₇), dimethylcyclohexene (C₈);
saturated polycyclic hydrocarbon compounds: thujane (C₁₀), carane (C₁₀), pinane (C₁₀), bornane (C₁₀), norcarane (C₇), norpinane (C₇), norbornane (C₇), adamantane (C₁₀), decalin (decahydronaphthalene) (C₁₀);
unsaturated polycyclic hydrocarbon compounds: camphene (C₁₀), limonene (C₁₀), pinene (C₁₀);
polycyclic hydrocarbon compounds having an aromatic ring: indene (C₉), indane (e.g., 2,3-dihydro-1H-indene) (C₉), tetraline (1,2,3,4-tetrahydronaphthalene) (C₁₀), acenaphthene (C₁₂), fluorene (C₁₃), phenalene (C₁₃), acephenanthrene (C₁₅), aceanthrene (C₁₆), cholanthrene (C₂₀).
Heterocyclyl: The term "heterocyclyl", as used herein, pertains to a monovalent moiety obtained by removing a hydrogen atom from a ring atom of a heterocyclic compound, which moiety has from 3 to 20 ring atoms (unless otherwise specified), of which from 1 to 10 are ring heteroatoms. Preferably, each ring has from 3 to 7 ring atoms, of which from 1 to 4 are ring heteroatoms.

In this context, the prefixes (e.g. C₃₋₂₀, C₃₋₇, C₅₋₆, etc.) denote the number of ring atoms, or range of number of ring atoms, whether carbon atoms or heteroatoms. For example, the term "C₅₋₆heterocyclyl", as used herein, pertains to a heterocyclyl group having 5 or 6 ring atoms. Examples of groups of heterocyclyl groups include C₃₋₂₀ heterocyclyl, C₅₋₂₀ heterocyclyl, C₃₋₁₅ heterocyclyl, C₅₋₁₅ heterocyclyl, C₃₋₁₂ heterocyclyl, C₅₋₁₂ heterocyclyl, C₃₋₁₀ heterocyclyl, C₅₋₁₀ heterocyclyl, C₃₋₇ heterocyclyl, C₅₋₇ heterocyclyl, and C₅₋₆ heterocyclyl.

Examples of monocyclic heterocyclyl groups include, but are not limited to, those derived from:
N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆), tetrahydropyridine (C₆), azepine (C₇);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃); thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂O₁: oxadiazine (C₆);
O₁S₁: oxathiole (C₅) and oxathiane (thioxane) (C₆); and,
N₁O₁S₁: oxathiazine (C₆).

Examples of substituted (non-aromatic) monocyclic heterocyclyl groups include those derived from saccharides, in cyclic form, for example, furanoses (C₅), such as arabinofuranose, lyxofuranose, ribofuranose, and xylofuranse, and pyranoses (C₆), such as allopyranose, altropyranose, glucopyranose, mannopyranose, gulopyranose, idopyranose, galactopyranose, and talopyranose.

Spiro-C₃₋₇ cycloalkyl or heterocyclyl: The term "spiro C₃₋₇ cycloalkyl or heterocyclyl" as used herein, refers to a C₃₋₇ cycloalkyl or C₃₋₇ heterocyclyl ring joined to another ring by a single atom common to both rings.

C₅₋₂₀ aryl: The term "C₅₋₂₀ aryl" as used herein, pertains to a monovalent moiety obtained by removing a hydrogen atom from an aromatic ring atom of a C₅₋₂₀ aromatic compound, said compound having one ring, or two or more rings (e.g., fused), and having from 5 to 20 ring atoms, and wherein at least one of said ring(s) is an aromatic ring. Preferably, each ring has from 5 to 7 ring atoms.

The ring atoms may be all carbon atoms, as in "carboaryl groups" in which case the group may conveniently be referred to as a "C₅₋₂₀ carboaryl" group.

Examples of C₅₋₂₀ aryl groups which do not have ring heteroatoms (i.e. C₅₋₂₀ carboaryl groups) include, but are not limited to, those derived from benzene (i.e. phenyl) (C₆), naphthalene (C₁₀), anthracene (C₁₄), phenanthrene (C₁₄), and pyrene (C₁₆).

Alternatively, the ring atoms may include one or more heteroatoms, including but not limited to oxygen, nitrogen, and sulfur, as in "heteroaryl groups". In this case, the group may conveniently be referred to as a "C₅₋₂₀ heteroaryl" group, wherein "C₅₋₂₀" denotes ring atoms, whether carbon atoms or heteroatoms. Preferably, each ring has from 5 to 7 ring atoms, of which from 0 to 4 are ring heteroatoms.

Examples of C₅₋₂₀ heteroaryl groups include, but are not limited to, C₅ heteroaryl groups derived from furan (oxole), thiophene (thiole), pyrrole (azole), imidazole (1,3-diazole), pyrazole (1,2-diazole), triazole, oxazole, isoxazole, thiazole, isothiazole, oxadiazole, tetrazole and oxatriazole; and C₆ heteroaryl groups derived from isoxazine, pyridine (azine), pyridazine (1,2-diazine), pyrimidine (1,3-diazine; e.g., cytosine, thymine, uracil), pyrazine (1,4-diazine) and triazine.

The heteroaryl group may be bonded via a carbon or hetero ring atom.

Examples of C₅₋₂₀ heteroaryl groups which comprise fused rings, include, but are not limited to, C₉ heteroaryl groups derived from benzofuran, isobenzofuran, benzothiophene, indole, isoindole; C₁₀ heteroaryl groups derived from quinoline, isoquinoline, benzodiazine, pyridopyridine; C₁₄ heteroaryl groups derived from acridine and xanthene.

The above defined groups eg alkyl, heterocyclyl, aryl etc, whether alone or part of another substituent, may themselves optionally be substituted with one or more groups selected from themselves and the additional substituents listed below.

Halo: -F, -Cl, -Br, and -I.

Hydroxy: -OH.

Ether: -OR, wherein R is an ether substituent, for example, a C₁₋₇ alkyl group (also referred to as a C₁₋₇ alkoxy group), a C₃₋₂₀ heterocyclyl group (also referred to as a C₃₋₂₀ heterocyclyloxy group), or a C₅₋₂₀ aryl group (also referred to as a C₅₋₂₀ aryloxy group), preferably a C₁₋₇ alkyl group.

Nitro: -NO₂.

Cyano (nitrile, carbonitrile): -CN.

Acyl (keto): -C(=O)R, wherein R is an acyl substituent, for example, H, a C₁₋₇ alkyl group (also referred to as C₁₋₇ alkylacyl or C₁₋₇ alkanoyl), a C₃₋₂₀ heterocyclyl group (also referred to as C₃₋₂₀ heterocyclylacyl), or a C₅₋₂₀ aryl group (also referred to as C₅₋₂₀ arylacyl), preferably a C₁₋₇ alkyl group. Examples of acyl groups include, but are not limited to, -C(=O)CH₃ (acetyl), -C(=O)CH₂CH₃ (propionyl), -C(=O)C(CH₃)₃ (butyryl), and -C(=O)Ph (benzoyl, phenone).

Carboxy (carboxylic acid): -COOH.

Ester (carboxylate, carboxylic acid ester, oxycarbonyl): -C(=O)OR, wherein R is an ester substituent, for example, a C₁₋₇ alkyl group, a C₃₋₂₀ heterocyclyl group, or a C₅₋₂₀ aryl group, preferably a C₁₋₇ alkyl group. Examples of ester groups include, but are not limited to, -C(=O)OCH₃, -C(=O)OCH₂CH₃, -C(=O)OC(CH₃)₃, and -C(=O)OPh.

Amido (carbamoyl, carbamyl, aminocarbonyl, carboxamide): -C(=O)NR¹R², wherein R¹ and R² are independently amino substituents, as defined for amino groups. Examples of amido groups include, but are not limited to, -C(=O)NH₂, -C(=O)NHCH₃, -C(=O)N(CH₃)₂, -C(=O)NHCH₂CH₃, and -C(=O)N(CH₂CH₃)₂, as well as amido groups in which R¹ and R², together with the nitrogen atom to which they are attached, form a heterocyclic structure as in, for example, piperidinocarbonyl, morpholinocarbonyl, thiomorpholinocarbonyl, and piperazinylcarbonyl.

Amino: -NR¹R², wherein R¹ and R¹ are independently amino substituents, for example, hydrogen, a C₁₋₇ alkyl group (also referred to as C₁₋₇ alkylamino or di-C₁₋₇ alkylamino), a C₃₋₂₀ heterocyclyl group, or a C₅₋₂₀ aryl group, preferably H or a C₁₋₇ alkyl group, or, in the case of a "cyclic" amino group, R¹ and R², taken together with the nitrogen atom to which they are attached, form a heterocyclic ring having from 4 to 8 ring atoms. Examples of amino groups include, but are not limited to, -NH₂, -NHCH₃, -NHCH(CH₃)₂, -N(CH₃)₂, -N(CH₂CH₃)₂, and -NHPh. Examples of cyclic amino groups include, but are not limited to, aziridinyl, azetidinyl, pyrrolidinyl, piperidino, piperazinyl, perhydrodiazepinyl, morpholino, and thiomorpholino. The cylic amino groups may be substituted on their ring by any of the substituents defined here, for example carboxy, carboxylate and amido.

Acylamido (acylamino): -NR¹C(=O)R², wherein R¹ is an amide substituent, for example, hydrogen, a C₁₋₇ alkyl group, a C₃₋₂₀ heterocyclyl group, or a C₅₋₂₀ aryl group, preferably H or a C₁₋₇ alkyl group, most preferably H, and R² is an acyl substituent, for example, a C₁₋₇ alkyl group, a C₃₋₂₀ heterocyclyl group, or a C₅₋₂₀ aryl group, preferably a C₁₋₇ alkyl group. Examples of acylamide groups include, but are not limited to, -NHC(=O)CH₃ , -NHC(=O)CH₂CH₃, and -NHC(=O)Ph. R¹ and R² may together form a cyclic structure, as in, for example, succinimidyl, maleimidyl, and phthalimidyl:

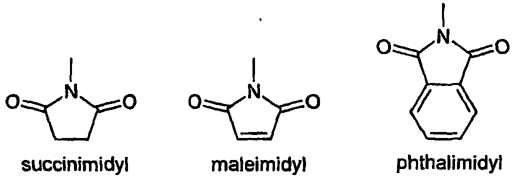

Ureido: -N(R¹)CONR²R³ wherein R² and R³ are independently amino substituents, as defined for amino groups, and R1 is a ureido substituent, for example, hydrogen, a C₁₋₇alkyl group, a C₃₋₂₀heterocyclyl group, or a C₅₋₂₀aryl group, preferably hydrogen or a C₁₋₇alkyl group. Examples of ureido groups include, but are not limited to, -NHCONH₂,-NHCONHMe, -NHCONHEt, -NHCONMe₂, -NHCONEt₂, -NMeCONH₂, -NMeCONHMe, -NMeCONHEt, -NMeCONMe₂, -NMeCONEt₂ and -NHC(=O)NHPh.

Acyloxy (reverse ester): -OC(=O)R, wherein R is an acyloxy substituent, for example, a C₁₋₇ alkyl group, a C₃₋₂₀ heterocyclyl group, or a C₅₋₂₀ aryl group, preferably a C₁₋₇ alkyl group. Examples of acyloxy groups include, but are not limited to, -OC(=O)CH₃ (acetoxy), - OC(=O)CH₂CH₃, -OC(=O)C(CH₃)₃, -OC(=O)Ph, -OC(=O)C₆H₄F, and -OC(=O)CH₂Ph.

Thiol : -SH.

Thioether (sulfide): -SR, wherein R is a thioether substituent, for example, a C₁₋₇ alkyl group (also referred to as a C₁₋₇ alkylthio group), a C₃₋₂₀ heterocyclyl group, or a C₅₋₂₀ aryl group, preferably a C₁₋₇ alkyl group. Examples of C₁₋₇ alkylthio groups include, but are not limited to, -SCH₃ and -SCH₂CH₃.

Sulfoxide (sulfinyl): -S(=O)R, wherein R is a sulfoxide substituent, for example, a C₁₋₇ alkyl group, a C₃₋₂₀ heterocyclyl group, or a C₅₋₂₀ aryl group, preferably a C₁₋₇ alkyl group. Examples of sulfoxide groups include, but are not limited to, -S(=O)CH₃ and -S(=O)CH₂CH₃.

Sulfonyl (sulfone): -S(=O)₂R, wherein R is a sulfone substituent, for example, a C₁₋₇ alkyl group, a C₃₋₂₀ heterocyclyl group, or a C₅₋₂₀ aryl group, preferably a C₁₋₇ alkyl group. Examples of sulfone groups include, but are not limited to, -S(=O)₂CH₃ (methanesulfonyl, mesyl), -S(=O)₂CF₃, -S(=O)₂CH₂CH₃, and 4-methylphenylsulfonyl (tosyl).

Thioamido (thiocarbamyl): -C(=S)NR¹R², wherein R¹ and R² are independently amino substituents, as defined for amino groups. Examples of amido groups include, but are not limited to, -C(=S)NH₂, -C(=S)NHCH₃, -C(=S)N(CH₃)₂, and -C(=S)NHCH₂CH₃.

Sulfonamino: -NR¹S(=O)₂R, wherein R¹ is an amino substituent, as defined for amino groups, and R is a sulfonamino substituent, for example, a C₁₋₇alkyl group, a C₃₋₂₀heterocyclyl group, or a C₅₋₂₀aryl group, preferably a C₁₋₇akyl group. Examples of sulfonamino groups include, but are not limited to, -NHS(=O)₂CH₃, -NHS(=O)₂Ph and -N(CH₃)S(=O)₂C₆H₅.

As mentioned above, the groups that form the above listed substituent groups, e.g. C₁₋₇ alkyl, C₃₋₂₀ heterocyclyl, and C₅₋₂₀ aryl, may themselves be substituted. Thus, the above definitions cover substituent groups which are substituted.

Accordingly, a further aspect of the present invention provides a compound of formula I: 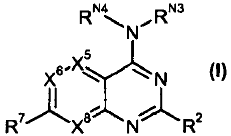 wherein:
X⁸ is N, and X⁵ and X⁶ are CH;
**R**⁷ is a C₅₋₂₀ aryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
**R^{N3}** and **R^{N4}**, together with the nitrogen to which they are bound, form a heterocyclic ring containing between 3 and 8 ring atoms optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino);
**R²** is NR^{N5}R^{N6}, a C₅₋₂₀ heteroaryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl,
C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇aLkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), or a C₅₋₂₀ aryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
wherein
**R^{N5}** and **R^{N6}** are independently H, a C₁₋₇ alkyl group, a C₅₋₂₀ heteroaryl group, a C₅₋₂₀ aryl group, or R^{N5} and R^{N6} together with the nitrogen to which they are bound form a heterocyclic ring containing between 3 and 8 ring atoms where each C₁₋₇alkyl, C₅₋₂₀heteroaryl, C₅₋₂₀aryl or heterocyclic ring is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
or a pharmaceutically acceptable salt thereof,
and wherein "C₅₋₂₀ aryl" as used herein, pertains to a monovalent moiety obtained by removing a hydrogen atom from an aromatic ring atom of a C₅₋₂₀ aromatic compound, said compound having one ring, or two or more rings (e.g., fused), and having from 5 to 20 ring atoms, and wherein at least one of said ring(s) is an aromatic ring and where the ring atoms may include one or more heteroatoms, including but not limited to oxygen, nitrogen, and sulphur; and
with the proviso that when R² is unsubstituted morpholino, R^{N3} and R^{N4} together with the nitrogen atom to which they are attached form an unsubstituted morpholino, R⁷ is not unsubstituted phenyl, and
when R² is unsubstituted piperidinyl, R^{N3} and R^{N4} together with the nitrogen atom to which they are attached form an unsubstituted piperidinyl, R⁷ is not unsubstituted phenyl..

According to a further aspect of the present invention there is provided a compound of formula I(A): 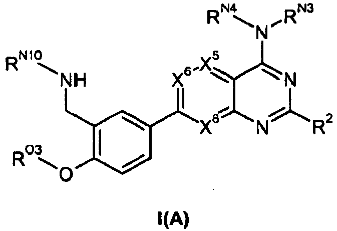 wherein:
one or two of X⁵, X⁶ and X⁸ is N, and the others are CH;
**R^{N3}** and **R^{N4}**, together with the nitrogen to which they are bound, form a heterocyclic ring containing between 3 and 8 ring atoms optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇akyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino);
**R²** is selected from NR^{N5}R^{N6}, a C₅₋₂₀ heteroaryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), and a C₅₋₂₀ aryl group optionally substituted by one or more groups selected from halo, hydroxyl, nitro, cyano, carboxy, and thiol, or C₁₋₇alkyl, C₂₋₇akenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
wherein **R^{N5}** and **R^{N6}** are independently H, a C₁₋₇ alkyl group, a C₅₋₂₀ heteroaryl group, a C₅₋₂₀ aryl group, or R^{N5} and R^{N6} together with the nitrogen to which they are bound form a heterocyclic ring containing between 3 and 8 ring atoms where each C₁₋₇alkyl, C₅₋₂₀heteroaryl, C₅₋₂₀aryl or heterocyclic ring is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino);
**R^{O3}** is hydrogen or a C₁₋₆ alkyl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇akyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino); and
**R^{N10}** is C(=O)R^{C2}, C(=S)R^{C3}, SO₂R^{S3}, a C₅₋₂₀heteroaryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), a C₅₋₂₀ aryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), or a C₁₋₁₀ alkyl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
where **R^{C2}** and **R^{C3}** are H, a C₅₋₂₀ aryl group, a C₅₋₂₀ heteroaryl group, a C₁₋₇ alkyl group or NRN¹¹R^{N12}, where **R^{N11}** and **R^{N12}** are independently H, a C₁₋₇ alkyl group, a C₅₋₂₀ heteroaryl group, a C₅₋₂₀ aryl group or R^{N11} and R^{N12} together with the nitrogen to which they are bound form a heterocyclic ring containing between 3 and 8 ring atoms, where each C₁₋₇alkyl, C₅₋₂₀heteroaryl, C₅₋₂₀aryl or heterocyclic ring is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyt, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), and R^{S3} is H, a C₅₋₂₀ aryl group, a C₅₋₂₀ heteroaryl group, or a C₁₋₇ alkyl group where each C₁₋₇alkyl, C₅₋₂₀heteroaryl or C₅₋₂₀aryl is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
or a pharmaceutically acceptable salt thereof,
and wherein "C₅₋₂₀ aryl" as used herein, pertains to a monovalent moiety obtained by removing a hydrogen atom from an aromatic ring atom of a C₅₋₂₀ aromatic compound, said compound having one ring, or two or more rings (e.g., fused), and having from 5 to 20 ring atoms, and wherein at least one of said ring(s) is an aromatic ring and where the ring atoms may include one or more heteroatoms, including but not limited to oxygen, nitrogen, and sulphur.

### Further Preferences

The following preferences can apply to each aspect of the present invention, where applicable. The preferences for each group may be combined with those for any or all of the other groups, as appropriate.

### X⁵, X⁶, and X⁸

X⁸ is N, and X⁵ and X⁶ are CH.

### R⁷

R⁷ is preferably selected from an optionally substituted C₅₋₂₀ aryl group.

If R⁷ is a C₅₋₂₀ aryl group, it is preferably a C₅₋₁₀ aryl and more preferably C₅₋₆ aryl group. Most preferably R⁷ is an optionally substituted phenyl group, wherein the optional substituents are preferably selected from halo, hydroxyl, C₁₋₇ alkyl and C₁₋₇ alkoxy.

In one embodiment, R⁷ is an optionally substituted C₅₋₁₀ aryl group, wherein the optional substituents are selected from cyano, halo, hydroxyl, and C₁₋₇ alkyl and C₁₋₇ alkoxy (wherein the alkyl groups may be optionally substituted by one or more groups selected from halo, hydroxyl, C₁₋₇ alkoxy, amino and C₅₋₆ aryl). In another embodiment, R⁷ is an optionally substituted C₅₋₆ aryl group, wherein the optional substituents are selected from cyano, halo, hydroxyl, and C₁₋₇ alkyl and C₁₋₇ alkoxy (wherein the alkyl groups may be optionally substituted by one or more groups selected from halo, hydroxyl, C₁₋₇ alkoxy, amino and C₅₋₆ aryl). In a further embodiment R⁷ is a thiophenyl group or a phenyl goup optionally substituted by one or more groups selected from chloro, hydroxyl, methyl, methoxy, ethoxy, i-propoxy, benzyloxy and hydroxymethyl. In a further embodiment R⁷ is 4-chlorophenyl, 4-methylphenyl, 4-methoxyphenyl, 3-hydroxymethyl-4-methoxy-phenyl, 3,5-dimethoxy-4-hydroxyphenyl, 4-hydroxyphenyl, 3-hydroxyphenyl or a 3-hydroxymethylphenyl group. In a still further embodiment R⁷ is an aryl group as defined in any of examples 1a, 1b, 1d, 1e, 1f, 1g, 1i, 1k, 1l, 1m, 1n, 1o, 1p, 1q, 1bb, 1bc, 1bd, 1be, 1bf, 1bg, 1bh, 1bi, 1bj or 1br.

### R^{N10}

R^{N10} is preferably selected from C(=S)R^{C3}, an optionally substituted C₅₋₂₀ heteroaryl group, an optionally substituted C₅₋₂₀ aryl group, and an optionally substituted C₁₋₁₀ alkyl group where R^{C3} is as previously defined.

If R^{N10} is C(=S)R^{C3}, then preferably R^{C3} is NR^{N11}R^{N12}, where R^{N11} and R^{N12} together with the nitrogen to which they are bound form a heterocyclic ring containing between 3 and 8 ring atoms.

If R^{N10} is a C₅₋₂₀ heteroaryl group, it is preferably a C₅₋₁₀ heteroaryl group and more preferably C₅₋₆ heteroaryl group. Most preferably it is an optionally substituted pyrazole group, wherein the optional substituents are preferably selected from halo, hydroxyl, C₁₋₇ alkyl and C₁₋₇ alkoxy.

If R^{N10} is a C₅₋₂₀ aryl group, it is preferably a C₅₋₁₀ aryl and more preferably C₅₋₆ aryl group. Most preferably it is an optionally substituted phenyl group, wherein the optional substituents are preferably selected from halo, hydroxyl, C₁₋₇ alkyl and C₁₋₇ alkoxy.

If R^{N10} is a C₁₋₁₀ alkyl group, it is preferably a C₁₋₁₀ alkyl group and more preferably C₁₋₁₀ alkyl group. Most preferably it is an optionally substituted C₁₋₆ alkyl group, wherein the optional substituents are preferably selected from halo, hydroxyl, C₁₋₇ alkyl, ether, for example C₁₋₇ alkoxy, thioether, for example C₁₋₇ alkylthio, C₅₋₂₀ aryl, C₃₋₂₀ heterocyclyl, C₅₋₂₀ heteroaryl, cyano, ester, for example -C(=O)OR where R is C₁₋₇alkyl, and amino, for example C₁₋₇alkylamino, di-C₁₋₇alkylamino and C₁₋₇alkoxycarbonylamino.

In one embodiment R^{N10} is a group as shown in any of examples 8a, 8b, 8c, 8d, 8e, 8f, 8g, 8h, 8i, 8j, 8k, 81, 8m, 8n, 8o, 8t, 8u, 8aa, 8ab, 8ac, 8ad, 8ae, 8af, 8ag, 8ah, 8ai, 8aj, 8ak, 8al, 8am, 8an, 8ao, 8ap, 8aq, 8ar, 8as, 8at, 8au, 8av, 8aw, 8ax, 8ay, 8az, 8ba, 8bb, 8bc, 8bd, 8be and 8bg.

### R^{O3}

R^{O3} is preferably an optionally substituted C₁₋₆ alkyl group. More preferably R^{O3} is an unsubstituted C₁₋₃ alkyl group, preferably a methyl group.

### R^{N3} and R^{N4}

R^{N3} and R^{N4} together with the nitrogen to which they are bound preferably form a heterocyclic ring containing between 5 and 7 ring atoms, which may optionally be substituted. Preferred optionally substituted groups include, but are not limited, to morpholino, thiomorpholino, piperidinyl, piperazinyl (preferably N-substituted), homopiperazinyl (preferably N-substituted) and pyrrolidinyl.

More preferably the group formed is morpholino or thiomorpholino, which are preferably unsubstituted. The most preferred group is morpholino.

### R²

In one embodiment R² is selected from NR^{N5}R^{N6}, an optionally substituted C₅₋₂₀ heteroaryl group, and an optionally substituted C₅₋₂₀ aryl group.

In another embodiment R² is selected from NR^{N5}R^{N6}, an optionally substituted C₅₋₆ heteroaryl group, and an optionally substituted C₆ aryl group.

In a further embodiment R² is phenyl group optionally substituted with one or more groups selected from hydroxyl, amino, nitro, carboxyl, formyl, cyano, methyl, amido, methyl, methoxymethyl and hydroxymethyl.

In a still further embodiment R² is an aryl group as shown in any of examples 9a, 9b, 9c, 9d, 9e, 9f, 9g, 9h, 9i, 9j, 9k, 91, 9m, 9n and 9ae.

Preferably R² is NR^{N5}R^{N6}, where R^{N5} and R^{N6} are as previously defined, and more preferably R^{N5} and R^{N6} together with the nitrogen to which they are bound form a heterocyclic ring containing between 3 and 8 ring atoms, which may optionally be substituted. The ring preferably has from 5 to 7 ring atoms. Preferred optionally substituted groups include, but are not limited, to morpholino, thiomorpholino, piperidinyl, piperazinyl (preferably N-substituted), homopiperazinyl (preferably N-substituted) and pyrrolidinyl.

Preferred N-substituents for the piperazinyl and homopiperazinyl groups include esters, in particular, esters bearing a C₁₋₇ alkyl group as an ester substituent, e.g. -C(=O)OCH₃, -C(=O)OCH₂CH₃ and -C(=O)OC(CH₃)₃.

Preferred C-substituents for the groups include C₁₋₄ alkyl, preferably methyl. The groups may bear one or more substituents, for example one or two substituents.

More preferred groups are morpholino and piperidinyl. These are preferably substituted with one or two methyl substituents. If these groups bear two methyl substituents, these are preferably on separate carbon atoms. Particularly preferred groups include:

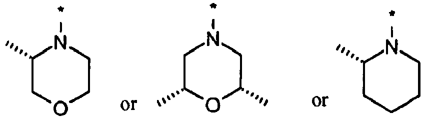

In an embodiment of the invention, there is provided a subset of compounds of formula **(I),** and pharmaceutically acceptable salts thereof, in which:
X⁸ is N, and X⁵ and X⁶ are CH;
R⁷ is an optionally substituted C₅₋₂₀ aryl group;
R^{N3} and R^{N4} together with the nitrogen to which they are bound form a heterocyclic ring containing between 5 and 7 ring atoms, which may optionally be substituted; and
R² is selected from NR^{N5}R^{N6}, an optionally substituted C₅₋₂₀ heteroaryl group, and an optionally substituted C₅₋₂₀ aryl group.

In another embodiment, there is provided a subset of compounds of formula (I), and pharmaceutically acceptable salts thereof, in which:
X⁸ is N, and X⁵ and X⁶ are CH;
R⁷ is an optionally substituted C₅₋₆ aryl group, wherein the optional substituents are selected from cyano, halo, hydroxyl, and C₁₋₇ alkyl and C₁₋₇ alkoxy (wherein the alkyl groups may be optionally substituted by one or more groups selected from halo, hydroxyl, C₁₋₇ alkoxy, amino and C₅₋₆ aryl);
R^{N3} and R^{N4} together with the nitrogen to which they are bound preferably form an optionally substituted morpholino, thiomorpholino, piperidinyl, piperazinyl (preferably N-substituted), homopiperazinyl (preferably N-substituted) or pyrrolidinyl group; and
R² is selected from NR^{N5}R^{N6}, an optionally substituted C₅₋₆ heteroaryl group, and an optionally substituted C₆ aryl group.

In another embodiment, there is provided a subset of compounds of formula (I), and pharmaceutically acceptable salts thereof, in which:
X⁸ is N, and X⁵ and X⁶ are CH;
R⁷ is an optionally substituted C₅₋₆ aryl group, wherein the optional substituents are selected from cyano, halo, hydroxyl, and C₁₋₇ alkyl and C₁₋₇ alkoxy (wherein the alkyl groups may be optionally substituted by one or more groups selected from halo, hydroxyl, C₁₋₇ alkoxy, amino and C₅₋₆ aryl);
R^{N3} and R^{N4} together with the nitrogen to which they are bound preferably form an optionally substituted morpholino, thiomorpholino, piperidinyl, piperazinyl (preferably N-substituted), homopiperazinyl (preferably N-substituted) or pyrrolidinyl group; and
R² is NR^{N5}R^{N6} where R^{N5} and R^{N6} together with the nitrogen to which they are bound form a heterocyclic ring containing between 5 to 7 ring atoms which may be optionally be substituted.

In a further embodiment, there is provided a subset of compounds of formula (I), and pharmaceutically acceptable salts thereof, in which:
X⁸ is N, and X⁵ and X⁶ are CH;
R⁷ is an optionally substituted C₅₋₆ aryl group, wherein the optional substituents are selected from cyano, halo, hydroxyl, and C₁₋₇ alkyl and C₁₋₇ alkoxy (wherein the alkyl groups may be optionally substituted by one or more groups selected from halo, hydroxyl, C₁₋₇ alkoxy, amino and C₅₋₆ aryl);
R^{N3} and R^{N4} together with the nitrogen to which they are bound preferably form an optionally substituted morpholino, thiomorpholino, piperidinyl, piperazinyl (preferably N-substituted), homopiperazinyl (preferably N-substituted) or pyrrolidinyl group; and
R² is NR^{N5}R^{N6} where R^{N5} and R^{N6} together with the nitrogen to which they are bound form an optionally substituted morpholino, thiomorpholino, piperidinyl, piperazinyl (preferably N-substituted), homopiperazinyl (preferably N-substituted) or pyrrolidinyl group.

In a further embodiment, there is provided a subset of compounds of formula (I), and pharmaceutically acceptable salts thereof, in which:
X⁸ is N, and X⁵ and X⁶ are CH;
R⁷ is a thiophenyl group or a phenyl goup optionally substituted by one or more groups selected from chloro, hydroxyl, methyl, methoxy, ethoxy, i-propoxy, benzyloxy and hydroxymethyl;
R^{N3} and R^{N4} together with the nitrogen to which they are bound preferably form an optionally substituted morpholino, thiomorpholino, piperidinyl, piperazinyl (preferably N-substituted), homopiperazinyl (preferably N-substituted) or pyrrolidinyl group; and
R² is NR^{N5}R^{N6} where R^{N5} and R^{N6} together with the nitrogen to which they are bound form an optionally substituted morpholino, thiomorpholino, piperidinyl, piperazinyl (preferably N-substituted), homopiperazinyl (preferably N-substituted) or pyrrolidinyl group.

In a further embodiment, there is provided a subset of compounds of formula (I), and pharmaceutically acceptable salts thereof, in which:
X⁸ is N, and X⁵ and X⁶ are CH;
R⁷ is a thiophenyl group or a phenyl goup optionally substituted by one or more groups selected from chloro, hydroxyl, methyl, methoxy, ethoxy, i-propoxy, benzyloxy and hydroxymethyl;
R^{N3} and R^{N4} together with the nitrogen to which they are bound preferably form an optionally substituted morpholino, thiomorpholino, piperidinyl, piperazinyl (preferably N-substituted), homopiperazinyl (preferably N-substituted) or pyrrolidinyl group; and
R² is NR^{N5}R^{N6} where R^{N5} and R^{N6} together with the nitrogen to which they are bound form a morpholino, thiomorpholino, piperidinyl, piperazinyl (preferably N-substituted), homopiperazinyl (preferably N-substituted) or pyrrolidinyl group optionally substituted on carbon with one or more C₁₋₄alkyl groups.

In a further embodiment, there is provided a subset of compounds of formula (I), and pharmaceutically acceptable salts thereof, in which:
X⁸ is N, and X⁵ and X⁶ are CH;
R⁷ is a thiophenyl group or a phenyl goup optionally substituted by one or more groups selected from chloro, hydroxyl, methyl, methoxy, ethoxy, i-propoxy, benzyloxy and hydroxymethyl;
R^{N3} and R^{N4} together with the nitrogen to which they are bound preferably form a morpholino or thiomorpholino; and
R² is NR^{N5}R^{N6} where R^{N5} and R^{N6} together with the nitrogen to which they are bound form a morpholino, thiomorpholino, piperidinyl, piperazinyl (preferably N-substituted), homopiperazinyl (preferably N-substituted) or pyrrolidinyl group optionally substituted on carbon with one or more C₁₋₄alkyl groups.

In a further embodiment, there is provided a subset of compounds of formula (I), and pharmaceutically acceptable salts thereof, in which:
X⁸ is N, and X⁵ and X⁶ are CH;
R⁷ is a 4-chlorophenyl, 4-methylphenyl, 4-methoxyphenyl, 3-hydroxymethyl-4-methoxy-phenyl, 3,5-dimethoxy-4-hydroxyphenyl, 4-hydroxyphenyl, 3-hydroxyphenyl or a 3-hydroxymethylphenyl group;
R^{N3} and R^{N4} together with the nitrogen to which they are bound preferably form a morpholino or thiomorpholino; and
R² is NR^{N5}R^{N6} where R^{N5} and R^{N6} together with the nitrogen to which they are bound form a 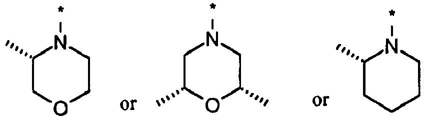 group.

In a further embodiment, there is provided a subset of compounds of formula (I), and pharmaceutically acceptable salts thereof, in which:
X⁸ is N, and X⁵ and X⁶ are CH;
R⁷ is a 4-chlorophenyl, 4-methylphenyl, 4-methoxyphenyl, 3-hydroxymethyl-4-methoxy-phenyl, 3,5-dimethoxy-4-hydroxyphenyl, 4-hydroxyphenyl, 3-hydroxyphenyl or a 3-hydroxymethylphenyl group;
R^{N3} and R^{N4} together with the nitrogen to which they are bound preferably form an unsubstituted morpholino; and
R² is NR^{N5}R^{N6} where R^{N5} and R^{N6} together with the nitrogen to which they are bound
form a 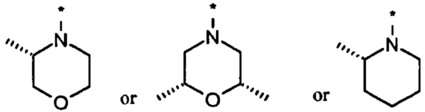 group.

In an embodiment of the invention, there is provided a subset of compounds of formula I(A), and pharmaceutically acceptable salts thereof, in which:
X⁸ is N, and X⁵ and X⁶ are CH;
R^{N10} is selected from C(=S)R^{C3}, an optionally substituted C₅₋₂₀ heteroaryl group, an optionally substituted C₅₋₂₀ aryl group, and an optionally substituted C₁₋₁₀ alkyl group where R^{C3} is as previously defined;
R^{O3} is an optionally substituted C₁₋₆ alkyl group;
R^{N3} and R^{N4} together with the nitrogen to which they are bound form a heterocyclic ring containing between 5 and 7 ring atoms, which may optionally be substituted; and
R² is selected from NR^{N5}R^{N6}, an optionally substituted C₅₋₂₀ heteroaryl group, and an optionally substituted C₅₋₂₀ aryl group.

In another embodiment, there is provided a subset of compounds of formula I(A), and pharmaceutically acceptable salts thereof, in which:
X⁸ is N, and X⁵ and X⁶ are CH;
R^{N10} is C(=S)R^{C3}, an optionally substituted C₅₋₆ heteroaryl group, an optionally substituted C₅₋₆ aryl group or an optionally substituted C₁₋₁₀ alkyl group where R^{C3} is NR^{N11}R^{N12} and where R^{N11} and R^{N12} together with the nitrogen to which they are bound form a heterocyclic ring containing between 3 and 8 ring atoms;.
R^{O3} is an unsubstituted C₁₋₃ alkyl group;
R^{N3} and R^{N4} together with the nitrogen to which they are bound preferably form an optionally substituted morpholino, thiomorpholino, piperidinyl, piperazinyl (preferably N-substituted), homopiperazinyl (preferably N-substituted) or pyrrolidinyl group; and
R² is selected from NR^{N5}R^{N6}, an optionally substituted C₅₋₆ heteroaryl group, and an optionally substituted C₆ aryl group.

In another embodiment, there is provided a subset of compounds of formula I(A), and pharmaceutically acceptable salts thereof, in which:
X⁸ is N, and X⁵ and X⁶ are CH;
RN¹⁰ is a C(=S)NR^{N11}R^{N12} group where R^{N11} and R^{N12} together with the nitrogen to which they are bound form a heterocyclic ring containing between 3 and 8 ring atoms, or a pyrazole group optionally substituted with one or more groups selected from halo, hydroxyl, C₁₋₇ alkyl and C₁₋₇ alkoxy, or a phenyl group optionally substituted with one or more groups selected from halo, hydroxyl, C₁₋₇ alkyl and C₁₋₇ alkoxy, or a C₁₋₆ alkyl group optionally substituted with one or more groups selected from halo, hydroxyl, C₁. ₇ alkyl, ether, for example C₁₋₇ alkoxy, thioether, for example C₁₋₇ alkylthio, C₅₋₂₀ aryl, C₃₋₂₀ heterocyclyl, C₅₋₂₀ heteroaryl, cyano, ester, for example -C(=O)OR where R is C₁₋₇alkyl, and amino, for example C₁₋₇alkylamino, di-C₁₋₇alkylamino and C₁₋₇alkoxycarbonylamino;
R^{O3} is a methyl group;
R^{N3} and R^{N4} together with the nitrogen to which they are bound preferably form an optionally substituted morpholino, thiomorpholino, piperidinyl, piperazinyl (preferably N-substituted), homopiperazinyl (preferably N-substituted) or pyrrolidinyl group; and
R² is NR^{N5}R^{N6} where R^{N5} and R^{N6} together with the nitrogen to which they are bound form a heterocyclic ring containing between 5 to 7 ring atoms which may be optionally be substituted.

In a further embodiment, there is provided a subset of compounds of formula I(A), and pharmaceutically acceptable salts thereof, in which:
X⁸ is N, and X⁵ and X⁶ are CH;
R^{N10} is a C(=S)NR^{N11}R^{N12} group where R^{N11} and R^{N12} together with the nitrogen to which they are bound form a heterocyclic ring containing between 3 and 8 ring atoms, or a pyrazole group optionally substituted with one or more groups selected from halo, hydroxyl, C₁₋₇ alkyl and C₁₋₇ alkoxy, or a phenyl group optionally substituted with one or more groups selected from halo, hydroxyl, C₁₋₇ alkyl and C₁₋₇ alkoxy, or a C₁₋₆ alkyl group optionally substituted with one or more groups selected from halo, hydroxyl, C₁₋₇ alkyl, ether, for example C₁₋₇ alkoxy, thioether, for example C₁₋₇ alkylthio, C₅₋₂₀ aryl, C₃₋₂₀ heterocyclyl, C₅₋₂₀ heteroaryl, cyano, ester, for example -C(=O)OR where R is C₁₋₇alkyl, and amino, for example C₁₋₇alkylamino, di-C₁₋₇alkylamino and C₁₋₇alkoxycarbonylamino;
R^{O3} is a methyl group;
R^{N3} and R^{N4} together with the nitrogen to which they are bound preferably form an optionally substituted morpholino, thiomorpholino, piperidinyl, piperazinyl (preferably N-substituted), homopiperazinyl (preferably N-substituted) or pyrrolidinyl group; and
R² is NR^{N5}R^{N6} where R^{N5} and R^{N6} together with the nitrogen to which they are bound form an optionally substituted morpholino, thiomorpholino, piperidinyl, piperazinyl (preferably N-substituted), homopiperazinyl (preferably N-substituted) or pyrrolidinyl group.

In a further embodiment, there is provided a subset of compounds of formula I(A), and pharmaceutically acceptable salts thereof, in which:
X⁸ is N, and X⁵ and X⁶ are CH;
R^{N10} is a C(=S)NR^{N11}R^{N12} group where R^{N11} and R^{N12} together with the nitrogen to which they are bound form a heterocyclic ring containing between 3 and 8 ring atoms, or a pyrazole group optionally substituted with one or more groups selected from halo, hydroxyl, C₁₋₇ alkyl and C₁₋₇ alkoxy, or a phenyl group optionally substituted with one or more groups selected from halo, hydroxyl, C₁₋₇ alkyl and C₁₋₇ alkoxy, or a C₁₋₆ alkyl group optionally substituted with one or more groups selected from halo, hydroxyl, C₁₋₇ alkyl, ether, for example C₁₋₇ alkoxy, thioether, for example C₁₋₇ alkylthio, C₅₋₂₀ aryl, C₃₋₂₀ heterocyclyl, C₅₋₂₀ heteroaryl, cyano, ester, for example -C(=O)OR where R is C₁₋₇alkyl, and amino, for example C₁₋₇alkylamino, di-C₁₋₇alkylamino and C₁₋₇alkoxycarbonylamino;
R^{O3} is a methyl group;
R^{N3} and R^{N4} together with the nitrogen to which they are bound preferably form an optionally substituted morpholino, thiomorpholino, piperidinyl, piperazinyl (preferably N-substituted), homopiperazinyl (preferably N-substituted) or pyrrolidinyl group; and
R² is NR^{N5}R^{N6} where R^{N5} and R^{N6} together with the nitrogen to which they are bound form an optionally substituted morpholino, thiomorpholino, piperidinyl, piperazinyl (preferably N-substituted), homopiperazinyl (preferably N-substituted) or pyrrolidinyl group.

In a further embodiment, there is provided a subset of compounds of formula I(A), and pharmaceutically acceptable salts thereof, in which:
X⁸ is N, and X⁵ and X⁶ are CH;
R^{N10} is a C(=S)NR^{N11}R^{N12} group where R^{N11} and R^{N12} together with the nitrogen to which they are bound form a heterocyclic ring containing between 3 and 8 ring atoms, or a pyrazole group optionally substituted with one or more groups selected from halo, hydroxyl, C₁₋₇ alkyl and C₁₋₇ alkoxy, or a phenyl group optionally substituted with one or more groups selected from halo, hydroxyl, C₁₋₇ alkyl and C₁₋₇ alkoxy, or a C₁₋₆ alkyl group optionally substituted with one or more groups selected from halo, hydroxyl, C₁₋₇ alkyl, ether, for example C₁₋₇ alkoxy, thioether, for example C₁₋₇ alkylthio, C₅₋₂₀ aryl, C₃₋₂₀ heterocyclyl, C₅₋₂₀ heteroaryl, cyano, ester, for example -C(=O)OR where R is C₁₋₇alkyl, and amino, for example C₁₋₇alkylamino, di-C₁₋₇alkylamino and C₁₋₇alkoxycarbonylamino;
R^{O3} is a methyl group;
R^{N3} and R^{N4} together with the nitrogen to which they are bound preferably form a morpholino or thiomorpholino group; and
R² is NR^{N5}R^{N6} where R^{N5} and R^{N6} together with the nitrogen to which they are bound form morpholino, thiomorpholino, piperidinyl, piperazinyl (preferably N-substituted), homopiperazinyl (preferably N-substituted) or pyrrolidinyl group optionally substituted on carbon by one or more C₁₋₄alkyl groups.

In a further embodiment, there is provided a subset of compounds of formula **I(A)**, and pharmaceutically acceptable salts thereof, in which:
X⁸ is N, and X⁵ and X⁶ are CH;
R^{N10} is a C(=S)NR^{N11}R^{N12} group where R^{N11} and R^{N12} together with the nitrogen to which they are bound form a heterocyclic ring containing between 3 and 8 ring atoms, or a pyrazole group optionally substituted with one or more groups selected from halo, hydroxyl, C₁₋₇ alkyl and C₁₋₇ alkoxy, or a phenyl group optionally substituted with one or more groups selected from halo, hydroxyl, C₁₋₇ alkyl and C₁₋₇ alkoxy, or a C₁₋₆ alkyl group optionally substituted with one or more groups selected from halo, hydroxyl, C₁₋₇ alkyl, ether, for example C₁₋₇ alkoxy, thioether, for example C₁₋₇ alkylthio, C₅₋₂₀ aryl, Z₃₋₂₀ heterocyclyl, C₅₋₂₀ heteroaryl, cyano, ester, for example -C(=O)OR where R is C₁₋₇akyl, and amino, for example C₁₋₇alkylamino, di-C₁₋₇alkylamino and C₁₋₇alkoxycarbonylamino;
R^{O3} is a methyl group;
R^{N3} and R^{N4} together with the nitrogen to which they are bound preferably form a morpholino or thiomorpholino group; and
R² is NR^{N5}R^{N6} where R^{N5} and R^{N6} together with the nitrogen to which they are bound form a 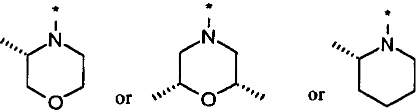 group.

In a further embodiment, there is provided a subset of compounds of formula **I(A)**, and pharmaceutically acceptable salts thereof, in which:
X⁸ is N, and X⁵ and X⁶ are CH;
R^{N10} is a C(=S)NR^{N11}R^{N12} group where R^{N11} and R^{N12} together with the nitrogen to which they are bound form a heterocyclic ring containing between 3 and 8 ring atoms, or a pyrazole group optionally substituted with one or more groups selected from halo, hydroxyl, C₁₋₇ alkyl and C₁₋₇ alkoxy, or a phenyl group optionally substituted with one or more groups selected from halo, hydroxyl, C₁₋₇ alkyl and C₁₋₇ alkoxy, or a C₁₋₆ alkyl group optionally substituted with one or more groups selected from halo, hydroxyl, C₁₋₇ alkyl, ether, for example C₁₋₇ alkoxy, thioether, for example C₁₋₇ alkylthio, C₅₋₂₀ aryl, C₃₋₂₀ heterocyclyl, C₅₋₂₀ heteroaryl, cyano, ester, for example -C(=O)OR where R is C₁₋₇alkyl, and amino, for example C₁₋₇alkylamino, di-C₁₋₇alkylamino and C₁₋₇alkoxycarbonylamino;
R^{O3} is a methyl group;
R^{N3} and R^{N4} together with the nitrogen to which they are bound preferably form an unsubstituted morpholino group; and
R² is NR⁵R^{N6} where R^{N5} and R^{N6} together with the nitrogen to which they are bound form a 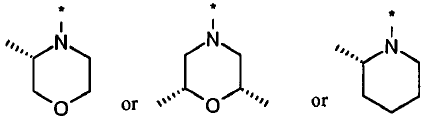 group.

In another aspect of the invention, particular compounds of the invention are any one of the Examples or pharmaceutically acceptable salts thereof.

In a further aspect of the invention, particular compounds of the invention are any one of Examples 1a, 1b, 1d, 1e, 1f, 1g, 1i, 1k, 1l, 1m, 1n, 1o, 1p, 1q, 1bb, 1bc, 1bd, 1be, 1bf, 1bg, 1bh, 1bi, 1br, 8a, 8b, 8c, 8d, 8e, 8f, 8g, 8h, 8i, 8j, 8k, 8l, 8m, 8n, 8o, 8t, 8u, 8aa, 8ab, 8ac, 8ad, 8ae, 8af, 8ag, 8ah, 8ai, 8aj, 8ak, 8al, 8am, 8an, 8ao, 8ap, 8aq, 8ar, 8as, 8at, 8au, 8av, 8aw, 8ax, 8ay, 8az, 8ba, 8bb, 8bc, 8bd, 8be, 8bg, 9a, 9b, 9c, 9d, 9e, 9f, 9g, 9h, 9i, 9j, 9k, 9l, 9m, 9n and 9ae, or pharmaceutically acceptable salts thereof.

### Isomers, Salts, Solvates, Protected Forms, and Prodrugs

Certain compounds may exist in one or more particlular geometric, optical, enantiomeric, diasteriomeric, epimeric, stereoisomeric, tautomeric, conformational, or anomeric forms, including but not limited to, *cis*- and *trans*-forms; *E*- and *Z*-forms; *c*-, *t*-, and *r*-forms; *endo*- and *exo*-forms; *R*-, *S*-, and *meso*-forms; *D*- and *L*-forms; *d*- and *l*-forms; (+) and (-) forms; keto-, enol-, and enolate-forms; syn- and anti-forms; synclinal- and anticlinal-forms; α- and β-forms; axial and equitorial forms; boat-, chair-, twist-, envelope-, and halfchair-forms; and combinations thereof, hereinafter collectively referred to as "isomers" (or "isomeric forms").

If the compound is in crystalline form, it may exist in a number of different polymorphic forms.

Note that, except as discussed below for tautomeric forms, specifically excluded from the term "isomers", as used herein, are structural (or constitutional) isomers (i.e. isomers which differ in the connections between atoms rather than merely by the position of atoms in space). For example, a reference to a methoxy group, -OCH₃, is not to be construed as a reference to its structural isomer, a hydroxymethyl group, -CH₂OH. Similarly, a reference to ortho-chlorophenyl is not to be construed as a reference to its structural isomer, meta-chlorophenyl. However, a reference to a class of structures may well include structurally isomeric forms falling within that class (e.g., C₁₋₇ alkyl includes *n*-propyl and *iso*-propyl; butyl includes *n*-, *iso-, sec-,* and *tert*-butyl; methoxyphenyl includes *ortho-, meta-,* and *para-*methoxyphenyl).

The above exclusion does not pertain to tautomeric forms, for example, keto-, enol-, and enolate-forms, as in, for example, the following tautomeric pairs: keto/enol, imine/enamine, amide/imino alcohol, amidine/amidine, nitroso/oxime, thioketone/enethiol, *N-*nitroso/hyroxyazo, and nitro/aci-nitro.

Unless otherwise specified, a reference to a particular compound includes all such isomeric forms, including (wholly or partially) racemic and other mixtures thereof. Methods for the preparation (e.g. asymmetric synthesis) and separation (e.g. fractional crystallisation and chromatographic means) of such isomeric forms are either known in the art or are readily obtained by adapting the methods taught herein, or known methods, in a known manner.

Unless otherwise specified, a reference to a particular compound also includes ionic; salt, solvate, and protected forms of thereof, for example, as discussed below, as well as its different polymorphic forms.

It may be convenient or desirable to prepare, purify, and/or handle a corresponding salt of the active compound, for example, a pharmaceutically-acceptable salt. Examples of pharmaceutically acceptable salts are discussed in ref. 25.

For example, if the compound is anionic, or has a functional group which may be anionic (e.g., -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al³⁺. Examples of suitable organic cations include, but are not limited to, ammonium ion (i.e., NH₄⁺) and substituted ammonium ions (e.g., NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group which may be cationic (e.g., -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous. Examples of suitable organic anions include, but are not limited to, those derived from the following organic acids: acetic, propionic, succinic, gycolic, stearic, palmitic, lactic, malic, pamoic, tartaric, citric, gluconic, ascorbic, maleic, hydroxymaleic, phenylacetic, glutamic, aspartic, benzoic, cinnamic, pyruvic, salicyclic, sulfanilic, 2-acetyoxybenzoic, fumaric, toluenesulfonic, methanesulfonic, ethanesulfonic, ethane disulfonic, oxalic, isethionic, valeric, and gluconic. Examples of suitable polymeric anions include, but are not limited to, those derived from the following polymeric acids: tannic acid, carboxymethyl cellulose.

It may be convenient or desirable to prepare, purify, and/or handle a corresponding solvate of the active compound. The term "solvate" is used herein in the conventional sense to refer to a complex of solute (e.g. active compound, salt of active compound) and solvent. If the solvent is water, the solvate may be conveniently referred to as a hydrate, for example, a mono-hydrate, a di-hydrate, a tri-hydrate, etc.

It may be convenient or desirable to prepare, purify, and/or handle the active compound in a chemically protected form. The term "chemically protected form," as used herein, pertains to a compound in which one or more reactive functional groups are protected from undesirable chemical reactions, that is, are in the form of a protected or protecting group (also known as a masked or masking group or a blocked or blocking group). By protecting a reactive functional group, reactions involving other unprotected reactive functional groups can be performed, without affecting the protected group; the protecting group may be removed, usually in a subsequent step, without substantially affecting the remainder of the molecule. See, for example, ref. 26.

For example, a hydroxy group may be protected as an ether (-OR) or an ester (-OC(=O)R), for example, as: a *t*-butyl ether; a benzyl, benzhydryl (diphenylmethyl), or trityl (triphenylmethyl) ether; a trimethylsilyl or *t*-butyldimethylsilyl ether; or an acetyl ester (-OC(=O)CH₃, -OAc).

For example, an aldehyde or ketone group may be protected as an acetal or ketal, respectively, in which the carbonyl group (>C=O) is converted to a diether (>C(OR)₂), by reaction with, for example, a primary alcohol. The aldehyde or ketone group is readily regenerated by hydrolysis using a large excess of water in the presence of acid.

For example, an amine group may be protected, for example, as an amide or a urethane, for example, as: a methyl amide (-NHCO-CH₃); a benzyloxy amide (-NHCO-OCH₂C₆H₅, -NH-Cbz); as a t-butoxy amide (-NHCO-OC(CH₃)₃, -NH-Boc); a 2-biphenyl-2-propoxy amide (-NHCO-OC(CH₃)₂C₆H₄C₆H₅, -NH-Bpoc), as a 9-fluorenylmethoxy amide (-NH-Fmoc), as a 6-nitroveratryloxy amide (-NH-Nvoc), as a 2-trimethylsilylethyloxy amide (-NH-Teoc), as a 2,2,2-trichloroethyloxy amide (-NH-Troc), as an allyloxy amide (-NH-Alloc), as a 2(-phenylsulphonyl)ethyloxy amide (-NH-Psec); or, in suitable cases, as an *N*-oxide (>NO ·).

For example, a carboxylic acid group may be protected as an ester for example, as: an C₁₋₇ alkyl ester (e.g. a methyl ester; a *t*-butyl ester); a C₁₋₇ haloalkyl ester (e.g. a C₁₋₇ trihaloalkyl ester); a triC₁₋₇ alkylsilyl-C₁₋₇ alkyl ester; or a C₅₋₂₀ aryl-C₁₋₇ alkyl ester (e.g. a benzyl ester; a nitrobenzyl ester); or as an amide, for example, as a methyl amide.

For example, a thiol group may be protected as a thioether (-SR), for example, as: a benzyl thioether; an acetamidomethyl ether (-S-CH₂NHC(=O)CH₃).

It may be convenient or desirable to prepare, purify, and/or handle the active compound in the form of a prodrug. The term "prodrug", as used herein, pertains to a compound which, when metabolised (e.g. *in vivo),* yields the desired active compound. Typically, the prodrug is inactive, or less active than the active compound, but may provide advantageous handling, administration, or metabolic properties.

For example, some prodrugs are esters of the active compound (e.g. a physiologically acceptable metabolically labile ester). During metabolism, the ester group (-C(=O)OR) is cleaved to yield the active drug. Such esters may be formed by esterification, for example, of any of the carboxylic acid groups (-C(=O)OH) in the parent compound, with, where appropriate, prior protection of any other reactive groups present in the parent compound, followed by deprotection if required. Examples of such metabolically labile esters include those wherein R is C₁₋₂₀ alkyl (e.g. -Me, -Et); C₁₋₇ aminoalkyl (e.g. aminoethyl; 2-(*N,N* diethylamino)ethyl; 2-(4-morpholino)ethyl); and acyloxy-C₁₋₇ alkyl (e.g. acyloxymethyl; acyloxyethyl; e.g. pivaloyloxymethyl; acetoxymethyl; 1-acetoxyethyl; 1-(1-methoxy-1-methyl)ethyl-carbonxyloxyethyl; 1-(benzoyloxy)ethyl; isopropoxy-carbonyloxymethyl; 1-isopropoxy-carbonyloxyethyl; cyclohexyl-carbonyloxymethyl; 1-cyclohexylcarbonyloxyethyl; cyclohexyloxy-carbonyloxymethyl; 1-cyclohexyloxy-carbonyloxyethyl; (4-tetrahydropyranyloxy) carbonyloxymethyl; 1-(4-tetrahydropyranyloxy)carbonyloxyethyl; (4-tetrahydropyranyl)carbonyloxymethyl; and 1-(4-tetrahydropyranyl)carbonyloxyethyl).

Further suitable prodrug forms include phosphonate and glycolate salts. In particular, hydroxy groups (-OH), can be made into phosphonate prodrugs by reaction with chlorodibenzylphosphite, followed by hydrogenation, to form a phosphonate group -OP(=O)(OH)₂. Such a group can be cleared by phosphotase enzymes during metabolism to yield the active drug with the hydroxy group.

Also, some prodrugs are activated enzymatically to yield the active compound, or a compound which, upon further chemical reaction, yields the active compound. For example, the prodrug may be a sugar derivative or other glycoside conjugate, or may be an amino acid ester derivative.

### Acronyms

For convenience, many chemical moieties are represented using well known abbreviations, including but not limited to, methyl (Me), ethyl (Et), *n*-propyl (nPr), *iso*-propyl (iPr), *n*-butyl (nBu), *tert*-butyl (tBu), *n*-hexyl (nHex), cyclohexyl (cHex), phenyl (Ph), biphenyl (biPh), benzyl (Bn), naphthyl (naph), methoxy (MeO), ethoxy (EtO), benzoyl (Bz), and acetyl (Ac).

For convenience, many chemical compounds are represented using well known abbreviations, including but not limited to, methanol (MeOH), ethanol (EtOH), iso-propanol (i-PrOH), methyl ethyl ketone (MEK), ether or diethyl ether (Et₂O), acetic acid (AcOH), dichloromethane (methylene chloride, DCM), trifluoroacetic acid (TFA), dimethylformamide (DMF), tetrahydrofuran (THF), and dimethylsulfoxide (DMSO).

### General Synthesis

Compounds of formula I can be represented by Formula 1: 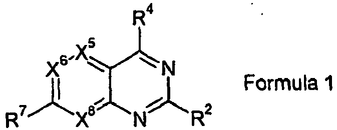 wherein R⁴ represents NR^{N3}R^{N4}.

Compounds of Formula 1 can be synthesised from compounds of Formula 2:

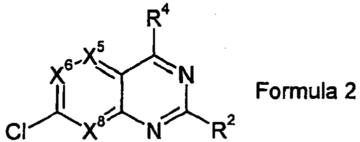

When R⁷ is NR^{N1}R^{N2}, this is by reaction with k⁷H. When R⁷ is an optionally substituted C₃₋₂₀ heterocyclyl group or C₅₋₂₀ aryl group, this is by reaction with R⁷B(OAlk)₂, where each Alk is independently C₁₋₇ alkyl or together with the oxygen to which they are attached form a C₅₋₇ heterocyclyl group. When R⁷ is an amide, urea or sulfonamide group, this is by reaction with ammonia followed by reaction of the resulting primary amide with the appropriate acid chloride, isocyanate or sulfonyl chloride. When R⁷ is OR^{O1} or SR^{S1}, this is by reaction with potassium carbonate in the appropriate alcohol or thiol solvent.

Therefore, according to a further aspect of the present invention there is provided a process for the preparation of a compound of formula 1, from a compound of Formula 2: 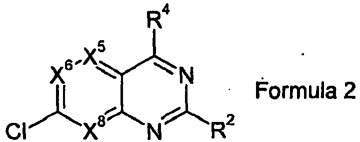 wherein:
**R⁴** is NR^{N3}R^{N4} where **R^{N3}** and **R^{N4},** together with the nitrogen to which they are bound, form a heterocyclic ring containing between 3 and 8 ring atoms;
**R² is** selected from H, halo, OR^{O2}, SR^{S2b}, NR^{N5}R^{N6}, an optionally substituted C₅₋₂₀ heteroaryl group, and an optionally substituted C₅₋₂₀ aryl group,
wherein **R^{O2}** and **R^{S2b}** are selected from H, an optionally substituted C₅₋₂₀ aryl group, an optionally substituted C₅₋₂₀ heteroaryl group, or an optionally substituted C₁₋₇ alkyl group, and **R^{N5}** and **R^{N6}** are independently selected from H, an optionally substituted C₁₋₇ alkyl group, an optionally substituted C₅₋₂₀ heteroaryl group, and an optionally substituted C₅₋₂₀ aryl group, or R^{N5} and R^{N6} together with the nitrogen to which they are bound form a heterocyclic ring containing between 3 and 8 ring atoms,
comprising
(a) when R⁷ is NR^{N1}R^{N2}, reaction of the compound of formula 2 with R⁷H; or
(b) when R⁷ is an optionally substituted C₃₋₂₀ heterocyclyl group or C₅₋₂₀ aryl group, reaction of the compound of formula 2 with R⁷B(OAlk)₂, where each Alk is independently C₁₋₇ alkyl or together with the oxygen to which they are attached form a C₅₋₇ heterocyclyl group, or
(c) when R⁷ is an amide, urea or sulfonamide group, reaction of a compound of formula 2 with ammonia followed by reaction of the resulting primary amine with the appropriate acid chloride, isocyanate or sulfonyl chloride, or
(d) when R⁷ is OR^{O1} or SR^{S1}, by reaction of the compound of formula 1 in the presence of base in the appropriate alcohol or thiol solvent.

Compounds of formula I(A) can be synthesised by reaction a compound of Formula 1a: 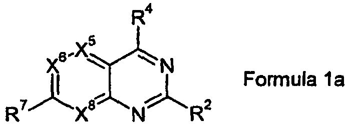
wherein R⁴ represents NR^{N3}R^{N4} , and
R⁷ is 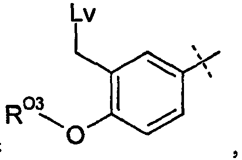 wherein Lv is a leaving group, such as a halogen, for example chlorine, or an OSO₂R group, where R is alkyl or aryl, such as methyl,
by reaction with R^{N10}NH₂.

Compounds of Formula 1a can be synthesised by reaction of a compound of Formula 1b 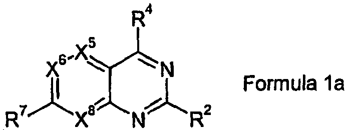
wherein R⁴ represents NR^{N3}R^{N4} , and
R⁷ is 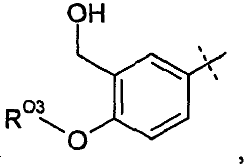
with an alkyl or aryl sulphonyl chloride in the presence of a base.

Compounds of Formula 1b can be synthesised by reacting a compound of Formula 2: 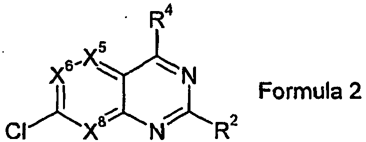 with R⁷B(OAlk)₂, where each Alk is independently C₁₋₇ alkyl or together with the oxygen to which they are attached form a C₅₋₇ heterocyclyl group.

Compounds of Formula 2 can be synthesised from compounds of Formula 3: 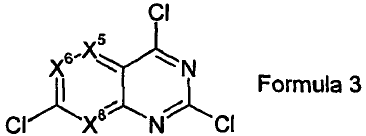 by reaction with HR⁴ (HNR^{N3}R^{N4}) followed by reaction with HR².

Compounds of Formula 3 can be synthesised from compounds of Formula 4: 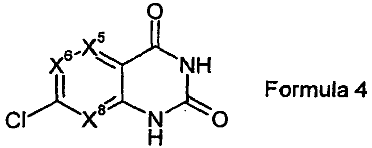 by treatment with POCl₃ and N,N-diisopropylamine, for example.

Compounds of Formula 4 can be synthesised from compounds of Formula 5: 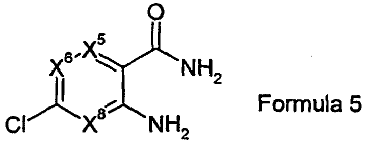 by treatment with oxalyl chloride, for example.

Compounds of Formula 5 can be synthesised from compounds of Formula 6, for example by reaction with liquid ammonia followed by reaction with thionyl chloride and ammonia gas:

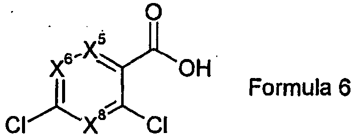

Alternatively, Compounds of Formula 1 can be synthesised from compounds of Formula 2A:

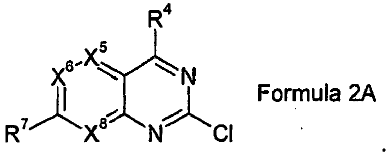

When R² is NR^{N5}R^{N6}, this is by reaction with R²H. When R² is an optionally substituted C₃₋₂₀ heterocyclyl group or C₅₋₂₀ aryl group, this is by reaction with R²B(OAlk)₂, where each Alk is independently C₁₋₇ alkyl or together with the oxygen to which they are attached form a C₅₋₇ heterocyclyl group. When R² is OR^{O2} or SR^{S2b}, this is by reaction with potassium carbonate in the appropriate alcohol or thiol solvent.

Therefore, according to a further aspect of the present invention there is provided a process for the preparation of a compound of formula 1 from a compound of formula 2A: 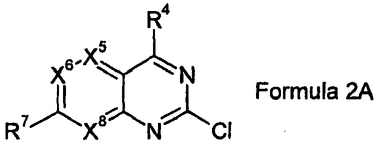 wherein
**R⁴** is NR^{N3}R^{N4} where **R^{N3}** and **R^{N4},** together with the nitrogen to which they are bound, form a heterocyclic ring containing between 3 and 8 ring atoms; and
**R⁷** is selected from halo, OR^{O1}, SR^{S1}, NR^{N1}R^{N2}, NR^{N7a}C(=O)R^{C1}, NR^{N7b}SO₂R^{S2a}, an optionally substituted C₅₋₂₀ heteroaryl group, or an optionally substituted C₅₋₂₀ aryl group,
where **R^{O1}** and **R^{S1}** are selected from H, an optionally substituted C₅₋₂₀ aryl group, an optionally substituted C₅₋₂₀ heteroaryl group, or an optionally substituted C₁₋₇ alkyl group; **R^{N1}** and **R¹²** are independently selected from H, an optionally substituted C₁₋₇ alkyl group, an optionally substituted C₅₋₂₀ heteroaryl group, an optionally substituted C₅₋₂₀ aryl group or R^{N1} and R^{N2} together with the nitrogen to which they are bound form a heterocyclic ring containing between 3 and 8 ring atoms;
**R^{C1}** is selected from H, an optionally substituted C₅₋₂₀ aryl group, an optionally substituted C₅₋₂₀ heteroaryl group, an optionally substituted C₁₋₇ alkyl group or NR^{N8}R^{N9}, where **R^{N8}** and **R^{N9}** are independently selected from H, an optionally substituted C₁₋₇ alkyl group, an optionally substituted C₅₋₂₀ heteroaryl group, an optionally substituted C₅₋₂₀ aryl group or R^{N8} and R^{N9} together with the nitrogen to which they are bound form a heterocyclic ring containing between 3 and 8 ring atoms;
**R^{S2a}** is selected from H, an optionally substituted C₅₋₂₀ aryl group, an optionally substituted C₅₋₂₀ heteroaryl group, or an optionally substituted C₁₋₇ alkyl group; and
**R^{N7a}** and **R^{N7b}** are selected from H and a C₁₋₄ alkyl group;
comprising
(a) when R² is NR^{N5}R^{N6}, reacting ^{a} compound of formula 2A with R²H, or
(b) when R² is an opionally substituted C₃₋₂₀ heterocyclyl group or C₅₋₂₀ aryl group, by reacting a compound of formula 2A with R²B(OAlk)₂, where each Alk is independently C₁₋₇ alkyl or together with the oxygen to which they are attached form a C₅₋₇ heterocyclyl group, or
(c) when R² is OR^{O2} or SR^{S2b}, by reacting a compound of formula 2A in the presence of a base in the appropriate alcohol or thiol solvent.

Compounds of Formula 2A can be synthesised from compounds of Formula 3: 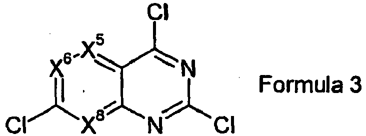 by reaction with HR⁴ (HNR^{N3}R^{N4}) followed by reaction with HR⁷ or HR⁷ equivalent. For example, when R⁷ is an optionally substituted C₃₋₂₀ heterocyclyl group or C₅₋₂₀ aryl group, this is by reaction with R⁷B(OAlk)₂, where each Alk is independently C₁₋₇ alkyl or together with the oxygen to which they are attached form a C₅₋₇ heterocyclyl group.

### Use

The present invention provides active compounds, specifically, active in inhibiting the activity of mTOR.

The term "active" as used herein, pertains to compounds which are capable of inhibiting mTOR activity, and specifically includes both compounds with intrinsic activity (drugs) as well as prodrugs of such compounds, which prodrugs may themselves exhibit little or no intrinsic activity.

One assay which may conveniently be used in order to assess the mTOR inhibition offered by a particular compound is described in the examples below.

The present invention further considers a method of inhibiting the activity of mTOR in a cell, comprising contacting said cell with an effective amount of an active compound, preferably in the form of a pharmaceutically acceptable composition. Such a method may be practised *in vitro* or *in vivo.*

For example, a sample of cells may be grown *in vitro* and an active compound brought into contact with said cells, and the effect of the compound on those cells observed. As examples of "effect", the inhibition of cellular growth in a certain time or the accumulation of cells in the G1 phase of the cell cycle over a certain time may be determined. Where the active compound is found to exert an influence on the cells, this may be used as a prognostic or diagnostic marker of the efficacy of the compound in methods of treating a patient carrying cells of the same cellular type.

The term "treatment", as used herein in the context of treating a condition, pertains generally to treatment and therapy, whether of a human or an animal (e.g. in veterinary applications), in which some desired therapeutic effect is achieved, for example, the inhibition of the progress of the condition, and includes a reduction in the rate of progress, a halt in the rate of progress, amelioration of the condition, and cure of the condition. Treatment as a prophylactic measure (i.e. prophylaxis) is also included.

The term "adjunct" as used herein relates to the use of active compounds in conjunction with known therapeutic means. Such means include cytotoxic regimes of drugs and/or ionising radiation as used in the treatment of different cancer types. Examples of adjunct anti-cancer agents that could be combined with compounds from the invention include, but are not limited to, the following: alkylating agents: nitrogen mustards, mechlorethamine, cyclophosphamide, ifosfamide, melphalan, chlorambucil: Nitrosoureas: carmustine (BCNU), lomustine (CCNU), semustine (methyl-CCNU), ethylenimine/methylmelamine, thriethylenemelamine (TEM), triethylene thiophosphoramide (thiotepa), hexamethylmelamine (HMM, altretamine): Alkyl sufonates; busulfan; Triazines, dacarbazine (DTIC): Antimetabolites; folic acid analogs, methotrexate, trimetrexate, pyrimidine analogs, 5-fluorouracil, fluorodeoxyuridine, gemcitabine, cytosine arabinoside (AraC, cytarabine), 5-azacytidine, 2,2'-difluorodeoxycytidine: Purine analogs; 6-mercaptopurine, 6-thioguanine, azathioprine, 2'-deoxycoformycin (pentostatin, erythrohydroxynonyladenine (EHNA), fludarabine phosphate, 2-Chlorodeoxyadenosine (cladribine, 2-CdA): Topoisomerase I inhibitors; camptothecin, topotecan, irinotecan, rubitecan: Natural products; antimitotic drugs, paclitaxel, vinca alkaloids, vinblastine (VLB), vincristine, vinorelbine, Taxotere^{™} (docetaxel), estramustine, estramustine phosphate; epipodophylotoxins, etoposide, teniposide: Antibiotics; actimomycin D, daunomycin (rubidomycin), doxorubicin (adriamycin), mitoxantrone, idarubicin, bleomycins, plicamycin (mithramycin), mitomycin C, dactinomycin: Enzymes; L-asparaginase, RNAse A: Biological response modifiers; interferon-alpha, IL-2, G-CSF, GM-CSF: Differentiation Agents; retinoic acid derivatives: Radiosensitizers;, metronidazole, misonidazole, desmethylmisonidazole, pimonidazole, etanidazole, nimorazole, RSU 1069, EO9, RB 6145, SR4233, nicotinamide, 5-bromodeozyuridine, 5-iododeoxyuridine, bromodeoxycytidine: Platinium coordination complexes; cisplatin, carboplatin: Anthracenedione; mitoxantrone, AQ4N Substituted urea, hydroxyurea; Methylhydrazine derivatives, N-methylhydrazine (MIH), procarbazine; Adrenocortical suppressant, mitotane (o.p'-DDD), aminoglutethimide: Cytokines; interferon (α, β, γ), interleukin; Hormones and antagonists; adrenocorticosteroids/antagonists, prednisone and equivalents, dexamethasone, aminoglutethimide; Progestins, hydroxyprogesterone caproate, medroxyprogesterone acetate, megestrol acetate; Estrogens, diethylstilbestrol, ethynyl estradiol/equivalents; Antiestrogen, tamoxifen; Androgens, testosterone propionate, fluoxymesterone/equivalents; Antiandrogens, flutamide, gonadotropin-releasing hormone analogs, leuprolide; Nonsteroidal antiandrogens, flutamide; EGFR inhibitors, VEGF inhibitors; Proteasome inhibitors.

Active compounds may also be used as cell culture additives to inhibit mTOR, for example, in order to sensitize cells to known chemotherapeutic agents or ionising radiation treatments *in vitro.*

Active compounds may also be used as part of an *in vitro* assay, for example, in order to determine whether a candidate host is likely to benefit from treatment with the compound in question.

### Cancer

The present invention provides active compounds which are anticancer agents or adjuncts for treating cancer. One of ordinary skill in the art is readily able to determine whether or not a candidate compound treats a cancerous condition for any particular cell type, either alone or in combination.

Examples of cancers include, but are not limited to, lung cancer, small cell lung cancer, gastrointestinal cancer, bowel cancer, colon cancer, breast carinoma, ovarian carcinoma, prostate cancer, testicular cancer, liver cancer, kidney cancer, bladder cancer, pancreas cancer, brain cancer, sarcoma, osteosarcoma, Kaposi's sarcoma, melanoma and leukemias.

Any type of cell may be treated, including but not limited to, lung, gastrointestinal (including, e.g., bowel, colon), breast (mammary), ovarian, prostate, liver (hepatic), kidney (renal), bladder, pancreas, brain, and skin.

The anti cancer treatment defined hereinbefore may be applied as a sole therapy or may involve, in addition to the compound of the invention, conventional surgery or radiotherapy or chemotherapy. Such chemotherapy may include one or more of the following categories of anti-tumour agents:-
(i) other antiproliferative/antineoplastic drugs and combinations thereof, as used in medical oncology, such as alkylating agents (for example cis platin, oxaliplatin, carboplatin, cyclophosphamide, nitrogen mustard, melphalan, chlorambucil, busulphan, temozolamide and nitrosoureas); antimetabolites (for example gemcitabine and antifolates such as fluoropyrimidines like 5 fluorouracil and tegafur, raltitrexed, methotrexate, cytosine arabinoside, and hydroxyurea); antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere and polokinase inhibitors); and topoisomerase inhibitors (for example epipodophyllotoxins like etoposide and teniposide, amsacrine, topotecan and camptothecin);
(ii) cytostatic agents such as antioestrogens (for example tamoxifen, fulvestrant, toremifene, raloxifene, droloxifene and iodoxyfene), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5*-reductase such as finasteride;
(iii) anti-invasion agents (for example c-Src kinase family inhibitors like 4-(6-chloro-2,3-methylenedioxyanilino)-7-[2-(4-methylpiperazin-1-yl] ethoxy]-5-tetrahydropyran-4-yloxyquinazoline (AZD0530; International Patent Application WO 01/94341) and N-(2-chloro-6-methylphenyl)-2-{6-[4-(2-hydroxyethyl)piperazin-1-yl]-2-methylpyrimidin-4-ylamino}thiazole-5-carboxamide (dasatinib, BMS-354825; J. Med. Chem., 2004, 47, 6658-6661), and metalloproteinase inhibitors like marimastat, inhibitors of urokinase plasminogen activator receptor function or antibodies to Heparanase);
(iv) inhibitors of growth factor function: for example such inhibitors include growth factor antibodies and growth factor receptor antibodies (for example the anti erbB2 antibody trastuzumab [Herceptin™], the anti-EGFR antibody panitumumab, the anti erbB1 antibody cetuximab [Erbitux, C225] and any growth factor or growth factor receptor antibodies disclosed by Stern et al. Critical reviews in oncology/haematology, 2005, Vol. 54, pp11-29); such inhibitors also include tyrosine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, ZD1839), N-(3-ethynylphenyl)-6,7-bis(2-methoxyethoxy)quinazolin-4-amine (erlotinib, OSI 774) and 6-acrylamido-N-(3-chloro-4-fluorophenyl)-7-(3-morpholinopropoxy)-quinazolin-4-amine (CI 1033), erbB2 tyrosine kinase inhibitors such as lapatinib, inhibitors of the hepatocyte growth factor family, inhibitors of the platelet-derived growth factor family such as imatinib, inhibitors of serine/threonine kinases (for example Ras/Raf signalling inhibitors such as farnesyl transferase inhibitors, for example sorafenib (BAY 43-9006)), inhibitors of cell signalling through MEK and/or AKT kinases, inhibitors of the hepatocyte growth factor family, c-kit inhibitors, abl kinase inhibitors, IGF receptor (insulin-like growth factor) kinase inhibitors; aurora kinase inhibitors (for example AZD1152, PH739358, VX-680, MLN8054, R763, MP235, MP529, VX-528 AND AX39459) and cyclin dependent kinase inhibitors such as CDK2 and/or CDK4 inhibitors;
(v) antiangiogenic agents such as those which inhibit the effects of vascular endothelial growth factor, [for example the anti vascular endothelial cell growth factor antibody bevacizumab (Avastin™) and VEGF receptor tyrosine kinase inhibitors such as 4-(4-bromo-2-fluoroanilino)-6-methoxy-7-(1-methylpiperidin-4-ylmethoxy)quinazoline (ZD6474; Example 2 within WO 01/32651), 4-(4-fluoro-2-methylindol-5-yloxy)-6-methoxy-7-(3-pyrrolidin-1-ylpropoxy)quinazoline (AZD2171; Example 240 within WO 00/47212), vatalanib (PTK787; WO 98/35985) and SU11248 (sunitinib; WO 01/60814), compounds such as those disclosed in International Patent Applications WO97/22596, WO 97/30035, WO 97/32856 and WO 98/13354 and compounds that work by other mechanisms (for example linomide, inhibitors of integrin avb3 function and angiostatin)];
(vi) vascular damaging agents such as Combretastatin A4 and compounds disclosed in International Patent Applications WO 99/02166, WO 00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene directed enzyme pro drug therapy) approaches such as those using cytosine deaminase, thymidine kinase or a bacterial nitroreductase enzyme and approaches to increase patient tolerance to chemotherapy or radiotherapy such as multi drug resistance gene therapy; and
(ix) immunotherapy approaches, including for example ex vivo and in vivo approaches to increase the immunogenicity of patient tumour cells, such as transfection with cytokines such as interleukin 2, interleukin 4 or granulocyte macrophage colony stimulating factor, approaches to decrease T cell anergy, approaches using transfected immune cells such as cytokine transfected dendritic cells, approaches using cytokine transfected tumour cell lines and approaches using anti idiotypic antibodies.

### Administration

The active compound or pharmaceutical composition comprising the active compound may be administered to a subject by any convenient route of administration, whether systemically/ peripherally or at the site of desired action, including but not limited to, oral (e.g. by ingestion); topical (including e.g. transdermal, intranasal, ocular, buccal, and sublingual); pulmonary (e.g. by inhalation or insufflation therapy using, e.g. an aerosol, e.g. through mouth or nose); rectal; vaginal; parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; by implant of a depot, for example, subcutaneously or intramuscularly.

The subject may be a eukaryote, an animal, a vertebrate animal, a mammal, a rodent (e.g. a guinea pig, a hamster, a rat, a mouse), murine (e.g. a mouse), canine (e.g. a dog), feline (e.g. a cat), equine (e.g. a horse), a primate, simian (e.g. a monkey or ape), a monkey (e.g. marmoset, baboon), an ape (e.g. gorilla, chimpanzee, orangutang, gibbon), or a human.

### Formulations

While it is possible for the active compound to be administered alone, it is preferable to present it as a pharmaceutical composition (e.g., formulation) comprising at least one active compound, as defined above, together with one or more pharmaceutically acceptable carriers, adjuvants, excipients, diluents, fillers, buffers, stabilisers, preservatives, lubricants, or other materials well known to those skilled in the art and optionally other therapeutic or prophylactic agents.

Thus, the present invention further provides pharmaceutical compositions, as defined above, and methods of making a pharmaceutical composition comprising admixing at least one active compound, as defined above, together with one or more pharmaceutically acceptable carriers, excipients, buffers, adjuvants, stabilisers, or other materials, as described herein.

The term "pharmaceutically acceptable" as used herein pertains to compounds, materials, compositions, and/or dosage forms which are, within the scope of sound medical judgement, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

Suitable carriers, diluents, excipients, etc. can be found in standard pharmaceutical texts. See, for example, refs. 27 to 29.

The formulations may conveniently be presented in unit dosage form and may be prepared by any methods well known in the art of pharmacy. Such methods include the step of bringing into association the active compound with the carrier which constitutes one or more accessory ingredients. In general, the formulations are prepared by uniformly and intimately bringing into association the active compound with liquid carriers or finely divided solid carriers or both, and then if necessary shaping the product.

Formulations may be in the form of liquids, solutions, suspensions, emulsions, elixirs, syrups, tablets, lozenges, granules, powders, capsules, cachets, pills, ampoules, suppositories, pessaries, ointments, gels, pastes, creams, sprays, mists, foams, lotions, oils, boluses, electuaries, or aerosols.

Formulations suitable for oral administration (e.g., by ingestion) may be presented as discrete units such as capsules, cachets or tablets, each containing a predetermined amount of the active compound; as a powder or granules; as a solution or suspension in an aqueous or non-aqueous liquid; or as an oil-in-water liquid emulsion or a water-in-oil liquid emulsion; as a bolus; as an electuary; or as a paste.

A tablet may be made by conventional means, e.g. compression or molding, optionally with one or more accessory ingredients. Compressed tablets may be prepared by compressing in a suitable machine the active compound in a free-flowing form such as a powder or granules, optionally mixed with one or more binders (e.g. povidone, gelatin, acacia, sorbitol, tragacanth, hydroxypropylmethyl cellulose); fillers or diluents (e.g. lactose, microcrystalline cellulose, calcium hydrogen phosphate); lubricants (e.g. magnesium stearate, talc, silica); disintegrants (e.g. sodium starch glycolate, cross-linked povidone, cross-linked sodium carboxymethyl cellulose); surface-active or dispersing or wetting agents (e.g., sodium lauryl sulfate); and preservatives (e.g., methyl p-hydroxybenzoate, propyl p-hydroxybenzoate, sorbic acid). Molded tablets may be made by molding in a suitable machine a mixture of the powdered compound moistened with an inert liquid diluent. The tablets may optionally be coated or scored and may be formulated so as to provide slow or controlled release of the active compound therein using, for example, hydroxypropylmethyl cellulose in varying proportions to provide the desired release profile. Tablets may optionally be provided with an enteric coating, to provide release in parts of the gut other than the stomach.

Formulations suitable for topical administration (e.g. transdermal, intranasal, ocular, buccal, and sublingual) may be formulated as an ointment, cream, suspension, lotion, powder, solution, past, gel, spray, aerosol, or oil. Alternatively, a formulation may comprise a patch or a dressing such as a bandage or adhesive plaster impregnated with active compounds and optionally one or more excipients or diluents.

Formulations suitable for topical administration in the mouth include losenges comprising the active compound in a flavored basis, usually sucrose and acacia or tragacanth; pastilles comprising the active compound in an inert basis such as gelatin and glycerin, or sucrose and acacia; and mouthwashes comprising the active compound in a suitable liquid carrier.

Formulations suitable for topical administration to the eye also include eye drops wherein the active compound is dissolved or suspended in a suitable carrier, especially an aqueous solvent for the active compound.

Formulations suitable for nasal administration, wherein the carrier is a solid, include a coarse powder having a particle size, for example, in the range of about 20 to about 500 microns which is administered in the manner in which snuff is taken, i.e. by rapid inhalation through the nasal passage from a container of the powder held close up to the nose. Suitable formulations wherein the carrier is a liquid for administration as, for example, nasal spray, nasal drops, or by aerosol administration by nebuliser, include aqueous or oily solutions of the active compound.

Formulations suitable for administration by inhalation include those presented as an aerosol spray from a pressurised pack, with the use of a suitable propellant, such as dichlorodifluoromethane, trichlorofluoromethane, dichoro-tetrafluoroethane, carbon dioxide, or other suitable gases.

Formulations suitable for topical administration via the skin include ointments, creams, and emulsions. When formulated in an ointment, the active compound may optionally be employed with either a paraffinic or a water-miscible ointment base. Alternatively, the active compounds may be formulated in a cream with an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active compound through the skin or other affected areas. Examples of such dermal penetration enhancers include dimethylsulfoxide and related analogues.

When formulated as a topical emulsion, the oily phase may optionally comprise merely an emulsifier (otherwise known as an emulgent), or it may comprises a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Suitable emulgents and emulsion stabilisers include Tween 60, Span 80, cetostearyl alcohol, myristyl alcohol, glyceryl monostearate and sodium lauryl sulphate. The choice of suitable oils or fats for the formulation is based on achieving the desired cosmetic properties, since the solubility of the active compound in most oils likely to be used in pharmaceutical emulsion formulations may be very low. Thus the cream should preferably be a non-greasy, non-staining and washable product with suitable consistency to avoid leakage from tubes or other containers. Straight or branched chain, mono- or dibasic alkyl esters such as diisoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the active compound, such carriers as are known in the art to be appropriate.

Formulations suitable for parenteral administration (e.g., by injection, including cutaneous, subcutaneous, intramuscular, intravenous and intradermal), include aqueous and non-aqueous isotonic, pyrogen-free, sterile injection solutions which may contain antioxidants, buffers, preservatives, stabilisers, bacteriostats, and solutes which render the formulation isotonic with the blood of the intended recipient; and aqueous and non-aqueous sterile suspensions which may include suspending agents and thickening agents, and liposomes or other microparticulate systems which are designed to target the compound to blood components or one or more organs. Examples of suitable isotonic vehicles for use in such formulations include Sodium Chloride Injection, Ringer's Solution, or Lactated Ringer's Injection. Typically, the concentration of the active compound in the solution is from about 1 ng/ml to about 10 µg/ml, for example from about 10 ng/ml to about 1 µg/ml. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets. Formulations may be in the form of liposomes or other microparticulate systems which are designed to target the active compound to blood components or one or more organs.

### Dosage

It will be appreciated that appropriate dosages of the active compounds, and compositions comprising the active compounds, can vary from patient to patient. Determining the optimal dosage will generally involve the balancing of the level of therapeutic benefit against any risk or deleterious side effects of the treatments of the present invention. The selected dosage level will depend on a variety of factors including, but not limited to, the activity of the particular compound, the route of administration, the time of administration, the rate of excretion of the compound, the duration of the treatment, other drugs, compounds, and/or materials used in combination, and the age, sex, weight, condition, general health, and prior medical history of the patient. The amount of compound and route of administration will ultimately be at the discretion of the physician, although generally the dosage will be to achieve local concentrations at the site of action which achieve the desired effect without causing substantial harmful or deleterious side-effects.

Administration *in vivo* can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell being treated, and the subject being treated. Single or multiple administrations can be carried out with the dose level and pattern being selected by the treating physician.

In general, a suitable dose of the active compound is in the range of about 100 µg to about 250 mg per kilogram body weight of the subject per day. Where the active compound is a salt, an ester, prodrug, or the like, the amount administered is calculated on the basis of the parent compound and so the actual weight to be used is increased proportionately.

### Examples

### General Experimental Methods

Thin Layer chromatography was carried out using Merck Kieselgel 60 F₂₅₄ glass backed plates. The plates were visualized by the use of a UV lamp (254 nm). Silica gel 60 (particle sizes 40-63 µm) supplied by E.M.Merck was employed for flash chromatography. ¹H NMR spectra were recorded at 300 MHz on a Bruker DPX-300 instrument. Chemical shifts were referenced relative to tetramethylsilane.

### Purification and identification of library samples

The samples were purified on Gilson LC units. Mobile phase A - 0.1% aqueous TFA, mobile phase B - Acetonitrile; flow rate 6 ml/min; Gradient - typically starting at 90% A/10% B for 1 minute, rising to 97% after 15 minutes, holding for 2 minutes, then back to the starting conditions. Column: Jones Chromatography Genesis 4µm, C18 column, 10 mm x 250 mm. Peak acquisition based on UV detection at 254 nm.

Mass spectra were recorded on a Finnegan LCQ instrument in positive ion mode. Mobile phase A - 0.1% aqueous formic acid. Mobile phase B - Acetonitrile; Flowrate 2 ml/min; Gradient - starting at 95% A/5% B for 1 minute, rising to 98% B after 5 minutes and holding for 3 minutes before returning to the starting conditions. Column: Varies, but always C18 50 mm x 4.6 mm (currently Genesis C18 4 µm. Jones Chromatography). PDA detection Waters 996, scan range 210-400 nm.

### Microwave synthesis

Reactions were carried out using a Personal Chemistry™ Emrys Optimiser microwave synthesis unit with robotic arm. Power range between. 0-300 W at 2.45 GHz. Pressure range between 0-20 bar; temperature increase between 2-5°C/sec; temp range 60-250°C.

### Synthesis of 2,4,7-substituted pyridopyrimidine derivatives

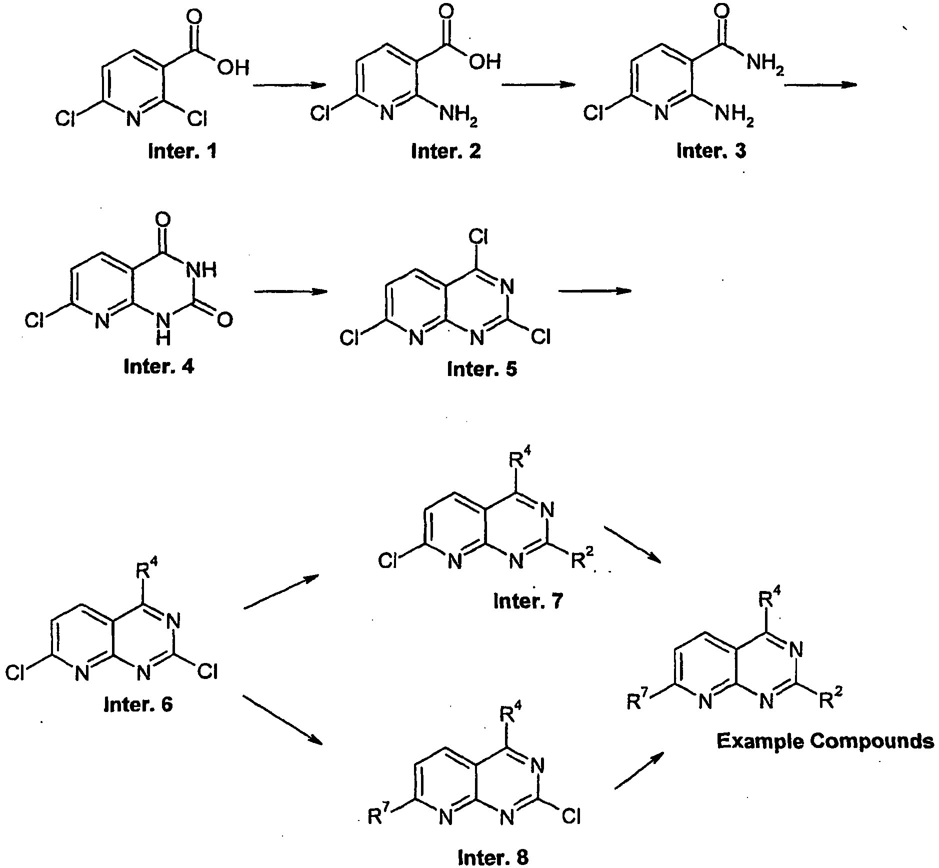

### Intermediates:

### a) 2-Amino-6-chloronicotinic acid (Inter. 2)

To 2,6-dichloronicotinic acid (**Inter. 1**)(1 equiv) was added liquid ammonia (sufficient to make a 0.6M solution of substrate in ammonia). The suspension was sealed in a pressure vessel which was then heated slowly to 130°C. It was noted that at this temperature a pressure of 18 bar was observed. This temperature and pressure was maintained for a further 16 hours whereupon the mixture was cooled to room temperature. The pressure vessel was opened and the reaction poured into ice cold water (1 reaction volume). The resulting solution was acidified to pH 1-2 using concentrated HCl which caused a precipitate to form. The acidic mixture was allowed to warm to room temperature and was stirred like this for a further 30 minutes. The suspension was then extracted with diethyl ether (3 x 400 ml). The combined organic extracts were then filtered and the filtrate concentrated *in vacuo* to give a white solid which was dried further over P₂O₅ to give the title compound (90% yield and 96% pure) in suitably pure form to be used without any further purification. m/z (LC-MS, ESP): 173 [M+H]⁺ R/T = 3.63 mins

### b) 2-Amino-6-chloronicotinamide (Inter. 3)

To a 0.3 M solution of 2-amino-6-chloronicotinic acid (**Inter. 2**)(1 equiv) in anhydrous THF, under an inert atmosphere, was added thionyl chloride (3.3 equiv) in a dropwise fashion. The reaction mixture was stirred at room temperature for 2 hours. After this time the reaction was concentrated *in vacuo* to give a crude yellow solid residue. The crude solid was dissolved in THF (equal to initial reaction volume) and concentrated *in vacuo* again to give a yellow solid residue. The residue was dissolved once more in THF and concentrated as before to give a solid residue which was then dissolved in THF (to give a solution of 0.3M) and ammonia gas bubbled through the solution for 1 hour. The resultant precipitate was removed by filtration and the filtrate concentrated *in vacuo* to give a yellow precipitate which was triturated with water at 50°C then dried to give the title compound (92% yield, 93% purity), suitably clean to be used without any further purification. m/z (LC-MS, ESP): 172 [M+H]⁺ R/T = 3.19 mins

### c) 7-Chloro-1H-pyrido[2,3-d]pyrimidine-2,4-dione (Inter. 4)

To a stirred solution (0.06 M) of 2-amino-6-chloronicotinamide (**Inter. 3)**(1 equiv) in anhydrous toluene under an inert atmosphere was added oxalyl chloride (1.2 equiv) in a dropwise manner. The resulting mixture was then heated to reflux (115°C) for 4 hours whereupon it was cooled and stirred for a further 16 hours. The crude reaction mixture was then concentrated to half its volume *in vacuo* and filtered to give the desired product in suitably pure form (95% yield, 96% purity) to be used without any further purification. m/z (LC-MS, ESP): 196 [M-H]⁻ R/T = 3.22 mins

### d) 2,4,7-Trichloro-pyrido[2,3-d]pyrimidine (Inter.5)

To a stirred 0.5 M suspension of the dione (**Inter. 4**)(1 equiv.) in anhydrous toluene under an inert atmosphere was slowly added diisopropylethylamine (3 equiv.). The reaction mixture was then heated to 70°C for 30 minutes and then cooled to room temperature prior to the addition of POCl₃ (3 equivalents). The reaction was then heated to 100°C for 2.5 hours before being cooled and concentrated *in vacuo* to give a crude slurry which was then suspended in EtOAc and filtered through a thin pad of Celite™. The filtrate was concentrated *in vacuo* to give a brown, oil which was dissolved in CH₂Cl₂ and stirred over silica gel for 30 minutes. After this time the silica was removed by filtration, the filtrate concentrated and the crude residue purified by flash chromatography (SiO₂ to give the title compound in analytically pure form (48% yield, 96% purity). m/z (LC-MS, ESP): 234 [M+H]⁺ R/T = 4.21 mins

### e) 4-Amino-2,7-dichloropyridopyrimidines (Inter. 6)

To a cooled (0-5°C) stirred solution (0.1 M) of the trichloro substrate (**Inter. 5**)(1 equiv.) in CH₂Cl₂ was added diisopropylethylamine (1 equiv.) in a dropwise fashion. The appropriate amine (1 equiv.) was then added to the reaction mixture portionwise over the period of 1 hour. The solution was maintained at room temperature with stirring for a further 1 hour before the mixture was washed with water (2 x 1 reaction volume). The aqueous extracts were combined and extracted with CH₂Cl₂ (2 x 1 reaction volume). The organic extracts were then combined, dried (sodium sulphate), filtered and concentrated *in vacuo* to give an oily residue which solidified upon prolonged drying. The solid was triturated with diethylether and then filtered and the cake washed with cold diethyl ether to leave the title compound in suitable clean form to be used without any further purification.
**Inter. 6a:** 2,7-Dichloro-4-morpholin-4-yl-pyrido[2,3-d]pyrimidine; R⁴= morpholino; (92% yield, 90% purity) m/z (LC-MS, ESP): 285 [M+H]⁺ R/T = 3.90 mins
Inter. 6b: 2,7-Dichloro-4-((2S,6R)-2,6-dimethyl-morpholin-4-yl)-pyrido[2,3-d]pyrimidine; R⁴=(2R,6S)-2,6-Dimethyl-morpholino; (99% yield, 90% purity) m/z (LC-MS, ESP): 313 [M+H]⁺ R/T = 4.39 mins

### f) 2,4-Diamino-7-chloropyridopyrimidines (Inter. 7)

To a solution (0.2 M) of the appropriate dichloro-substrate (**Inter. 6a or 6b**)(1 equiv) in anhydrous dimethyl acetamide under an inert atmosphere was added diisopropylethylamine (1 equiv) followed by the appropriate amine (1 equiv.). The resulting mixture was heated for 48 hours at 70°C before being cooled to ambient temperature. The reaction was diluted with CH₂Cl₂ (1 reaction volume) and then washed with water (3x1 reaction volumes). The organic extract was concentrated *in vacuo* to give a syrup which was dissolved in EtOAC (1 reaction volume) and washed with saturated brine solution before being dried (sodium sulphate) and concentrated *in vacuo* to give an oil. The crude residue was purified by flash chromatography (SiO₂, eluted with EtOAc:Hex (7:3) going to (1:1)) to give the title compound as a yellow solid that was suitably clean to be used without any further purification.
**Inter. 7a**: 7-Chloro-2-((2S,6R)-2,6-dimethyl-morpholin-4-yl)-4-morpholin-4-yl-pyrido[2,3-d]pyrimidine; R⁴ = morpholine, R² = cis-dimethylmorpholine; (45% yield, 85% purity) m/z (LC-MS, ESP): 348 [M+H]⁺ R/T = 4.16 mins
**Inter. 7b**: 7-Chloro-4-(2-methyl-piperidin-1-yl)-2-morpholin-4-yl- -pyrido[2,3-d]pyrimidine; R⁴ = morpholine, R² =2-methylpiperidine; (57% yield, 95% purity) m/z (LC-MS, ESP): 348.1 [M+H]⁺ R/T = 3.42 mins
**Inter. 7c**: 7-Chloro-4-((2S,6R)-2,6-dimethyl-morpholin-4-yl)-2-((S)-3-methyl-morpholin-4-yl)pyrido[2,3-d]pyrimidine (intermediate for compound 11k:) R⁴ = cis-dimethylmorpholine, R²= (S)-3-Methyl-morpholine; (48% yield, 90% purity) m/z (LC-MS, ESP): 378 [M+H]⁺ R/T = 3.74 mins
**Inter. 7d**: 7-Chloro-2-((S)-3-methyl-morpholin-4-yl)-4-morpholin-4-yl-pyrido[2,3-d]pyrimidine (Intermediate for compound 11a): R⁴ = morpholine, R²= (S)-3-Methylmorpholine; (70% yield, 97% purity) m/z (LC-MS, ESP): 350 [M+H]⁺ R/T = 3.44 mins
**Inter. 7e**: 7-Chloro-2-(2-ethyl-piperidin-1-yl)-4-morpholin-4-yl-pyrido[2,3-d]pyrimidine (intermediate for compound 11ay): R⁴ = morpholine, R²= 2-Ethyl-piperidine; (56% yield, 95% purity) m/z (LC-MS, ESP): 362 [M+H]⁺ R/T = 3.78 mins

### g) 4-amino-7-aryl-2-chloropyridopyrimidines (Inter. 8)

To a solution (0.09 M) of the appropriate boronic acid or ester (1 equiv) in water (1 volume) was added the appropriate 2,7-dichloro-4-amino pyridopyrimidine (1 equiv) (**Inter. 6a** or **6b**) potassium carbonate (2.5 equiv) and acetonitrile (1 volume). The mixture was degassed by bubbling nitrogen through the solution while sonicating for 15 minutes before the addition of by tetrakis(triphenylphosphine) palladium (0.03 equiv). The mixture was degassed for a further 5 minutes before heating under an inter atmosphere at 95 °C for 2 hours. Upon completion, the reaction was cooled to room temperature and filtered under vacuum. The filtrate was concentrated *in vacuo* to give a solid residue which was dissolved in CH₂Cl₂ (1 volume) and washed with water (1 volume). The organic extract was then dried (MgSO₄), filtered and *concentrated in vacuo* to give an amorphous solid which was triturated with Et₂O to leave the desired product as a fine powder.
**Inter. 8a** (R⁴=Morpholine, R⁷= 4-chlorophenyl)
2-Chloro-7-(4-chloro-phenyl)-4-morpholin-4-yl-pyrido[2,3-d]pyrimidine; 1H NMR (300 MHz, *Solvent* CDCl₃ δppm 8.29-7.96 (m, 2H), 7.75 (d, *J* = 8.70 1Hz, 1H), 7.54-7.21 (m, 2H), 5.29 (s, 1H), 3.91 (m, 8H).

### Example 1

### R⁷=aryl (Examples 1a-bx)

The appropriate chloro-substrate (**Inter. 7a-e**)(1 equiv.) was dissolved in a toluene/ethanol (1:1) solution (0.02 M). Sodium carbonate (2 equiv.) and the appropriate boronic acid (1 equiv.) were then added followed by tetrakis(triphenylphosphine) palladium (0.1 equiv). The reaction vessel was sealed and the mixture exposed to microwave radiation (140°C, medium absorption setting) for 30 minutes. Upon completion the samples were filtered through a silica cartridge, washed with EtOAc and then concentrated *in vacuo.* The crude residue was then purified by preparative HPLC to give the compounds listed below.

| | **R⁷** | **R⁴** | **R²** | Retention Time (mins) | [m/z] | Purity (%) |
|---|---|---|---|---|---|---|
| **1a** | 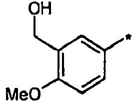 | 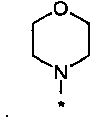 | 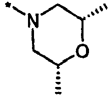 | 7.66 | 466.6 | 89 |
| **1b** | 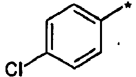 | 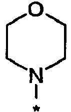 | 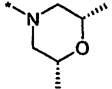 | 9.56 | 440.4 | 85 |
| **1c** | 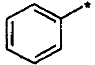 | 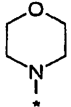 | 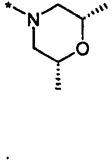 | 8.57 | 406.5 | 88 |
| **1d** | 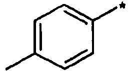 | 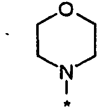 | 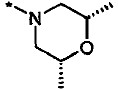 | 4.56 | 420.5 | 89 |
| **1e** | 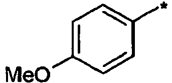 | 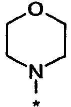 | 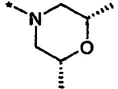 | 4.41 | 436.5 | 98 |
| **1f** | 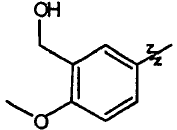 | 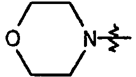 | 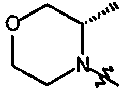 | 4.13 | 452.3 | 99 |
| **1g** | 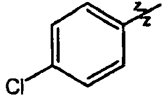 | 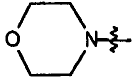 | 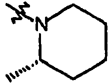 | 4.82 | 424 | 99 |
| **1h** | 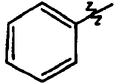 | 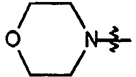 | 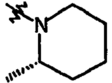 | 4.66 | 390.1 | 96 |
| **1i** | 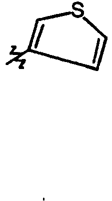 | 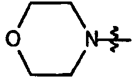 | 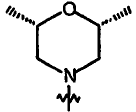 | 8.12 | 412.3 | 93 |
| **1j** | 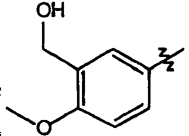 | 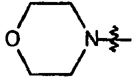 | 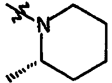 | 4.38 | 450.1 | 96 |
| **1k** | 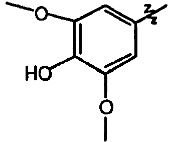 | 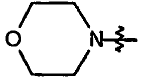 | 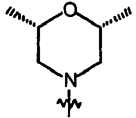 | 7.75 | 482.5 | 89 |
| **1l** | 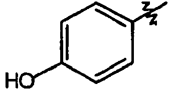 | 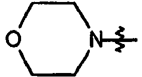 | 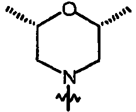 | 7.88 | 422.4 | 99 |
| **1m** | 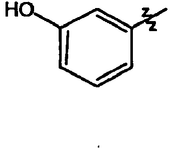 | 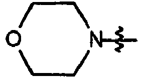 | 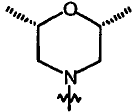 | 9.08 | 478.4 | 99 |
| **1n** | 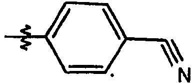 | 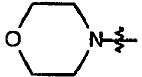 | 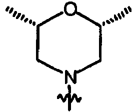 | 8.52 | 431.4 | 95 |
| **1o** | 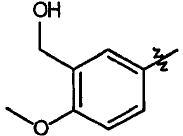 | 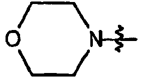 | 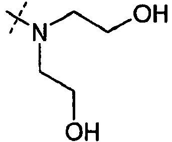 | 3.89 | 456.4 | 93 |
| **1p** | 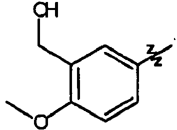 | 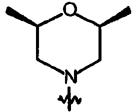 | 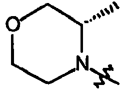 | 4.36 | 480.5 | 100 |
| **1q** | 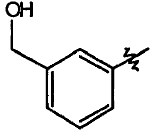 | 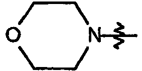 | 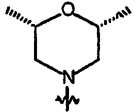 | 4.09 | 436.4 | 100 |
| **1r** | 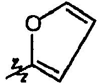 | 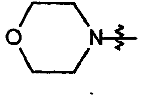 | 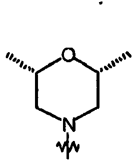 | 4.03 | 396.2 | 100 |
| **1s** | 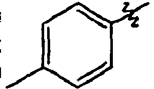 | 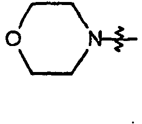 | 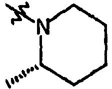 | 10.19 | 404.2 | 97 |
| **1t** | 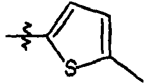 | 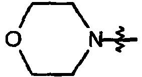 | 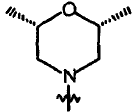 | 4.66 | 426.3 | 98 |
| **1u** | 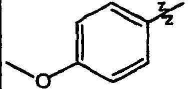 | 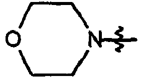 | 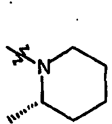 | 4.67 | 420.1 | 95 |
| **1v** | 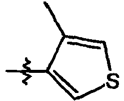 | 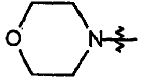 | 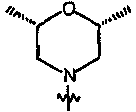 | 4.53 | 426.3 | 99 |
| **1w** | 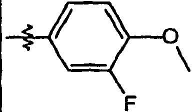 | 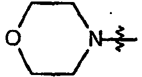 | 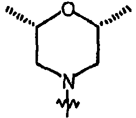 | 9.11 | 454.4 | 100 |
| **1x** | 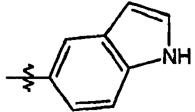 | 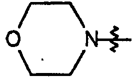 | 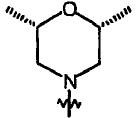 | 8.93 | 445.3 | 97 |
| **1y** | 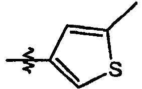 | 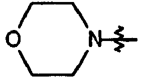 | 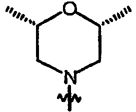 | 4.72 | 426.4 | 99 |
| **1z** | 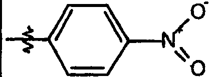 | 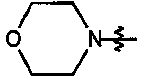 | 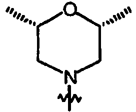 | 8.89 | 451.5 | 80 |
| **1aa** | 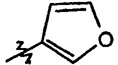 | 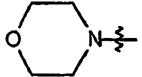 | 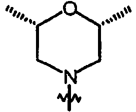 | 7.66 | 396.4 | 99 |
| **1ab** | 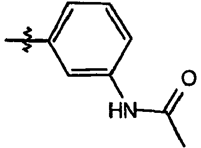 | 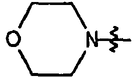 | 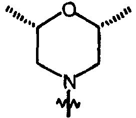 | 7.65 | 463.4 | 85 |
| **1ac** | 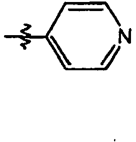 | 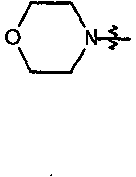 | 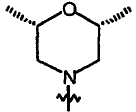 | 3.62 | 407.4 | 99 |
| **1ad** | 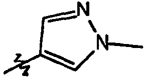 | 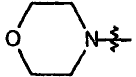 | 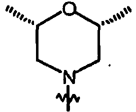 | 7.06 | 410.4 | 99 |
| **1ae** | 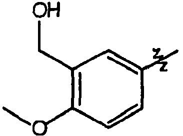 | 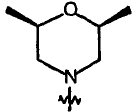 | 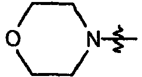 | 4.24 | 466.5 | 100 |
| **1af** | 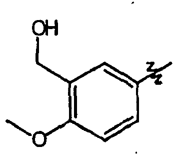 | 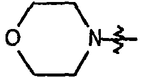 | 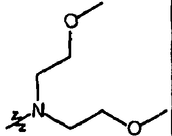 | 4.33 | 484.4 | 96 |
| **1ag** | 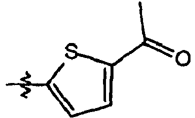 | 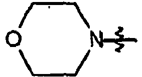 | 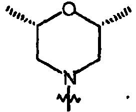 | 4.44 | 454.3 | 98 |
| **1ah** | 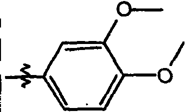 | 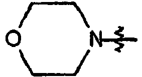 | 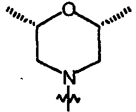 | 8.43 | 466.4 | 97 |
| **1ai** | 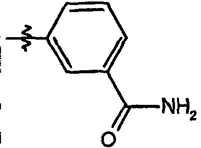 | 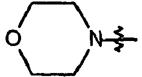 | 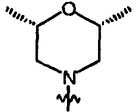 | 6.98 | 449.4 | 100 |
| **1aj** | 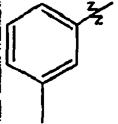 | 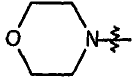 | 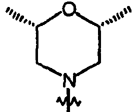 | 9.06 | 420.4 | 90 |
| **1ak** | 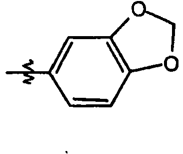 | 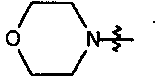 | 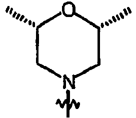 | 8.95 | 450.4 | 96 |
| **1al** | 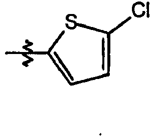 | 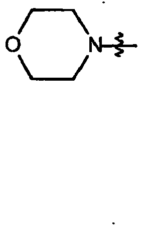 | 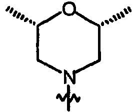 | 9.45 | 446.2 | 88 |
| **1am** | 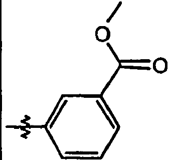 | 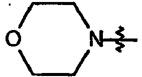 | 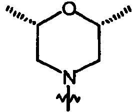 | 8.92 | 464.4 | 99 |
| **1an** | 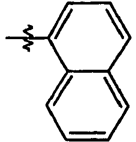 | 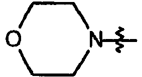 | 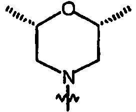 | 9.85 | 456.4 | 97 |
| **1ao** | 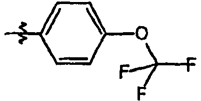 | 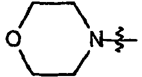 | 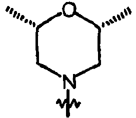 | 10.42 | 490.4 | 97 |
| **1ap** | 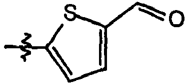 | 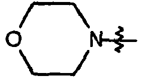 | 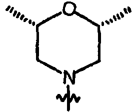 | 4.41 | 440.3 | 99 |
| **1aq** | 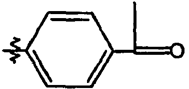 | 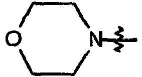 | 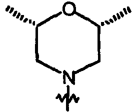 | 8.4 | 448.5 | 97 |
| **1ar** | 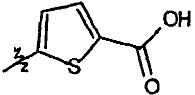 | 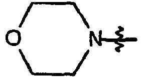 | 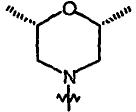 | 7.66 | 456.4 | 99 |
| **1as** | 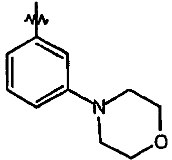 | 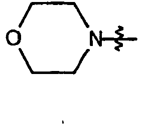 | 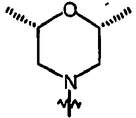 | 8.46 | 491.5 | 99 |
| **1at** | 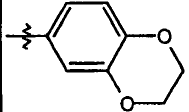 | 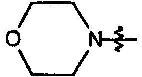 | 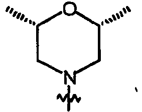 | 8.97 | 464.4 | 99 |
| **1au** | 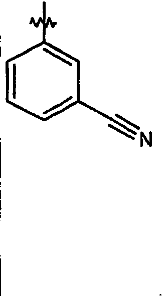 | 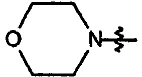 | 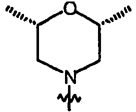 | 8.35 | 431.5 | 93 |
| **1av** | 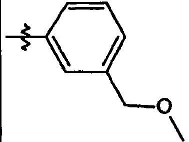 | 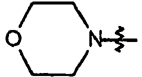 | 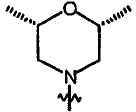 | 8.59 | 450.4 | 99 |
| **1aw** | 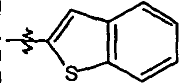 | 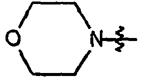 | 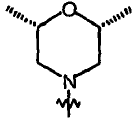 | 10.1 | 462.4 | 98 |
| **1ax** | 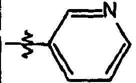 | 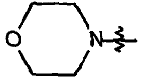 | 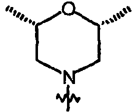 | 6.03 | 407.4 | 100 |
| **1ay** | 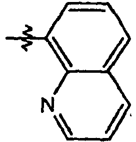 | 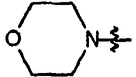 | 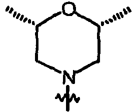 | 7.63 | 457.5 | 99 |
| **1az** | 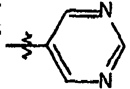 | 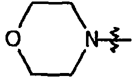 | 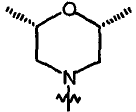 | 6.4 | 408.4 | 99 |
| **1ba** | 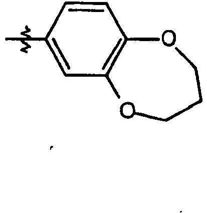 | 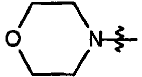 | 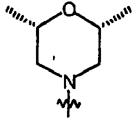 | 9.08 | 478.4 | 99 |
| **1bb** | 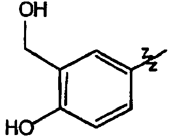 | 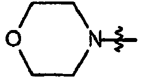 | 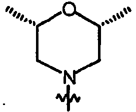 | 3.95 | 452.3 | 95 |
| **1bc** | 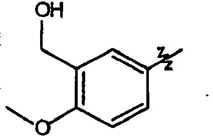 | 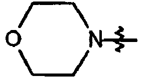 | 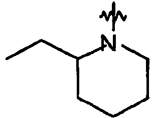 | 9.01 | 464.4 | 99 |
| **1bd** | 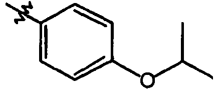 | 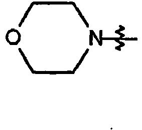 | 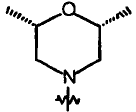 | 4.69 | 464.3 | 98 |
| **1be** | 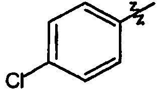 | 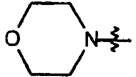 | 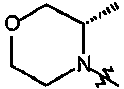 | 4.31 | 426.3 | 100 |
| **1bf** | 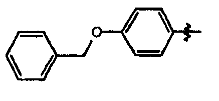 | 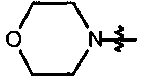 | 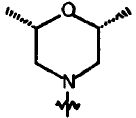 | 4.9 | 512.4 | 100 |
| **1bg** | 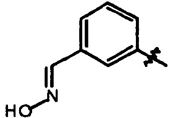 | 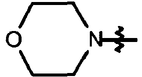 | 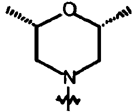 | 7.82 | 449.5 | 90 |
| **1bh** | 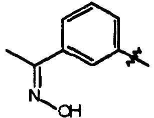 | 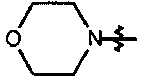 | 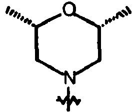 | 8.18 | 463.5 | 89 |
| **1bi** | 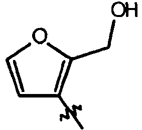 | 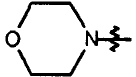 | 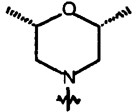 | 3.89 | 426.4 | 100 |
| **Ref. Cpd. 1bj** | 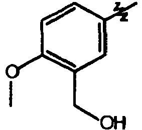 | 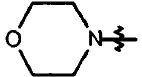 | 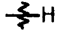 | 5.45 | 353.2 | 100 |
| **1bk** | 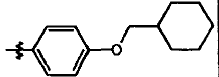 | 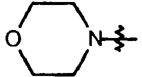 | 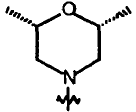 | 5.43 | 518.4 | 96 |
| **1bl** | 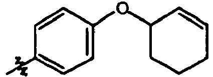 | 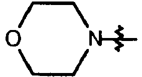 | 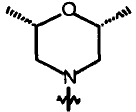 | 11.28 | 502.3 | 95 |

| | | | | | | |
|---|---|---|---|---|---|---|
| Ref. Cpd. = Reference compound (not considered an Example) | | | | | | |

| | **R⁷** | **R⁴** | **R²** | Retention Time (mins) | [m/z] | Purity (%) |
|---|---|---|---|---|---|---|
| **1bm** | 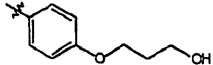 | 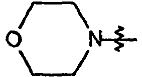 | 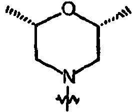 | 4.13 | 480.4 | 97 |
| **1bn** | 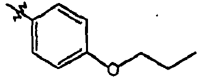 | 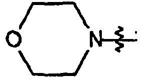 | 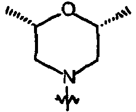 | 4.79 | 464.4 | 97 |
| **1bo** | 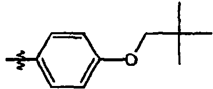 | 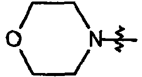 | 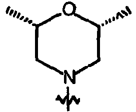 | 5.2 | 492.4 | 93 |
| **1bp** | 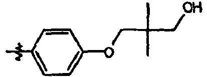 | 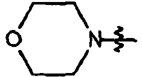 | 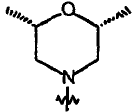 | 4.5 | 508.4 | 90 |
| **1bq** | 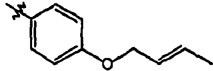 | 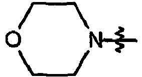 | 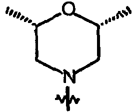 | 4.81 | 476.4 | 100 |
| **1br** | 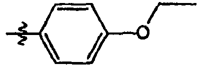 | 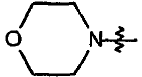 | 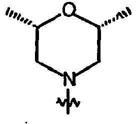 | 9.44 | 450.3 | 98 |
| **1bs** | 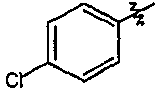 | 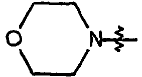 | 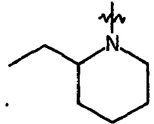 | 4.65 | 424.3 | 99 |
| **1bt** | 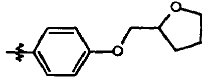 | 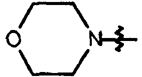 | 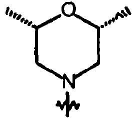 | 9.26 | 506.3 | 98 |
| **1bu** | 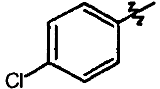 | 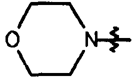 | 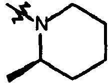 | 4.78 | 438.4 | 99 |
| **1bv** | 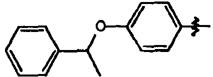 | 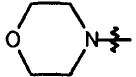 | 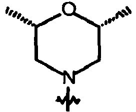 | 11.43 | 526.3 | 99 |
| **1bw** | 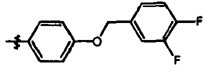 | 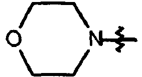 | 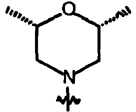 | 11.54 | 548.3 | 98 |
| **1bx** | 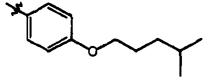 | 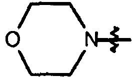 | 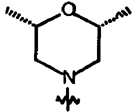 | 5.37 | 506.4 | 95 |

Example 1bj:- 1H NMR (300 MHz, *Solvent* CDCl₃) δ ppm 8.72 (s, 1H), 8.46 (d, J = 8.70), 8.37 d, 2.34 Hz, 1H), 8.17 (dd, J = 8.60, 2.34 Hz, 1H), 8.02 (d, J = 8.77 Hz, 1H), 7.14 (d, J = 8.68 Hz, 1H), 4.59 (s, 1H), 3.95-3.71 (m, 8H), 2.50 (m, 3H).

### Reference Compound 2

### R⁷=Substituted Amino (Reference Compounds 2a-2bg)

To a solution of the appropriate chloro-substrate (**Inter. 7a-e**)(1 equiv.) in 1,4-dioxane (0.04M) was added the appropriate amine (1.2 equiv.), cesium carbonate (2 equiv.), Xantphos™ and tris(dibenzylideneacetone)dipalladium(0). The reaction vessel was sealed and exposed to heating under the influence of microwave radiation (normal absorption setting, 150°C, 20 minutes). The crude reaction mixture was then purified by preparative HPLC to give the compounds listed below.

| | **R⁴** | **R²** | **R⁷** | **Purity %** | **m/z [MH]⁺** | **RT (mins)** |
|---|---|---|---|---|---|---|
| **2a** | 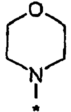 | 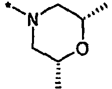 | 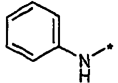 | 95 | 421 | 4.65 |
| **2b** | 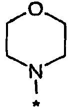 | 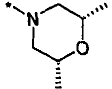 | 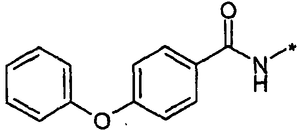 | 96 | 513 | 4.12 |
| **2c** | 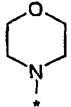 | 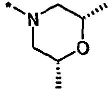 | 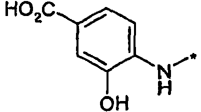 | 95 | 451 | 3.6 |
| **2d** | 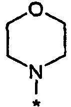 | 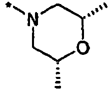 | 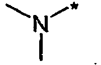 | 98 | 373.1 | 7.18 |
| **2e** | 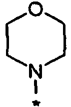 | 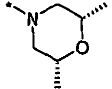 | 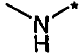 | 83 | 389.2 | 3.73 |
| **2f** | 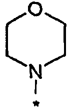 | 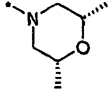 | 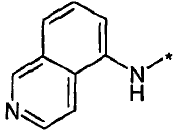 | 100 | 472.2 | 3.49 |
| **2g** | 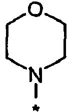 | 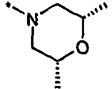 | 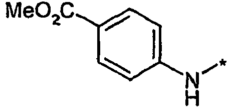 | 96 | 479.5 | 8.81 |
| **2h** | 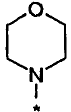 | 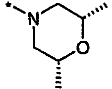 | 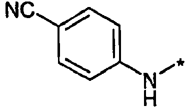 | 85 | 446.5 | 8.34 |
| **2i** | 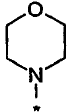 | 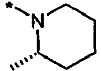 | 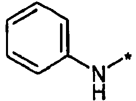 | 91 | 405.5 | 9.41 |
| **2j** | 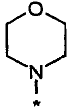 | 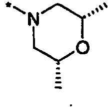 | 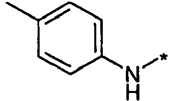 | 99 | 435.5 | 4.61 |
| **2k** | 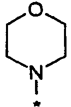 | 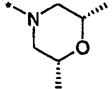 | 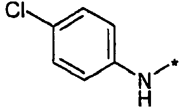 | 86 | 455.5 | 9.68 |
| **2l** | 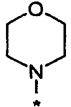 | 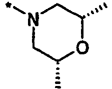 | 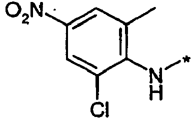 | 96 | 514.5 | 9.38 |
| **2m** | 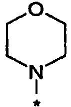 | 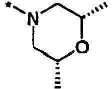 | 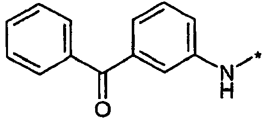 | 100 | 525.6 | 10.46 |
| **2n** | 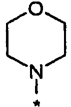 | 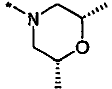 | 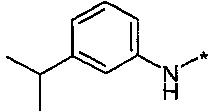 | 96 | 463.6 | 10.62 |
| **2o** | 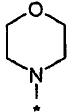 | 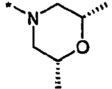 | 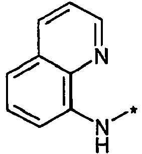 | 92 | 472.5 | 9.44 |
| **2p** | 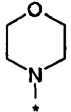 | 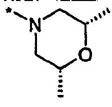 | 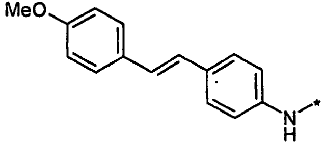 | 98 | 553.6 | 11.83 |
| **2q** | 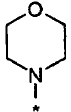 | 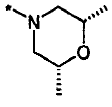 | 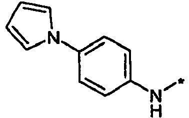 | 97 | 486.6 | 10.22 |
| **2r** | 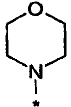 | 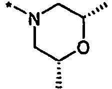 | 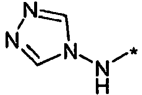 | 100 | 412.4 | 3.5 |
| **2s** | 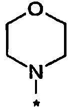 | 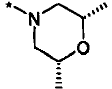 | 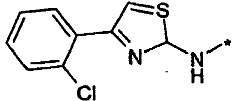 | 92 | 538.2 | 10.59 |
| **2t** | 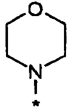 | 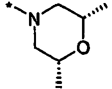 | 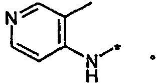 | 86 | 436.2 | 3.44 |
| **2u** | 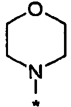 | 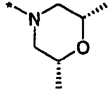 | 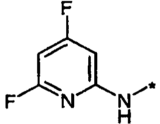 | 81 | 459.4 | 4.26 |
| **2v** | 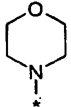 | 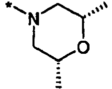 | 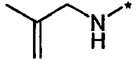 | 88 | 399.6 | 7.96 |
| **2w** | 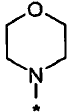 | 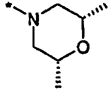 | 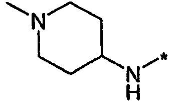 | 90 | 442.6 | 5.13 |
| **2x** | 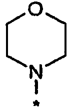 | 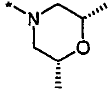 | 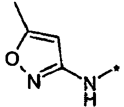 | 94 | 426.4 | 8.52 |
| **2y** | 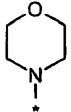 | 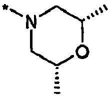 | 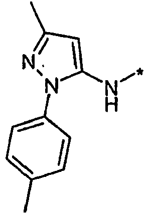 | 95 | 515.6 | 8.71 |
| **2z** | 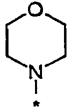 | 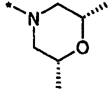 | 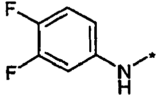 | 92 | 445.4 | 9.49 |
| **2aa** | 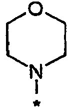 | 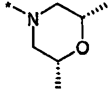 | 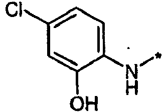 | 98 | 471.5 | 9.04 |
| **2ab** | 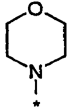 | 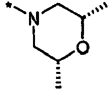 | 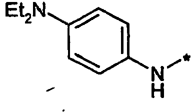 | 69 | 492.6 | 6.25 |
| **2ac** | 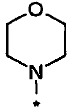 | 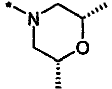 | 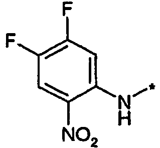 | 73 | 502.5 | 9.48 |
| **2ad** | 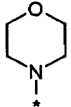 | 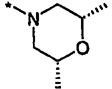 | 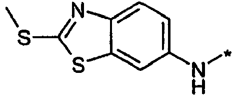 | 97 | 524.2 | 9.68 |
| **2ae** | 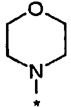 | 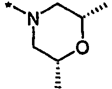 | 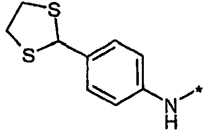 | 96 | 525.4 | 4.79 |
| **2af** | 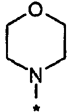 | 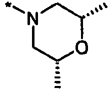 | 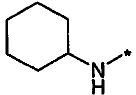 | 98 | 427.5 | 4.55 |
| **2ag** | 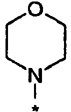 | 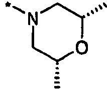 | 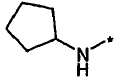 | 100 | 413.4 | 4.36 |
| **2ah** | 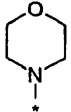 | 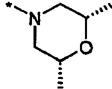 | 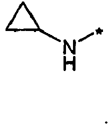 | 95 | 385.3 | 3.88 |
| **2ai** | 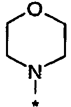 | 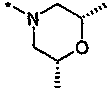 | 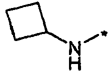 | 89 | 399.5 | 8.13 |
| **2aj** | 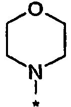 | 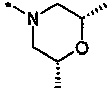 | 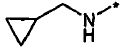 | 94 | 399.5 | 8.18 |
| **2ak** | 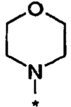 | 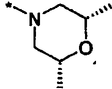 | 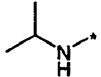 | 91 | 378.4 | 7.81 |
| **2al** | 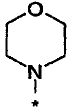 | 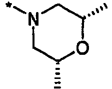 | 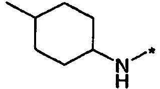 | 99 | 441.6 | 9.95 |
| **2am** | 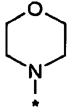 | 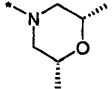 | 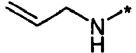 | 92 | 385.5 | 7.46 |
| **2an** | 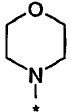 | 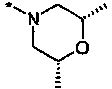 | 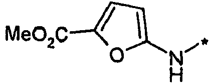 | 99 | 469.4 | 4.31 |
| **2ao** | 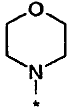 | 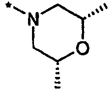 | 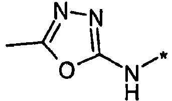 | 98 | 427.5 | 3.91 |
| **2ap** | 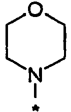 | 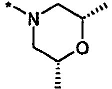 | 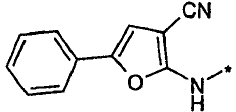 | 83 | 512.2 | 4.97 |
| **2aq** | 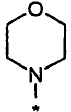 | 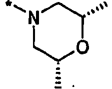 | 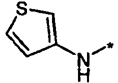 | 94 | 427.1 | 94 |
| **2ar** | 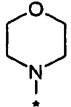 | 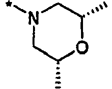 | 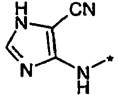 | 99 | 436.1 | 3.9 |
| **2as** | 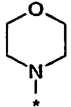 | 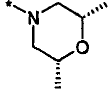 | 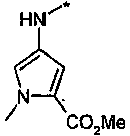 | 98 | 482.1 | 4.36 |
| **2at** | 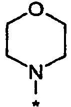 | 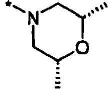 | 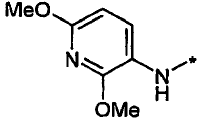 | 98 | 510.5 | 8.96 |
| **2au** | 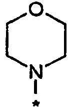 | 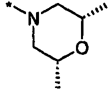 | 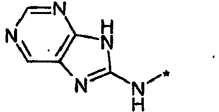 | 98 | 463.6 | 4.03 |
| **2av** | 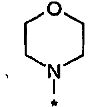 | 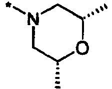 | 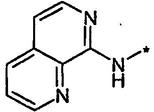 | 99 | 473.6 | 4.12 |
| **2aw** | 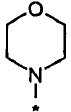 | 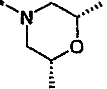 | 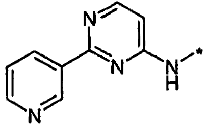 | 99 | 500.6 | 4.17 |
| **2ax** | 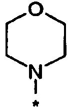 | 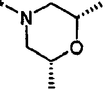 | 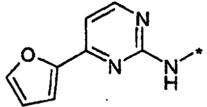 | 82 | 489.5 | 4.84 |
| **2ay** | 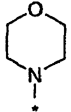 | 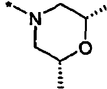 | 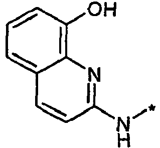 | 98 | 488.5 | 4.56 |
| **2az** | 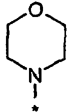 | 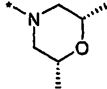 | 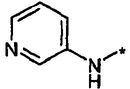 | 100 | 422.5 | 3.73 |
| **2ba** | 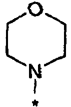 | 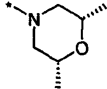 | 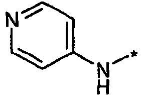 | 100 | 422.5 | 3.6 |
| **2bb** | 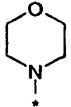 | 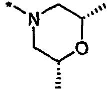 | 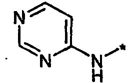 | 99 | 423.5 | 3.94 |
| **2bc** | 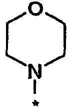 | 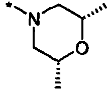 | 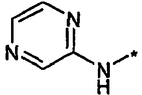 | 97 | 423.5 | 4.11 |
| **2bd** | 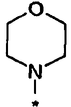 | 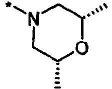 | 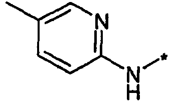 | 100 | 436.5 | 4.00 |
| **2be** | 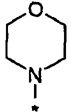 | 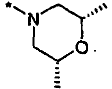 | 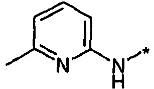 | 99 | 436.5 | 4.49 |
| **2bf** | 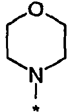 | 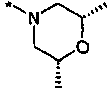 | 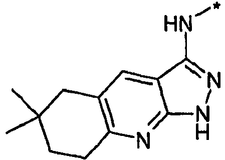 | 95 | 544.6 | 5.23 |

| | **R⁴** | **R²** | **R⁷** | **Purity %** | **m/x [MH]⁺** | **RT (mins)** |
|---|---|---|---|---|---|---|
| **2bg** | 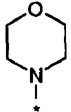 | 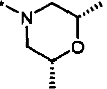 | 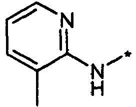 | 99 | 436.5 | 3.94 |
| **2bf** | 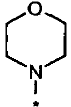 | 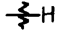 | 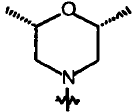 | 5.35 | 330.3 | 96 |

Reference Compound 2bf:- 1H NMR (300 MHz, *Solvent* CDCl₃) δ ppm 8.50 (s, 1H), 8.05 (d, J=9.32Hz, 1H), 7.10 (d, J = 9.37 Hz, 1H), 4.46 (d, J = 12.95 Hz, 1H), 3.82-3.50 (m, 11H), 3.31 (s, 1H), 2.70-2.43 (m, 4H), 1.17 (d, J = 6.30 Hz, 1H)

### Reference Compound 3

### R⁷ = NH₂ (Reference Compound 3a-b)

To a solution of the appropriate chloro-substrate (**Inter. 7a-e**)(1 equiv) in isopropanol (0.6 M) was added concentrated aqueous ammonia solution (5 volumes). The reaction vessel was sealed and heated under the influence of microwave radiation (high absorption setting, 150°C, 30 minutess). The reaction mixture was then cooled, diluted with water (3 volumes) and extracted with EtOAc (2x3 volumes). The combines organic extracts were dried (sodium sulphate), filtered and concentrated *in vacuo* to give a crude residue that was further purified by flash chromatography (SiO₂) (Eluent-EtOAc/MeOH (95:5) going to (9:1)) to give the compounds below in analytically pure form.

| | **R⁴** | **R²** | **R⁷** | **Purity %** | **m/z [MH]⁺** | **RT (mins)** |
|---|---|---|---|---|---|---|
| **3a** | 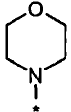 | 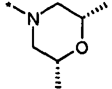 | 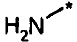 | 95 | 345.2 | 3.86 |
| **3b** | 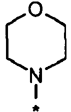 | 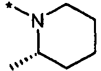 | 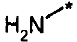 | 91 | 329.4 | 7.33 |

### Reference Compound 4

### R⁷=Urea (Reference Compound 4a-i)

To a solution (0.02M) of the appropriate amino-substrate (e.g. **Reference Compounde 3a** or **3b**)(1 equiv.) in anhydrous THF was added the appropriate isocyanate (2 equiv.). The reaction vessel was sealed and heated to 60°C for 1 hour. The crude reaction mixture was then purified by preparative HPLC to give the compounds below.

| | **R⁴** | **R²** | **R⁷** | **Purity %** | **m/z [MH]⁺** | **RT (mins)** |
|---|---|---|---|---|---|---|
| **4a** | 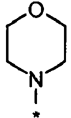 | 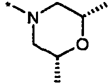 | 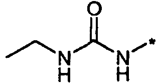 | 99 | 416.5 | 3.8 |

### Reference Compound 5

### R⁷=Amido (Reference Compounds 5a-g)

To a solution (0.02M) of the appropriate amine-substrate (**Reference Compound 3a** or **3b**)(1 equiv) in anhydrous CH₂Cl₂ was added triethylamine (2 equivalents) and the appropriate acid chloride (2 equivalents). The mixture was stirred at room temperature for 1 hour whereupon the solvent was allowed to evaporate at atmospheric pressure and the crude residue purified by preparative HPLC to give the compounds below.

| | **R⁴** | **R²** | **R⁷** | **Purity %** | **m/z [MH]⁺** | **RT (mins)** |
|---|---|---|---|---|---|---|
| **5a** | 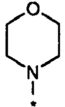 | 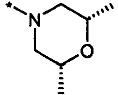 | 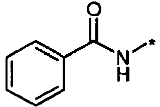 | 95 | 402.2 | 4.49 |
| **5b** | 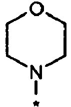 | 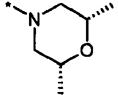 | 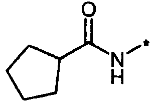 | 96 | 441.2 | 4.16 |
| **5c** | 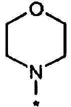 | 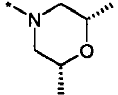 | 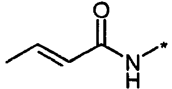 | 100 | 413.2 | 3.89 |
| **5d** | 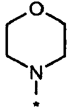 | 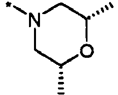 | 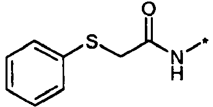 | 98 | 495.2 | 4.34 |
| **5e** | 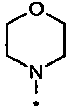 | 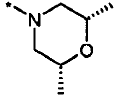 | 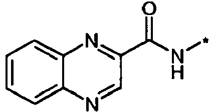 | 83 | 501.2 | 4.28 |
| **5f** | 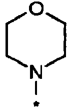 | 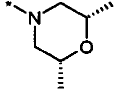 | 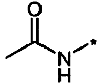 | 95 | 387.2 | 6.04 |
| **5g** | 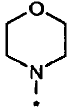 | 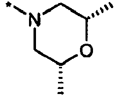 | 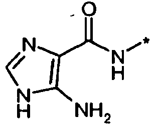 | 99 | 454.5 | 3.85 |

### Reference Compound 6

### R⁷=Sulfonamido

To a solution (0.02M) of the appropriate amine-substrate (**Reference Compound 3a** or **3b**)(1 equiv.) in anhydrous CH₂Cl₂ was added triethylamine (2 equiv.) and the appropriate sulfonyl chloride (2 equiv.). The mixture was stirred at room temperature for 1 hour whereupon the solvent was allowed to evaporate at atmospheric pressure and the crude mixture purified by preparative HPLC to give the desired product.

| | **R⁴** | **R²** | **R⁷** | **Purity %** | **m/z [MH]⁺** | **RT (mins)** |
|---|---|---|---|---|---|---|
| **6** | 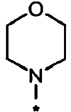 | 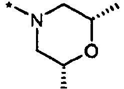 | 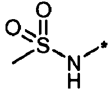 | 85 | 423.5 | 3.87 |

### Reference Compound 7

### R⁷= alkoxy

To a solution of the appropriate chloro-substrate (**Inter. 7a-e**) (1 equiv.) in the appropriate alcohol solvent (0.16 M) was added potassium carbonate (5 equiv.). The reaction vessel was sealed and heated under the influence of microwave radiation (160°C, high absorption setting, 30 minutes). The reaction mixture was then concentrated to dryness and purified by preparative HPLC to give the desired product.

| | **R⁴** | **R²** | **R⁷** | **Purity %** | **m/z [MH]⁺** | **RT (mins)** |
|---|---|---|---|---|---|---|
| **7** | 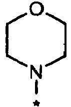 | 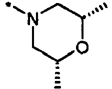 | 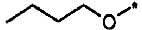 | 100 | 402.2 | 4.49 |

### Example 8

### R⁷= 4-alkoxy-3-aminomethyl-phenyl (Examples 8a - 8bh)

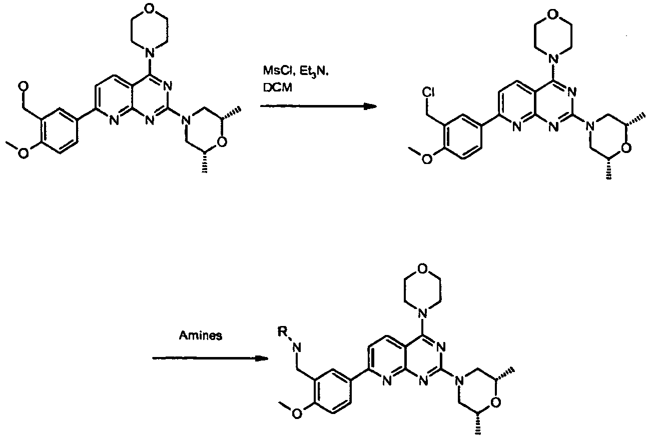

To a 0.1 M solution of Example 1a (1 equiv.) in CH₂Cl₂ was added Et₃N (1 equiv.) followed by methanesulfonyl chloride (1.1 equiv.) which was added dropwise. The reaction was stirred under an inert atmosphere for 90 minutes whereupon it was quenched with water (2x1 volume), the organis extract separated and dried (MgSO₄) filtered and concentrated *in vacuo* The crude yellow gum was then diluted in diethyl ether and stirred vigorously. The resultant yellow precipitate was then collected by filtration to give the title compound (98%) in suitable clean form to be used without further purification.

### 7-(3-Chloromethyl-4-methoxy-phenyl)-2-((2S,6R)-2,6-dimethyl-morpholin-4-yl)-4-morpholin-4-yl-pyrido[2,3-d]pyrimidine; (98.9% yield, 94% purity) m/z (LC-MS, ESP): Did not ionise [M+H]⁺ R/T = 3.98 mins

To a 0.03 M solution of 7-(3-Chloromethyl-4-methoxy-phenyl)-2-((2S,6R)-2,6-dimethyl-morpholin-4-yl)-4-morpholin-4-yl pyrido[2,3-d]pyrimidine (1 equiv.) in dimethylformamide was added potassium carbonate (2.6 equiv.) followed by the triethylamine (1 equiv.) and finally the appropriate amine (1.1 equiv.). The reaction mixture was then heated to 40°C for 16 hours whereupon it was purified by preparative HPLC to give the desired product.

| | | Purity (%) | Retention time (mins) | [m/z] |
|---|---|---|---|---|
| **8a** | 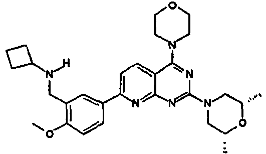 | 99 | 3.86 | 519.5 |
| **8b** | 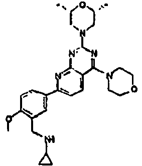 | 77 | 6.32 | |
| **8c** | 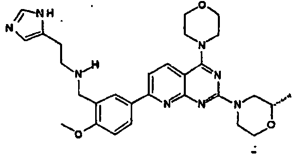 | 98 | 5.29 | 559.5 |
| **8d** | 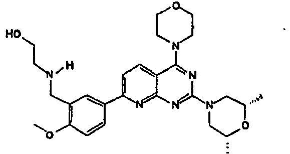 | 98 | 5.96 | 509.4 |
| **8e** | 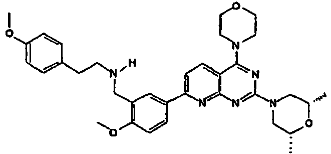 | 87 | 7.56 | 599.5 |
| **8f** | 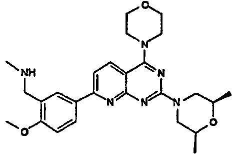 | 100 | 6 | 479.5 |
| **8g** | 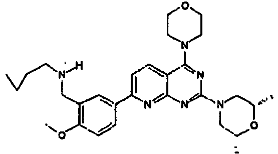 | 99 | 3.88 | 519.5 |
| **8h** | 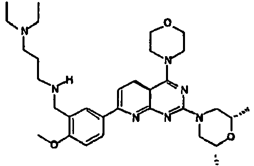 | 99 | 3.57 | 578.5 |
| **8i** | 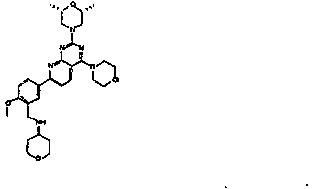 | 99 | 6.21 | 549.6 |
| **8j** | 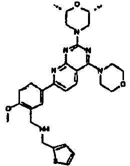 | 95 | 6.97 | 561.5 |
| **8k** | 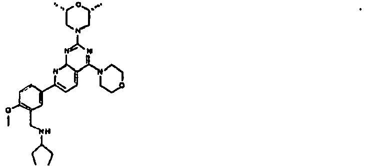 | 99 | 6.8 | 533.5 |
| **8l** | 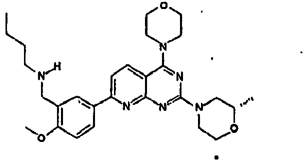 | 99 | 3.95 | 521.5 |
| **8m** | 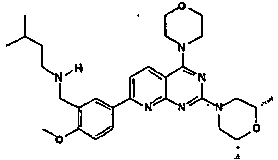 | 99 | 4.04 | 535.4 |
| **8n** | 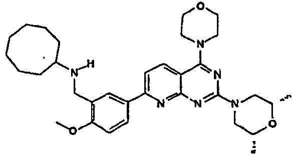 | 99 | 4.18 | 575.5 |
| **8o** | 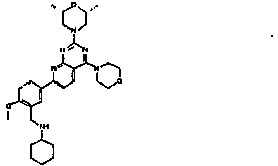 | 99 | 7.14 | 547.5 |
| **8p** | 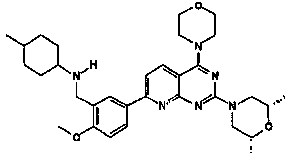 | 75 | 7.75 | 561.5 |
| **8q** | 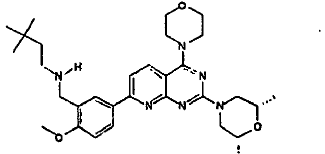 | 99 | 4.12 | 549.4 |
| **8r** | 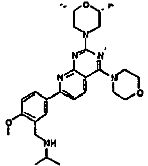 | 93 | 6.36 | 507.6 |
| **8s** | 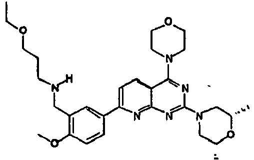 | 100 | 6.71 | 551.5 |
| **8t** | 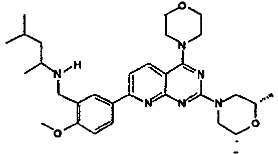 | 99 | 4.11 | 549.5 |
| **8u** | 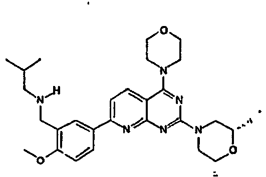 | 98 | 6.73 | 521.5 |
| **8v** | 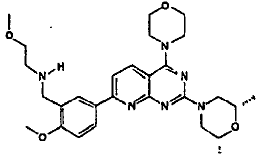 | 99 | 6.25 | 523.4 |
| **8w** | 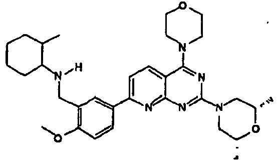 | 99 | 4.1 | 561.5 |
| **8x** | 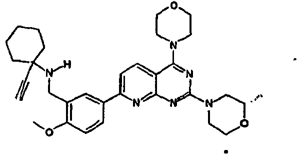 | 99 | 4.14 | 571.4 |
| **8y** | 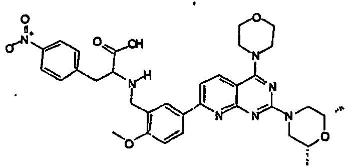 | 99 | 4.12 | 658.4 |
| **8z** | 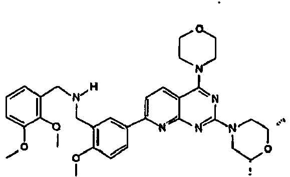 | 99 | 4.07 | 615.5 |
| **8aa** | 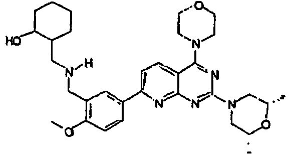 | 99 | 3.73 | 577.4 |
| **8ab** | 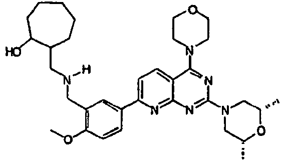 | 99 | 3.81 | 591.4 |
| **8ac** | 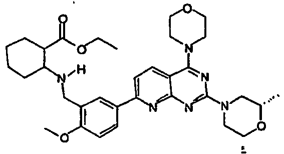 | 99 | 3.92 | 619.4 |
| **8ad** | 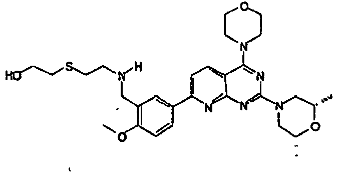 | 99 | 3.55 | 569.3 |
| **8ae** | 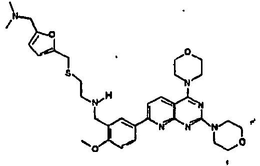 | 99 | 3.42 | 662.3 |
| **8af** | 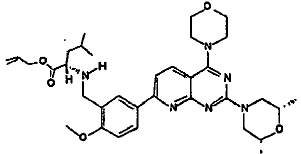 | 99 | 4.01 | 619.4 |
| **8ag** | 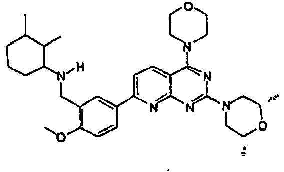 | 99 | 3.96 | 575.3 |
| **8ah** | 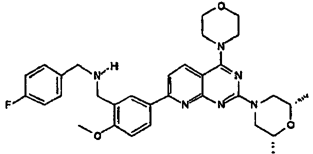 | 99 | 4.12 | 573.5 |
| **8ai** | 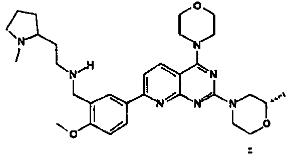 | 99 | 3.61 | 576.5 |
| **8aj** | 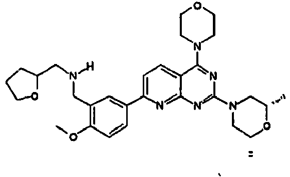 | 99 | 3.91 | 549.4 |
| **8ak** | 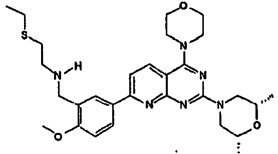 | 99 | 4.01 | 553.4 |
| **8al** | 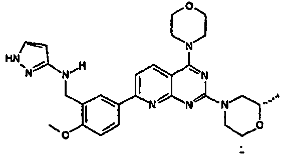 | 83 | 4.18 | 531.4 |
| **8am** | 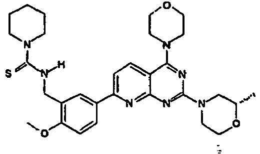 | | | |
| **8an** | 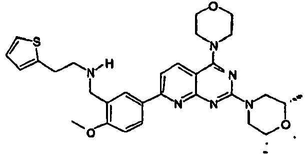 | 92 | 4.10 | 575.4 |
| **8ao** | 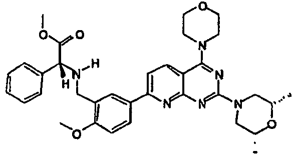 | 97 | 4.17 | 613.4 |
| **8ap** | 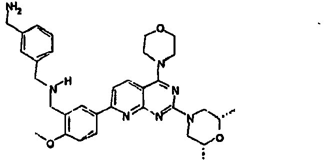 | 99 | 3.64 | 584.5 |
| **8aq** | 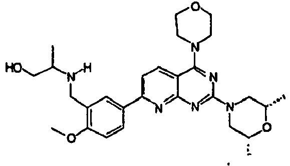 | 99 | 3.79 | 523.4 |
| **8ar** | 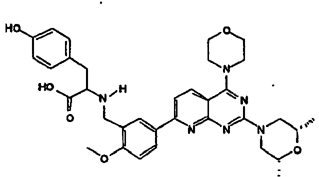 | 98 | 4.10 | 629.5 |
| **8as** | 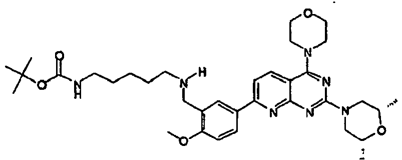 | 99 | 4.20 | 650.5 |
| **8at** | 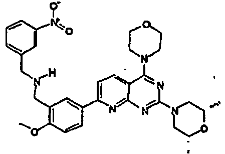 | 99 | 4.11 | 600.4 |
| **8au** | 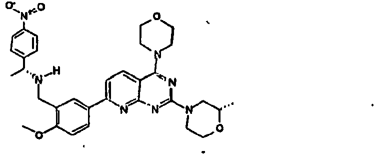 | 86 | 4.20 | 614.4 |
| **8av** | 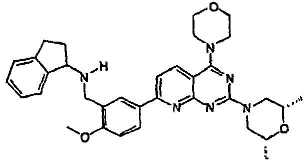 | 94 | 4.20 | 581.5 |
| **8aw** | 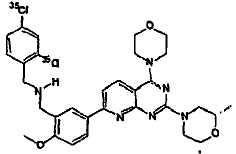 | 99 | 4.33 | 623.4 |
| **8ax** | 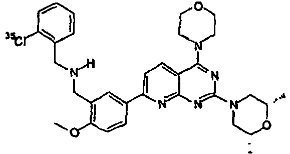 | 99 | 4.17 | 589.4 |
| **8ay** | 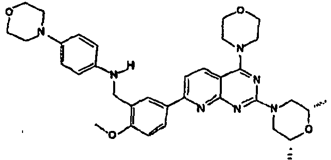 | 83 | 4.22 | 626.5 |
| **8az** | 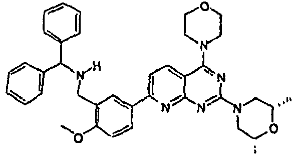 | 96 | 4.41 | 631.5 |
| **8ba** | 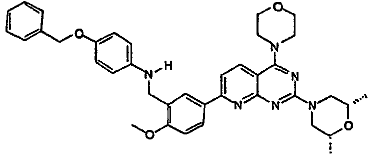 | 89 | 5.14 | 647.5 |
| **8bb** | 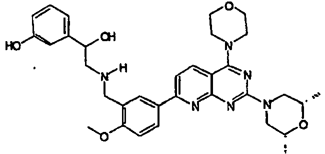 | 99 | 3.95 | 601.4 |
| **8bc** | 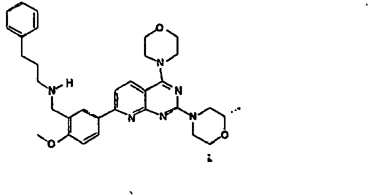 | 99 | 4.27 | 583.4 |
| **8bd** | 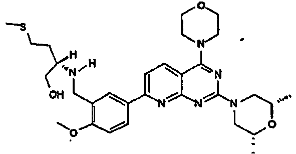 | 99 | 3.97 | 583.4 |
| **8be** | 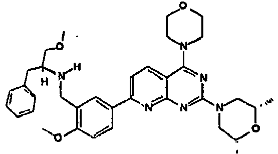 | 99 | 4.29 | 613.5 |
| **8bf** | 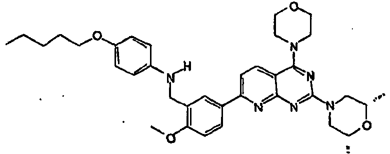 | 74 | 10.36 | 627.6 |
| **8bg** | 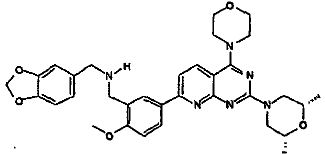 | 93 | 7.13 | 599.2 |

### Example 9

### R²=aryl ; R⁷=aryl (Examples 9a - 9ae)

To the appropriate 2-chloro-pyridopyrimidine (**Inter. 8a**)(1 equiv) in acetonitril/water (1:1 ratio - 0.025 M) was added the appropriate boronic acid or ester (1 equiv), potassium carbonate (3.5 equiv) and tetrakis(triphenylphosphine) palladium (0.05 equiv). The mixture was heated to 95 °C under an inert atmosphere for 2 hours whereupon it was cooled to room temperature. The reaction was then filtered through a thin silica pad, which was washed with 1:1 mixture MeOH/CH₂Cl₂ (1 volume), concentrated *in vacuo* and then purified by preparative HPLC to give the desired product (9a-9ae)

| | | Purity (%) | Retention time (mins) | [m/z] |
|---|---|---|---|---|
| **9a** | 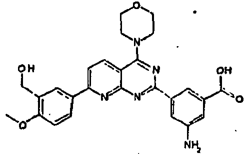 | 92 | 7.17 | 488.3 |
| **9b** | 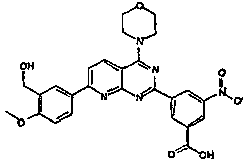 | 86 | 10.16 | 518.3 |
| **9c** | 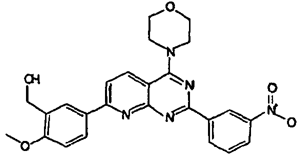 | 95 | 10.72 | 474.3 |
| **9d** | 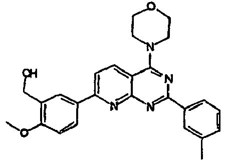 | 99 | 4.58 | 443.4 |
| **9e** | 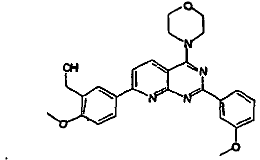 | 95 | 4.53 | 459.4 |
| **9f** | 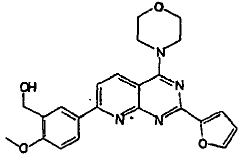 | 97 | 7.21 | 419.3 |
| **9g** | 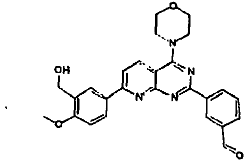 | 83 | 4.35 | 457.3 |
| **9h** | 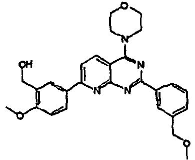 | 97 | 4.42 | 473.5 |
| **9i** | 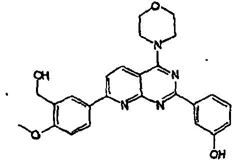 | 97 | 4.01 | 445.3 |
| **9j** | 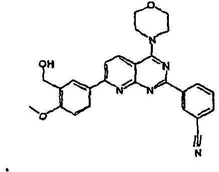 | 91 | 4.70 | 454.3 |
| **9k** | 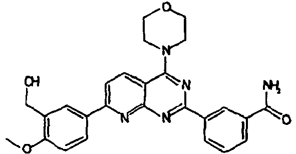 | 79 | 7.19 | 472.3 |
| **9l** | 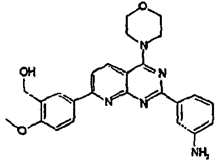 | 89 | 7.24 | 444.3 |
| **9m** | 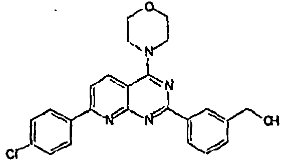 | 98 | 4.45 | 433.2 |
| **9n** | 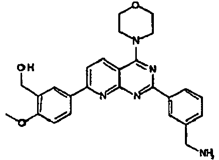 | 99 | 3.54 | 458.3 |
| **9o** | 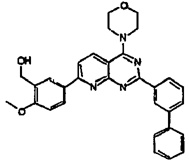 | 96 | 11.05 | 505.3 |
| **9p** | 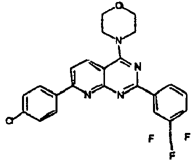 | 98 | 7.49 | 471.2 |
| **9q** | 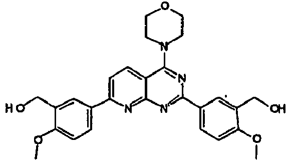 | 92 | 4.63 | 489.3 |
| **9r** | 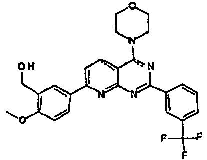 | 99 | 11.32 | 497.3 |
| **9s** | 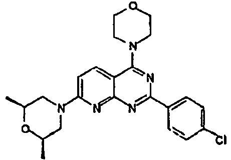 | | | |
| **9t** | 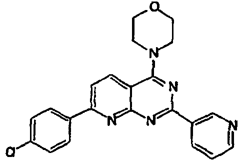 | 94 | 5.20 | 404.2 |
| **9u** | 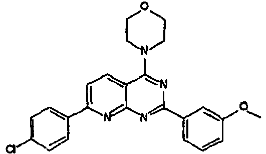 | 96 | 5.24 | 433.2 |
| **9v** | 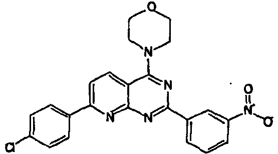 | 99 | 7.18 | 448.2 |
| **9w** | 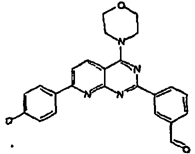 | 83 | 6.25 | 431.2 |
| **9x** | 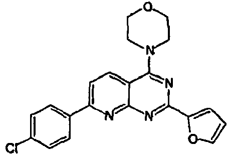 | 99 | 5.18 | 393.2 |
| **9y** | 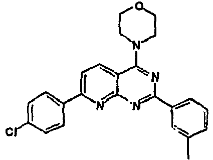 | 81 | 5.83 | 417.2 |
| **9z** | 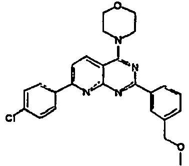 | 80 | 2.71 | 447.2 |
| **9aa** | 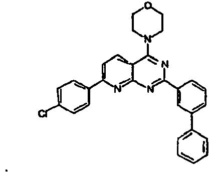 | 97 | 6.27 | 479.4 |
| **9ab** | 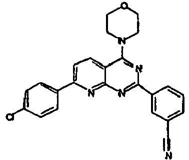 | 96 | 6.20 | 428.3 |
| **9ac** | 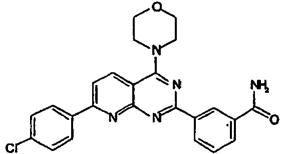 | 97 | 2.11 | 446.2 |
| **9ad** | 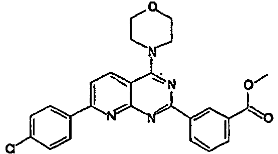 | 95 | 6.51 | 461.2 |
| **9ae** | 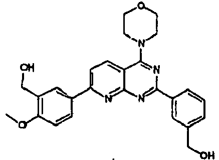 | 87 | 7.28 | 459.3 |

### Example 10

### Enzyme Assay

For mTOR enzyme activity assays, mTOR protein was isolated from HeLa cell cytoplasmic extract by immunoprecipitation, and activity determined essentially as described previously using recombinant PHAS-1 as a substrate (ref. 21).

The following compounds were tested in this assay:- 1a, 1b, 1c, 1d, 1e, 1f, 1g, 1h, 1j, 1k, 1l, 1l, 1m, 1n, 1o, 1p, 1q, 1r, 1s, It, 1u, 1v, 1w, 1x, 1y, 1z, 1aa, 1ab, 1ac, 1ad, 1ae, 1af, 1ag, 1ah, 1ai, 1aj, 1ak, 1al, 1am, 1an, 1ao, 1ap, 1aq, 1ar, 1as, 1at, 1au, 1av, 1aw, 1ax, 1ay, 1az, 1ba, 1bb, 1bc, 2a, 2b, 2c, 2d, 2e, 2f, 2g, 2h, 2i, 2j, 2k, 21, 2m, 2n, 2o, 2p, 2q, 2r, 2s, 2t, 2u, 2v, 2w, 2x, 2y, 2z, 2aa, 2ab, 2ac, 2ad, 2ae, 2af, 2ag, 2ah, 2ai, 2aj, 2ak, 2al, 2am, 2an, 2ao, 2ap, 2aq, 2ar, 2as, 2at, 2au, 2av, 2aw, 2ax, 2ay, 2az, 2ba, 2bb, 2bc, 2bd, 2be, 2bf, 2bg, 8bm, 8bn, 4a, 5a, 5b, 5c, 5d, 5e, 5f, 5g, 6, 7, 8a, 8b, 8c, 8d, 8e, 8f, 8g, 8h, 8i, 8j, 8k, 81, 8m, 8n, 8o, 8p, 8q, 8r, 8s, 8t, 8u, 8v, 8w, 8x, 8y, 8z, 8aa, 8ab, 8ac, 8ad, 8ae, 8af, 8ag, 8ah, 8ai, 8aj, 8ak, 8al, 8am, 8an, 8ao, 8ap, 8aq, 8ar, 8as, 8at, 8au, 8av, 8aw, 8ax, 8az, 8ba, 8bb, 8bc, 8bd, 8be, 8bf and 8bg.

All the compounds tested exhibited IC₅₀ values against mTOR of less than 10µm. The following compounds exhibited IC₅₀ values against mTOR of less than 1µm:
1aa, 1ab, 1ac, 1ad, 1ae, 1af, 1ag, 1ah, 1ai, 1aj , 1al, 1am, 1an, 1ao, 1ap, 1aq, 1ar, 1as, 1at, 1au, 1av, 1aw, 1ax, 1c, 1k, 1r, 1s, It, 1u, 1v, 1w, 1x, 1y, 1z, 2a, 2aa, 2ac, 2ad, 2af, 2ah, 2ap, 2aq, 2ar, 2as, 2av, 2aw, 2ax, 2az, 2b, 2bb, 2bd, 2be, 2c, 2e, 2g, 2h, 2i, 2j, 2k, 2n, 2p, 2q, 2t, 2u, 2z, 2bi, 3a, 3b, 5c, 8p, 8q, 8r, 8s, 8t, 8u, 8v, 8w, 8x, 8y, 8ae, 8af, 8am, 8ao, 8at, 8au, 8ay, 8az, 8ba, 8bd and 8bf, with the following compounds exhibiting IC₅₀ values against mTOR of less than 100nM: 1a, 1b, 1d, 1e, If, 1g, 1i, 1k, 11, 1m, In, 1o, 1p, 1q, 1bb, 1bc, 2ba, 8a, 8b, 8c, 8d, 8e, 8f, 8g, 8h, 8i, 8j, 8k, 81, 8m, 8n, 8o, 8aa, 8ab, 8ac, 8ad, 8ag, 8ah, 8ai, 8aj, 8ak, 8al, 8an, 8ap, 8aq, 8ar, 8as, 8av, 8aw, 8ax, 8bb, 8bc, 8be, 8bg. For example, Compound 1k has an IC₅₀ of 0.043µM

### Example 11

### Alternative Enzyme Assay

The assay used AlphaScreen technology (Gray et al., Analytical Biochemistry, 2003, 313: 234-245) to determine the ability of test compounds to inhibit phosphorylation by recombinant mTOR.

A C-terminal truncation of mTOR encompassing amino acid residues 1362 to 2549 of mTOR (EMBL Accession No. L34075) was stably expressed as a FLAG-tagged fusion in HEK293 cells as described by Vilella-Bach et al., Journal of Biochemistry, 1999, 274, 4266-4272. The HEK293 FLAG-tagged mTOR (1362-2549) stable cell line was routinely maintained at 37°C with 5% CO₂ up to a confluency of 70-90% in Dulbecco's modified Eagle's growth medium (DMEM; Invitrogen Limited, Paisley, UK Catalogue No. 41966-029) containing 10% heat-inactivated foetal calf serum (FCS; Sigma, Poole, Dorset, UK, Catalogue No. F0392), 1% L-glutamine (Gibco, Catalogue No. 25030-024) and 2 mg/ml Geneticin (G418 sulphate; Invitrogen Limited, UK Catalogue No. 10131-027). Following expression in the mammalian HEK293 cell line, expressed protein was purified using the FLAG epitope tag using standard purification techniques.

Test compounds were prepared as 10 mM stock solutions in DMSO and diluted into water as required to give a range of final assay concentrations. Aliquots (2 µl) of each compound dilution were placed into a well of a Greiner 384-well low volume (LV) white polystyrene plate (Greiner Bio-one). A 30 µl mixture of recombinant purified mTOR enzyme, 1 µM biotinylated peptide substrate (Biotin-Ahx-Lys-Lys-Ala-Asn-Gln-Val-Phe-Leu-Gly-Phe-Thr-Tyr-Val-Ala-Pro-Ser-Val-Leu-Glu-Ser-Val-Lys-Glu-NH₂; Bachem UK Ltd), ATP (20 µM) and a buffer solution [comprising Tris-HCl pH7.4 buffer (50 mM), EGTA (0.1 mM), bovine serum albumin (0.5 mg/ml), DTT (1.25 mM) and manganese chloride (10 mM)] was agitated at room temperature for 90 minutes.

Control wells that produced a maximum signal corresponding to maximum enzyme activity were created by using 5% DMSO instead of test compound. Control wells that produced a minimum signal corresponding to fully inhibited enzyme were created by adding EDTA (83 mM) instead of test compound. These assay solutions were incubated for 2 hours at room temperature.

Each reaction was stopped by the addition of 10 µl of a mixture of EDTA (50 mM), bovine serum albumin (BSA; 0.5 mg/ml) and Tris-HCl pH7.4 buffer (50 mM) containing p70 S6 Kinase (T389) 1A5 Monoclonal Antibody (Cell Signalling Technology, Catalogue No. 9206B) and AlphaScreen Streptavidin donor and Protein A acceptor beads (200 ng; Perkin Elmer, Catalogue No. 6760002B and 6760137R respectively) were added and the assay plates were left for about 20 hours at room temperature in the dark. The resultant signals arising from laser light excitation at 680 nm were read using a Packard Envision instrument.

Phosphorylated biotinylated peptide is formed *in situ* as a result of mTOR mediated phosphorylation. The phosphorylated biotinylated peptide that is associated with AlphaScreen Streptavidin donor beads forms a complex with the p70 S6 Kinase (T3 89) 1A5 Monoclonal Antibody that is associated with Alphascreen Protein A acceptor beads. Upon laser light excitation at 680 nm, the donor bead : acceptor bead complex produces a signal that can be measured. Accordingly, the presence of mTOR kinase activity results in an assay signal. In the presence of an mTOR kinase inhibitor, signal strength is reduced. mTOR enzyme inhibition for a given test compound was expressed as an IC₅₀ value. The following compounds were screened in this assay:- 1bd, 1be, 1bk, 1bl, 1bm, 1bn, 1bo, 1bp, 1bq, 1br, 1bs, 1bt, 1bu, 1bv, 1bw, 1bx, 2bf, 9a, 9b, 9c, 9d, 9e, 9f, 9g, 9i, 9j, 9m, 9n, 9o, 9p, 9q, 9r, 9s, 9t, 9u, 9v, 9w, 9x, 9y, 9z, 9aa, 9ab, 9ac, 9ad and 9ae.

All the compounds tested exhibited IC₅₀ values against mTOR of less than 10µm. The following compounds exhibited IC₅₀ values against mTOR of less than 1µm: 1bk, 1bm, 1bn, 1bo, 1bp, 1bq, 1br, 1bs, 1bt, 1bu, 9m, 9n, 9p, 9r, 9aa and 9ad, with the following compounds exhibiting IC₅₀ values against mTOR of less than 300nM: 1bd, 1be, 9a, 9b, 9c, 9d, 9e, 9f, 9g, 9ae, 91, 9j, 9k, 9i, 9h, 1bj, 1bi, 1bh, 1bg and 1bf. For example, Compound 1b has an IC₅₀ of 0.057 µM

### Example 12

### Cell proliferation assay (GIso)

Cell growth was assessed using the sulforhodamine B (SRB) assay (A). T47D (ECACC, 85102201) cells were routinely passaged in RPMI (Invitrogen, 42401018) plus 10% foetal calf serum (FCS), 1% L-glutamine (Gibco BRL, 25030) to a confluence not greater than 80%. To undertake the assay, T47D cells were seeded at 2.5x10³ cells/ well in 90µl RPMI plus 10% foetal calf serum, 1% L-glutamine in 96 well plates (Costar, 3904) and incubated at 37°C (+5% CO₂) in a humidified incubator. Once the cells had fully adhered (typically following 4-5 hours incubation) the plate was removed from the incubator and 10µL of the diluent added to the control wells (A1-12 and B1-12). Compound was prepared in a six point semi-log dilution at 10x the final concentration required e.g. for a 6 point range of 30µM to 100nM in semi-log steps dilution started at 300µM in stock plate. Dosing was completed by addition of 10µL of compound at highest concentration to C1-12 through to the lowest concentration in H1-12. The plates were then incubated for 120 hours prior to SRB analysis.

Upon completion of incubation, media was removed and the cells fixed with 100µl of ice cold 10% (w/v) trichloroacetic acid. The plates were incubated at 4°C for 20 minutes and then washed four times with water. Each well of cells was then stained with 100µl of 0.4% (w/v) SRB (Sulforhodamine B, Sigma, Poole, Dorset, UK, Catalogue number S-9012) in 1% acetic acid for 20 minutes before washing four times with 1% acetic acid. Plates were then dried for 2 hours at room temperature. The dye from the stained cells was solubilized by the addition of 100µl of 10mM Tris Base into each well. Plates were gently shaken and left at room temperature for 30 minutes before measuring the optical density at 564nM on a Microquant microtiter plate reader. The concentration of inhibitor eliciting a 50% reduction in growth (GI₅₀) was determined by analysis of staining intensity of the treated cells as a percentage of the vehicle control wells using Excelfit software.
(A) Skehan, P., Storung, R., Scudiero, R., Monks, A., McMahon, J., Vistica, D., Warren, J. T., Bokesch, H., Kenny, S. and Boyd, M. R. (1990) New colorimetric cytotoxicity assay for anticancer-drug screening. J. Natl. Cancer Inst. 82, 1107-1112.
All the compounds tested exhibited GI₅₀ values of less than 10µM.
The following compounds exhibited GI₅₀ values of less than 1µM 8c, 8e, 8h, 8m, 8n, 8o, 8p, 8q, 8s, 8v, 8w, 8x, 8y, 8z, 8aa, and 8ac, with the following compounds exhibiting GI₅₀ values of less than 300nM : 1g, 8a, 8b, 8d, 8f, 8g, 8i, 8k, 81, 8r, 8t, 8u, 8ab, 8ad, 8ae, 8af, 8ag, 8ah, 8ai, 8aj, 8ak, 8al, 8am, 8an, 8ao, 8ap, 8aq, 8ar, 8as, 8at, 8au, 8av, 8aw, 8ax, 8ay, 8az, 8ba, 8bb, 8bc, 8bd, 8be, 8bf, and 8bg. For example, Compound 1r has a GI₅₀ of 0.232µM.

### Example 13

### In Vitro phospho-Ser473 Akt assay

This assay determines the ability of test compounds to inhibit phosphorylation of Serine 473 in Akt as assessed using Acumen Explorer technology (Acumen Bioscience Limited), a plate reader that can be used to rapidly quantitate features of images generated by laser-scanning.

A MDA-MB-468 human breast adenocarcinoma cell line (LGC Promochem, Teddington, Middlesex, UK, Catalogue No. HTB-132) was routinely maintained at 37°C with 5% CO₂ up to a confluency of 70-90% in DMEM containing 10% heat-inactivated FCS and 1% L-glutamine.

For the assay, the cells were detached from the culture flask using 'Accutase' (Innovative Cell Technologies Inc., San Diego, CA, USA; Catalogue No. AT104) using standard tissue culture methods and resuspended in media to give 1.7x10⁵ cells per ml. Aliquots (90 µl) were seeded into each of the inner 60 wells of a black Packard 96 well plate (PerkinElmer, Boston, MA, USA; Catalogue No. 6005182) to give a density of ~15000 cells per well. Aliquots (90 µl) of culture media were placed in the outer wells to prevent edge effects. The cells were incubated overnight at 37°C with 5% CO₂ to allow them to adhere.

On day 2, the cells were treated with test compounds and incubated for 2 hours at 37°C with 5% CO₂. Test compounds were prepared as 10 mM stock solutions in DMSO and serially diluted as required with growth media to give a range of concentrations that were 10-fold the required final test concentrations. Aliquots (10 µl) of each compound dilution were placed in a well (in triplicate) to give the final required concentrations. As a minimum reponse control, each plate contained wells having a final concentration of 100 µM LY294002 (Calbiochem, Beeston, UK, Catalogue No. 440202). As a maximum response control, wells contained 1% DMSO instead of test compound. Following incubation, the contents of the plates were fixed by treatment with a 1.6% aqueous formaldehyde solution (Sigma, Poole, Dorset, UK, Catalogue No. F1635) at room temperature for 1 hour.

All subsequent aspiration and wash steps were carried out using a Tecan 96 well plate washer (aspiration speed 10 mm/sec). The fixing solution was removed and the contents of the plates were washed with phosphate-buffered saline (PBS; 50 µl; Gibco, Catalogue No. 10010015). The contents of the plates were treated for 10 minutes at room temperature with an aliquot (50 µl) of a cell permeabilisation buffer consisting of a mixture of PBS and 0.5% Tween-20. The 'permeabilisation' buffer was removed and non-specific binding sites were blocked by treatment for 1 hour at room temperature of an aliquot (50 µl) of a blocking buffer consisting of 5% dried skimmed milk ['Marvel' (registered trade mark); Premier Beverages, Stafford, GB] in a mixture of PBS and 0.05% Tween-20. The 'blocking' buffer was removed and the cells were incubated for 1 hour at room temperature with rabbit anti phospho-Akt (Ser473) antibody solution (50 µl per well; Cell Signalling, Hitchin, Herts, U.K., Catalogue No 9277) that had been diluted 1:500 in 'blocking' buffer. Cells were washed three times in a mixture of PBS and 0.05% Tween-20. Subsequently, cells were incubated for 1 hour at room temperature with Alexafluor488 labelled goat anti-rabbit IgG (50 µl per well; Molecular Probes, Invitrogen Limited, Paisley, UK, Catalogue No. A11008) that had been diluted 1:500 in `blocking' buffer. Cells were washed 3 times with a mixture of PBS and 0.05% Tween-20. An aliquot of PBS (50 µl) was added to each well and the plates were sealed with black plate sealers and the fluorescence signal was detected and analysed.

Fluorescence dose response data obtained with each compound were analysed and the degree of inhibition of Serine 473 in Akt was expressed as an IC₅₀ value.
The following compounds were tested and exhibited a cellular IC₅₀, measured by pAkt, of less than 25µM: 1bj, 1bd, 1bh, 1bi, 9j, 9a, 9aa, 9r, 9d, 9f, 9e with the following compounds exhibiting a cellular IC₅₀, measured by pAkt, of less than 2.5µM: 1br, 1bg, 1be, 91, 9n, 9k, 9h, 9g, 9m and 9i. For example, compound 91 has a pAkt IC50 of 2.2uM

### Reference List

1) Brown, et al., Nature, 369, 756-758 (1994)
2) Chiu, et al., Proc Natl Acad Sci, 91, 12574-12578 (1994)
3) Sabatini, et al., Cell, 78, 35-43, (1994)
4) Sabers, et al., J Biol Chem, 270, 825-822 (1995)
5) Abraham, Curr Opin Immunol, 8, 412-418 (1996)
6) Schmelze and Hall, Cell,103, 253-262 (2000)
7) Burnett, et al., Proc Natl Acad Sci, 95, 1432-1437 (1998)
8) Terada, et al., Proc Natl Acad Sci, 91,11477-11481 (1994)
9) Jeffries, et al., EMBOJ, 16, 3693-3704 (1997)
10) Bjornsti and Houghton, Nat Rev Cancer, 4, 335-348 (2004)
11) Gingras, et al., Genes Dev, 13, 1422-1437 (1999)
12) Gingras, et al., Genes Dev, 15, 807-826 (2001)
13) Neuhaus, et al., Liver Transplantation, 7, 473-484 (2001)
14) Woods and Marks, Ann Rev Med, 55, 169-178 (2004)
15) Dahia, Endocrine-Related Cancer, 7, 115-129 (2000)
16) Cristofano and Pandolfi, Cell, 100, 387-390 (2000)
17) Samuels, et al., Science, 304, 554 (2004)
18) Huang and Houghton, Curr Opin Pharmacol, 3, 371-377 (2003)
19) Sawyers, Cancer Cell, 4, 343-348 (2003)
20) Huang and Houghton, Curr Opin in Invest Drugs, 3, 295-304 (2002)
21) Brunn, et al., EMBO J, 15, 5256-5267 (1996)
22) Edinger, et al., Cancer Res, 63, 8451-8460, (2003)
23) Lawrence, et al., Curr Top Microbiol Immunol, 279, 199-213 (2004)
24) Eshleman, et al., Cancer Res, 62, 7291-7297 (2002)
25) Berge, et al., J. Pharm. Sci., 66, 1-19 (1977).
26) Green, T. and Wuts, P., "Protective Groups in Organic Synthesis", 3rd Edition, John Wiley and Sons (1999).
27) "Handbook of Pharmaceutical Additives", 2nd Edition (eds. M. Ash and I. Ash), 2001 (Synapse Information Resources, Inc., Endicott, New York, USA).
28) "Remington's Pharmaceutical Sciences", 20th edition, pub. Lippincott, Williams & Wilkins, 2000.
29) "Handbook of Pharmaceutical Excipients", 2nd edition, 1994.

## Claims

1. A compound of formula I: 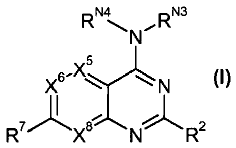 wherein:
X⁸ is N, and X⁵ and X⁶ are CH;
R⁷ is a C₅-₂₀ aryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃-₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino);
R^{N3} and R^{N4}, together with the nitrogen to which they are bound, form a heterocyclic ring containing between 3 and 8 ring atoms optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino);
R² is selected from NR^{N5}R^{N6}, a C₅₋₂₀ heteroaryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl,
thioamido and sulfonamino), and a C₅₋₂₀ aryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl,
thioamido and sulfonamino),
wherein R^{N5} and R^{N6} are independently selected from H, a C₁₋₇ alkyl group, a C₅₋₂₀ heteroaryl group, and a C₅₋₂₀ aryl group, or R^{N5} and R^{N6} together with the nitrogen to which they are bound form a heterocyclic ring containing between 3 and 8 ring atoms where each C₁₋₇alkyl, C₅₋₂₀heteroaryl, C₅₋₂₀aryl or heterocyclic ring is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl,
thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and
sulfonamino),
or a pharmaceutically acceptable salt thereof,
and wherein
"C₅-₂₀ aryl" as used herein, pertains to a monovalent moiety obtained by removing a hydrogen atom from an aromatic ring atom of a C₅₋₂₀ aromatic compound, said compound having one ring, or two or more rings (e.g., fused), and having from 5 to 20 ring atoms, and wherein at least one of said ring(s) is an aromatic ring and where the ring atoms may include one or more heteroatoms, including but not limited to oxygen, nitrogen, and sulphur;
"alkyl" as used herein, pertains to a monovalent moiety obtained by removing a hydrogen atom from a carbon atom of a hydrocarbon compound having from 1 to 20 carbon atoms (unless otherwise specified), which may be aliphatic or alicyclic, and which may be saturated or unsaturated (e.g. partially unsaturated, fully unsaturated); "alkenyl", as used herein, pertains to an alkyl group having one or more carbon-carbon double bonds;
"alkynyl", as used herein, pertains to an alkyl group having one or more carbon-carbon triple bonds;
"cycloalkyl", as used herein, pertains to an alkyl group which is also a cyclyl group; that is, a monovalent moiety obtained by removing a hydrogen atom from an alicyclic ring atom of a carbocyclic ring of a carbocyclic compound, which carbocyclic ring may be saturated or unsaturated (e.g. partially unsaturated, fully unsaturated), which moiety has from 3 to 20 carbon atoms (unless otherwise specified), including from 3 to 20 ring atoms;
"ether", as used herein, pertains to a -OR group, wherein R is a C₁₋₇ alkyl group, a C₃₋₂₀ heterocyclyl group, or a C₅₋₂₀ aryl group;
"acyl", as used herein, pertains to a -C(=O)R group, wherein R is H, a C₁₋₇ alkyl group, a C₃₋₂₀ heterocyclyl group, or a C₅₋₂₀ aryl group;
"ester", as used herein, pertains to a -C(=O)OR group, wherein R is a C₁₋₇ alkyl group, a C₃₋₂₀ heterocyclyl group, or a C₅₋₂₀ aryl group;
"amido", as used herein, pertains to a -C(=O)NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"amino", as used herein, pertains to a -NR¹R² group, wherein R¹ and R² are independently hydrogen, a C₁₋₇ alkyl group, a C₃-₂₀ heterocyclyl group, or a C₅-₂₀ aryl group, or R¹ and R², taken together with the nitrogen atom to which they are attached, form a heterocyclic ring having from 4 to 8 ring atoms;
"acylamido", as used herein, pertains to a -NR¹C(=O)R² group, wherein R¹ hydrogen, a C₁-₇ alkyl group, a C₃-₂₀ heterocyclyl group, or a C₅₋₂₀ aryl group, R² is a C₁₋₇ alkyl group, a C₃-₂₀ heterocyclyl group, or a C₅-₂₀ aryl group, or R¹ and R² may together with the atoms to which they are attached form a succinimidyl, maleimidyl, and phthalimidyl group;
"ureido", as used herein, pertains to a -N(R¹)CONR²R³ group, wherein R² and R³ are independently hydrogen, a C₁-₇ alkyl group, a C₃₋₂₀ heterocyclyl group, or a C₅₋₂₀ aryl group, or R² and R³, taken together with the nitrogen atom to which they are attached, form a heterocyclic ring having from 4 to 8 ring atoms, and R¹ is hydrogen, a C₁₋₇alkyl group, a C₃₋₂₀oheterocyclyl group, or a C₅₋₂₀aryl group;
"acyloxy", as used herein, pertains to a -OC(=O)R group, wherein R is a C₁-₇ alkyl group, a C₃₋₂₀ heterocyclyl group, or a C₅₋₂₀ aryl group;
"thioether", as used herein, pertains to a -SR group, wherein R is a C₁₋₇ alkyl group, a C₃₋₂₀ heterocyclyl group, or a C₅₋₂₀ aryl group;
"sulfoxide", as used herein, pertains to a -S(=O)R group, wherein R is a C₁-₇ alkyl group, a C₃₋₂₀ heterocyclyl group, or a C₅₋₂₀ aryl group;
"sulfonyl", as used herein, pertains to a -S(=O)₂R group, wherein R is a C₁-₇ alkyl group, a C₃₋₂₀ heterocyclyl group, or a C₅₋₂₀ aryl group;
"thioamido", as used herein, pertains to a -C(=S)NR¹R² group, wherein R¹ and R² are independently hydrogen, a C₁-₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"sulfonamino", as used herein, pertains to a -NR¹S(=O)₂R group, wherein R¹ is hydrogen, a C₁₋₇ alkyl group, a C₃₋₂₀ heterocyclyl group, or a C₅₋₂₀ aryl group, and R is a C₁₋₇alkyl group, a C₃₋₂₀heterocyclyl group, or a C₅₋₂₀aryl group; and
with the proviso that when R² is unsubstituted morpholino, R^{N3} and R^{N4} together with the nitrogen atom to which they are attached form an unsubstituted morpholino, R⁷ is not unsubstituted phenyl, and
when R² is unsubstituted piperidinyl, R^{N3} and R^{N4} together with the nitrogen atom to which they are attached form an unsubstituted piperidinyl, R⁷ is not unsubstituted phenyl.

2. A compound according to claim 1, wherein R⁷ is a phenyl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl,
C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), and wherein the ring heteroatoms of any C₅₋₂₀
heteroaryl group or C₃₋₂₀heterocyclyl are selected from nitrogen, oxygen and sulphur, and
wherein
"C₅₋₂₀ aryl" as used herein, pertains to a monovalent moiety obtained by removing a hydrogen atom from an aromatic ring atom of a C₅₋₂₀ aromatic compound, said compound having one ring, or two or more rings (e.g., fused), and having from 5 to 20 ring atoms, and wherein at least one of said ring(s) is an aromatic ring and where the ring atoms may include one or more heteroatoms, including but not limited to oxygen, nitrogen, and sulphur;
"alkyl" as used herein, pertains to a monovalent moiety obtained by removing a hydrogen atom from a carbon atom of a hydrocarbon compound having from 1 to 20 carbon atoms (unless otherwise specified), which may be aliphatic or alicyclic, and which may be saturated or unsaturated (e.g. partially unsaturated, fully unsaturated); "alkenyl", as used herein, pertains to an alkyl group having one or more carbon-carbon double bonds;
"alkynyl", as used herein, pertains to an alkyl group having one or more carbon-carbon triple bonds;
"cycloalkyl", as used herein, pertains to an alkyl group which is also a cyclyl group; that is, a monovalent moiety obtained by removing a hydrogen atom from an alicyclic ring atom of a carbocyclic ring of a carbocyclic compound, which carbocyclic ring may be saturated or unsaturated (e.g. partially unsaturated, fully unsaturated), which moiety has from 3 to 20 carbon atoms (unless otherwise specified), including from 3 to 20 ring atoms;
"ether", as used herein, pertains to a -OR group, wherein R is a C₁₋₇ alkyl group, a C₃₋₂₀ heterocyclyl group, or a C₅₋₂₀ aryl group;
"acyl", as used herein, pertains to a -C(=O)R group, wherein R is H, a C₁₋₇ alkyl group, a C₃₋₂₀ heterocyclyl group, or a C₅₋₂₀ aryl group;
"ester", as used herein, pertains to a -C(=O)OR group, wherein R is a C₁₋₇ alkyl group, a C₃₋₂₀ heterocyclyl group, or a C₅₋₂₀ aryl group;
"amido", as used herein, pertains to a -C(=O)NR¹R² group, wherein R¹ and R² are independently hydrogen, a C₁-₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"amino", as used herein, pertains to a -NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"acylamido", as used herein, pertains to a -NR¹C(=O)R² group, wherein R¹ hydrogen, a C₁₋₇ alkyl group, a C₃₋₂₀ heterocyclyl group, or a C₅₋₂₀ aryl group, R² is a C₁₋₇ alkyl group, a C₃₋₂₀ heterocyclyl group, or a C₅₋₂₀ aryl group, or R¹ and R² may together with the atoms to which they are attached form a succinimidyl, maleimidyl, and phthalimidyl group;
"ureido", as used herein, pertains to a -N(R¹)CONR²R³ group, wherein R² and R³ are independently hydrogen, a C₁₋₇ alkyl group, a C₃₋₂₀ heterocyclyl group, or a C₅₋₂₀ aryl group, or R² and R³, taken together with the nitrogen atom to which they are attached, form a heterocyclic ring having from 4 to 8 ring atoms, and R¹ is hydrogen, a C₁₋₇alkyl group, a C₃₋₂₀heterocyclyl group, or a C₅₋₂₀aryl group;
"acyloxy", as used herein, pertains to a -OC(=O)R group, wherein R is a C₁₋₇ alkyl group, a C₃₋₂₀ heterocyclyl group, or a C₅₋₂₀ aryl group;
"thioether", as used herein, pertains to a -SR group, wherein R is a C₁₋₇ alkyl group, a C₃₋₂₀ heterocyclyl group, or a C₅₋₂₀ aryl group;
"sulfoxide", as used herein, pertains to a -S(=O)R group, wherein R is a C₁₋₇ alkyl group, a C₃₋₂₀ heterocyclyl group, or a C₅₋₂₀ aryl group;
"sulfonyl", as used herein, pertains to a -S(=O)₂R group, wherein R is a C₁₋₇ alkyl group, a C₃₋₂₀ heterocyclyl group, or a C₅₋₂₀ aryl group;
"thioamido", as used herein, pertains to a -C(=S)NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms; and
"sulfonamino", as used herein, pertains to a -NR¹S(=O)₂R group, wherein R¹ is hydrogen, a C₁₋₇ alkyl group, a C₃₋₂₀ heterocyclyl group, or a C₅₋₂₀ aryl group, and R is a C₁₋₇alkyl group, a C₃₋₂₀heterocyclyl group, or a C₅₋₂₀aryl group.

3. A compound according to claim 2, wherein R⁷ is a phenyl group optionally substituted by one or more groups selected from chloro, hydroxyl, methyl, methoxy, ethoxy, i-propoxy, benzyloxy and hydroxymethyl.

4. A compound according to claim 2, wherein R⁷ is 4-chlorophenyl, 4-methylphenyl, 4-methoxyphenyl, 3-hydroxymethyl-4-methoxy-phenyl, 3,5-dimethoxy-4-hydroxyphenyl, 4-hydroxyphenyl, 3-hydroxyphenyl or a 3-hydroxymethylphenyl group.

5. A compound according to claim 1 wherein the compound is a compound of formula I(A): 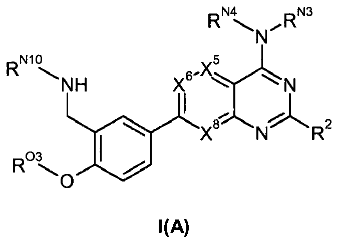 wherein:
X⁸ is N, and X⁵ and X⁶ are CH;
R^{N3} and R^{N4}, together with the nitrogen to which they are bound, form a heterocyclic ring containing between 3 and 8 ring atoms optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and
sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino);
R² is selected from NR^{N5}R^{N6}, a C₅₋₂₀ heteroaryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), and a C₅₋₂₀ aryl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
wherein R^{N5} and R^{N6} are independently selected from H, a C₁₋₇ alkyl group, a C₅₋₂₀ heteroaryl group, and a C₅₋₂₀ aryl group, or R^{N5} and R^{N6} together with the nitrogen to which they are bound form a heterocyclic ring containing between 3 and 8 ring atoms where each C₁₋₇alkyl, C₅₋₂₀heteroaryl, C₅₋₂₀aryl or heterocyclic ring is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁-₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino);
R^{O3} is selected from hydrogen or a C₁₋₆ alkyl group optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino); and
R^{N10} is selected from C(=O)R^{C2}, C(=S)R^{C3}, SO₂R^{S3}, a C₅₋₂₀ heteroaryl group optionally substituted by one or more groups selected from halo, hydroxyl, nitro, cyano, carboxy, and
thiol, or C₁-₇alkyl, C₂₋₇alkenyl, C₂₋₇alkynyl, C₃₋₇cycloalkyl, C₃₋₇cycloalkenyl, C₃₋₂₀heterocyclyl, C₅-₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), a C₅₋₂₀ aryl group optionally substituted by one or more groups selected from halo, hydroxyl, nitro, cyano, carboxy, and
thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), or a C₁₋₁₀ alkyl group optionally substituted by one or more groups selected from halo, hydroxyl, nitro, cyano, carboxy, and
thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino) where R^{C2} and R^{C3} are selected from H, a C₅₋₂₀ aryl group, a C₅₋₂₀ heteroaryl group, a C₁₋₇ alkyl group or NR^{N11}R^{N12}, where R^{N11} and R^{N12} are independently selected from H, a C₁₋₇ alkyl group, a C₅₋₂₀ heteroaryl group,
a C₅₋₂₀ aryl group or R^{N11} and R^{N12} together with the nitrogen to which they are bound form a heterocyclic ring containing between 3 and 8 ring atoms, where each C₁₋₇alkyl, C₅₋₂₀ heteroaryl, C₅₋₂₀aryl or heterocyclic ring is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino); and R^{S3} is selected from H, a C₅₋₂₀ aryl group, a C₅₋₂₀ heteroaryl group, or a C₁₋₇ alkyl group where each C₁₋₇alkyl, C₅₋₂₀heteroaryl or C₅₋₂₀aryl is optionally substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino),
or a pharmaceutically acceptable salt thereof,
and wherein
"C₅₋₂₀ aryl" as used herein, pertains to a monovalent moiety obtained by removing a hydrogen atom from an aromatic ring atom of a C₅₋₂₀ aromatic compound, said compound having one ring, or two or more rings (e.g., fused), and having from 5 to 20 ring atoms, and wherein at least one of said ring(s) is an aromatic ring and where the ring atoms may include one or more heteroatoms, including but not limited to oxygen, nitrogen, and sulphur;
"alkyl" as used herein, pertains to a monovalent moiety obtained by removing a hydrogen atom from a carbon atom of a hydrocarbon compound having from 1 to 20 carbon atoms (unless otherwise specified), which may be aliphatic or alicyclic, and which may be saturated or unsaturated (e.g. partially unsaturated, fully unsaturated);
"alkenyl", as used herein, pertains to an alkyl group having one or more carbon-carbon double bonds;
"alkynyl", as used herein, pertains to an alkyl group having one or more carbon-carbon triple bonds;
"cycloalkyl", as used herein, pertains to an alkyl group which is also a cyclyl group; that is, a monovalent moiety obtained by removing a hydrogen atom from an alicyclic ring atom of a carbocyclic ring of a carbocyclic compound, which carbocyclic ring may be saturated or unsaturated (e.g. partially unsaturated, fully unsaturated), which moiety has from 3 to 20 carbon atoms (unless otherwise specified), including from 3 to 20 ring atoms;
"ether", as used herein, pertains to a -OR group, wherein R is a C₁₋₇ alkyl group, a C₃₋₂₀ heterocyclyl group, or a C₅₋₂₀ aryl group;
"acyl", as used herein, pertains to a -C(=O)R group, wherein R is H, a C₁₋₇ alkyl group, a C₃₋₂₀ heterocyclyl group, or a C₅₋₂₀ aryl group;
"ester", as used herein, pertains to a -C(=O)OR group, wherein R is a C₁₋₇ alkyl group, a C₃₋₂₀ heterocyclyl group, or a C₅₋₂₀ aryl group;
"amido", as used herein, pertains to a -C(=O)NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"amino", as used herein, pertains to a -NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"acylamido", as used herein, pertains to a -NR¹C(=O)R² group, wherein R¹ hydrogen, a C₁₋₇ alkyl group, a C₃₋₂₀ heterocyclyl group, or a C₅₋₂₀ aryl group, R² is a C₁₋₇ alkyl group, a C₃₋₂₀ heterocyclyl group, or a C₅₋₂₀ aryl group, or R¹ and R² may together with the atoms to which they are attached form a succinimidyl, maleimidyl, and phthalimidyl group;
"ureido", as used herein, pertains to a -N(R¹)CONR²R³ group, wherein R² and R³ are independently hydrogen, a C₁₋₇ alkyl group, a C₃₋₂₀ heterocyclyl group, or a C₅₋₂₀ aryl group, or R² and R³, taken together with the nitrogen atom to which they are attached, form a heterocyclic ring having from 4 to 8 ring atoms, and R¹ is hydrogen, a C₁₋₇alkyl group, a C₃₋₂₀heterocyclyl group, or a C₅₋₂₀aryl group;
"acyloxy", as used herein, pertains to a -OC(=O)R group, wherein R is a C₁₋₇ alkyl group, a C₃₋₂₀ heterocyclyl group, or a C₅₋₂₀ aryl group;
"thioether", as used herein, pertains to a -SR group, wherein R is a C₁₋₇ alkyl group, a C₃₋₂₀ heterocyclyl group, or a C₅₋₂₀ aryl group;
"sulfoxide", as used herein, pertains to a -S(=O)R group, wherein R is a C₁₋₇ alkyl group, a C₃₋₂₀ heterocyclyl group, or a C₅₋₂₀ aryl group;
"sulfonyl", as used herein, pertains to a -S(=O)₂R group, wherein R is a C₁₋₇ alkyl group, a C₃₋₂₀ heterocyclyl group, or a C₅-₂₀ aryl group;
"thioamido", as used herein, pertains to a -C(=S)NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms; and
"sulfonamino", as used herein, pertains to a -NR¹S(=O)₂R group, wherein R¹ is hydrogen, a C₁₋₇ alkyl group, a C₃-₂₀ heterocyclyl group, or a C₅₋₂₀ aryl group, and R is a C₁₋₇alkyl group, a C₃₋₂₀heterocyclyl group, or a C₅₋₂₀aryl group.

6. A compound according to any one of claims 1 to 5 wherein R^{N3} and R^{N4} together with the nitrogen to which they are bound form a morpholino group.

7. A compound according to any one of claims 1 to 6, wherein R² is NR^{N5}R^{N6}, where R^{N5} and R^{N6} together with the nitrogen to which they are bound form a heterocyclic ring containing between 3 and 8 ring atoms, which may optionally be substituted by one or more groups selected from halo, hydroxyl, nitro, cyano, carboxy, and thiol, or C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino (each optionally substituted with one or more groups selected from halo, hydroxyl, nitro, cyano, carboxy, thiol, C₁₋₇alkyl, C₂₋₇alkenyl, C₂₋₇alkynyl, C₃₋₇cycloalkyl, C₃₋₇cycloalkenyl, C₃₋₂₀heterocyclyl, C₅₋₂₀aryl, C₅₋₂₀heteroaryl, ether, acyl, ester, amido, amino, acylamido, ureido, acyloxy, thioether, sulfoxide, sulfonyl, thioamido and sulfonamino), and wherein the ring heteroatoms of any C₅₋₂₀ heteroaryl group, C₃₋₂₀heterocyclyl or heterocyclic ring containing between 3 and 8 ring atoms are selected from nitrogen, oxygen and sulphur, and
wherein
"C₅₋₂₀ aryl" as used herein, pertains to a monovalent moiety obtained by removing a hydrogen atom from an aromatic ring atom of a C₅₋₂₀ aromatic compound, said compound having one ring, or two or more rings (e.g., fused), and having from 5 to 20 ring atoms, and wherein at least one of said ring(s) is an aromatic ring and where the ring atoms may include one or more heteroatoms, including but not limited to oxygen, nitrogen, and sulphur;
"alkyl" as used herein, pertains to a monovalent moiety obtained by removing a hydrogen atom from a carbon atom of a hydrocarbon compound having from 1 to 20 carbon atoms (unless otherwise specified), which may be aliphatic or alicyclic, and which may be saturated or unsaturated (e.g. partially unsaturated, fully unsaturated); "alkenyl", as used herein, pertains to an alkyl group having one or more carbon-carbon double bonds;
"alkynyl", as used herein, pertains to an alkyl group having one or more carbon-carbon triple bonds;
"cycloalkyl", as used herein, pertains to an alkyl group which is also a cyclyl group; that is, a monovalent moiety obtained by removing a hydrogen atom from an alicyclic ring atom of a carbocyclic ring of a carbocyclic compound, which carbocyclic ring may be saturated or unsaturated (e.g. partially unsaturated, fully unsaturated), which moiety has from 3 to 20 carbon atoms (unless otherwise specified), including from 3 to 20 ring atoms;
"ether", as used herein, pertains to a -OR group, wherein R is a C₁₋₇ alkyl group, a C₃₋₂₀ heterocyclyl group, or a C₅₋₂₀ aryl group;
"acyl", as used herein, pertains to a -C(=O)R group, wherein R is H, a C₁₋₇ alkyl group, a C₃₋₂₀ heterocyclyl group, or a C₅₋₂₀ aryl group;
"ester", as used herein, pertains to a -C(=O)OR group, wherein R is a C₁₋₇ alkyl group, a C₃₋₂₀ heterocyclyl group, or a C₅₋₂₀ aryl group;
"amido", as used herein, pertains to a -C(=O)NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"amino", as used herein, pertains to a -NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms;
"acylamido", as used herein, pertains to a -NR¹C(=O)R² group, wherein R¹ hydrogen, a C₁₋₇ alkyl group, a C₃₋₂₀ heterocyclyl group, or a C₅₋₂₀ aryl group, R² is a C₁₋₇ alkyl group, a C₃₋₂₀ heterocyclyl group, or a C₅₋₂₀ aryl group, or R¹ and R² may together with the atoms to which they are attached form a succinimidyl, maleimidyl, and phthalimidyl group;
"ureido", as used herein, pertains to a -N(R¹)CONR²R³ group, wherein R² and R³ are independently hydrogen, a C₁₋₇ alkyl group, a C₃₋₂₀ heterocyclyl group, or a C₅₋₂₀ aryl
group, or R² and R³, taken together with the nitrogen atom to which they are attached, form a heterocyclic ring having from 4 to 8 ring atoms, and R¹ is hydrogen, a C₁₋₇alkyl group, a C₃₋₂₀heterocyclyl group, or a C₅₋₂₀aryl group;
"acyloxy", as used herein, pertains to a -OC(=O)R group, wherein R is a C₁₋₇ alkyl group, a C₃₋₂₀ heterocyclyl group, or a C₅₋₂₀ aryl group;
"thioether", as used herein, pertains to a -SR group, wherein R is a C₁₋₇ alkyl group, a C₃₋₂₀ heterocyclyl group, or a C₅₋₂₀ aryl group;
"sulfoxide", as used herein, pertains to a -S(=O)R group, wherein R is a C₁₋₇ alkyl group, a C₃₋₂₀ heterocyclyl group, or a C₅₋₂₀ aryl group;
"sulfonyl", as used herein, pertains to a -S(=O)₂R group, wherein R is a C₁₋₇ alkyl group, a C₃₋₂₀ heterocyclyl group, or a C₅₋₂₀ aryl group;
"thioamido", as used herein, pertains to a -C(=S)NR¹R² group, wherein R¹ and R² are independently hydrogen, a C₁₋₇ alkyl group, a C₃₋₂₀ heterocyclyl group, or a C₅₋₂₀ aryl group, or R¹ and R², taken together with the nitrogen atom to which they are attached, form a heterocyclic ring having from 4 to 8 ring atoms; and
"sulfonamino", as used herein, pertains to a -NR¹S(=O)₂R group, wherein R¹ is hydrogen, a C₁₋₇ alkyl group, a C₃₋₂₀ heterocyclyl group, or a C₅₋₂₀ aryl group, and R is a C₁₋₇alkyl group, a C₃₋₂₀heterocyclyl group, or a C₅₋₂₀aryl group.

8. A compound according to claim 7, wherein R² is selected from morpholino, thiomorpholino, piperidinyl, piperazinyl, homopiperazinyl and pyrrolidinyl wherein each morpholino, thiomorpholino, piperidinyl, piperazinyl, homopiperazinyl and pyrrolidinyl may be optionally subtituted on carbon by one or more methyl groups.

9. A compound according to claim 8, wherein R² is

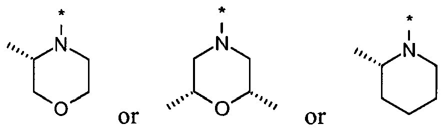

10. A compound according to Claim 1, selected from any of the following Examples
| | **R⁷** | **NR³R⁴** | **R²** |
|---|---|---|---|
| **1a** | 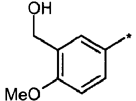 | 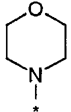 | 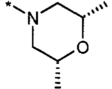 |
| **1b** | 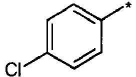 | 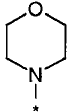 | 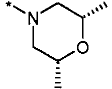 |
| **1c** | 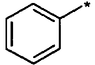 | 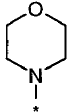 | 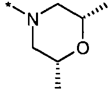 |
| **1d** | 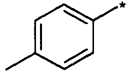 | 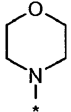 | 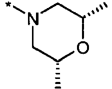 |
| **1e** | 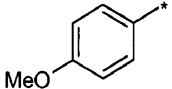 | 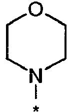 | 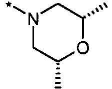 |
| **1f** | 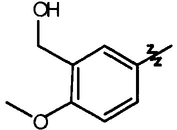 | 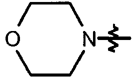 | 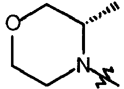 |
| **1g** | 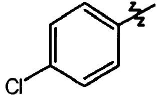 | 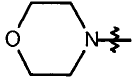 | 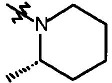 |
| **1h** | 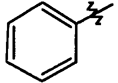 | 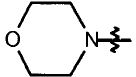 | 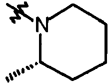 |
| **1i** | 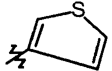 | 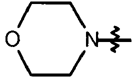 | 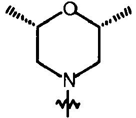 |
| **1j** | 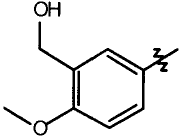 | 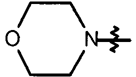 | 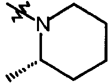 |
| **1k** | 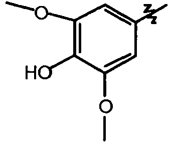 | 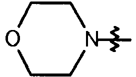 | 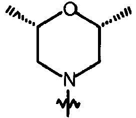 |
| **1l** | 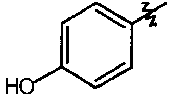 | 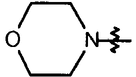 | 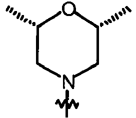 |
| **1m** | 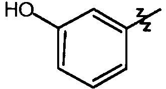 | 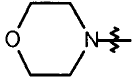 | 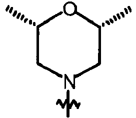 |
| **1n** | 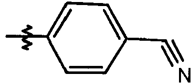 | 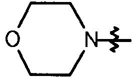 | 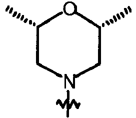 |
| **1o** | 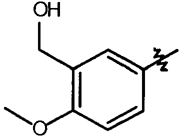 | 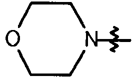 | 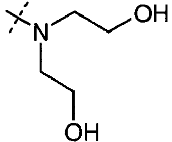 |
| **1p** | 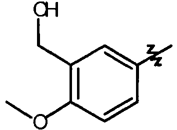 | 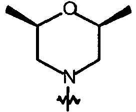 | 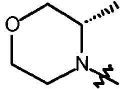 |
| **1q** | 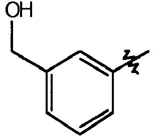 | 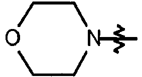 | 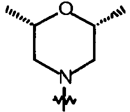 |
| **1r** | 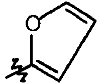 | 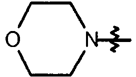 | 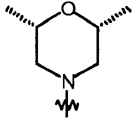 |
| **1s** | 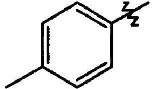 | 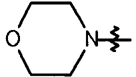 | 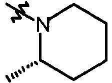 |
| **1t** | 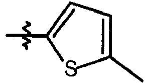 | 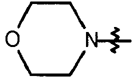 | 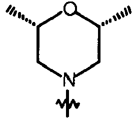 |
| **1u** | 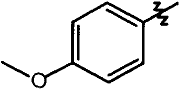 | 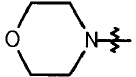 | 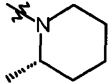 |
| **1v** | 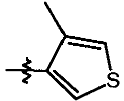 | 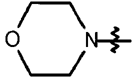 | 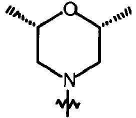 |
| **1w** | 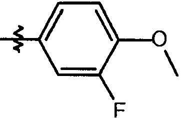 | 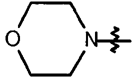 | 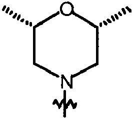 |
| **1x** | 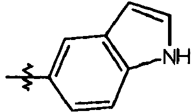 | 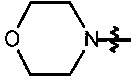 | 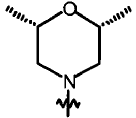 |
| **1y** | 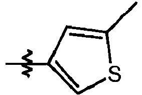 | 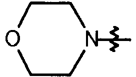 | 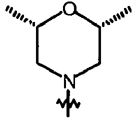 |
| **1z** | 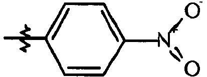 | 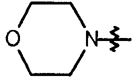 | 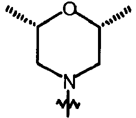 |
| **1aa** | 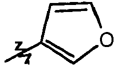 | 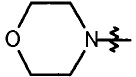 | 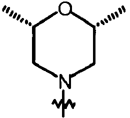 |
| **1ab** | 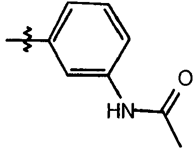 | 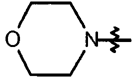 | 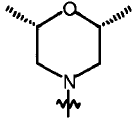 |
| **1ac** | 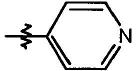 | 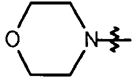 | 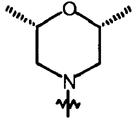 |
| **1ad** | 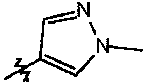 | 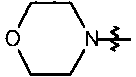 | 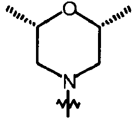 |
| **1ae** | 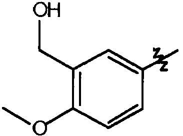 | 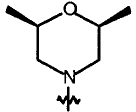 | 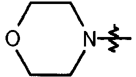 |
| **1af** | 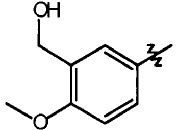 | 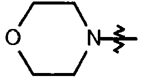 | 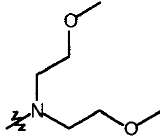 |
| **1ag** | 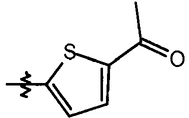 | 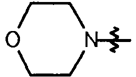 | 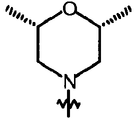 |
| **1ah** | 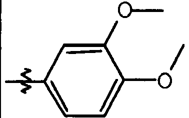 | 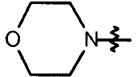 | 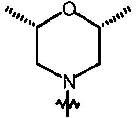 |
| **1ai** | 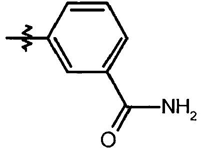 | 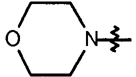 | 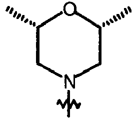 |
| **1aj** | 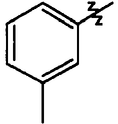 | 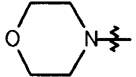 | 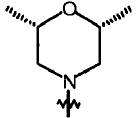 |
| **1ak** | 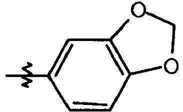 | 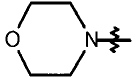 | 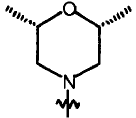 |
| **1al** | 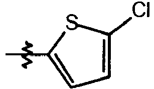 | 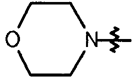 | 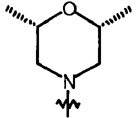 |
| **1am** | 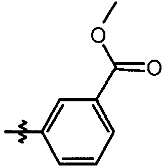 | 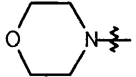 | 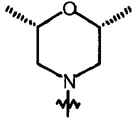 |
| **1an** | 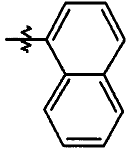 | 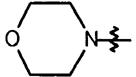 | 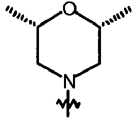 |
| **1ao** | 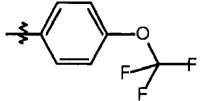 | 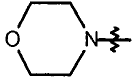 | 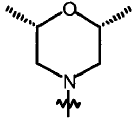 |
| **1ap** | 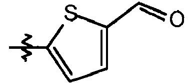 | 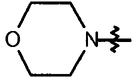 | 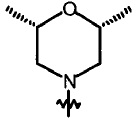 |
| **1aq** | 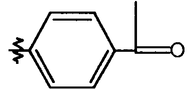 | 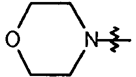 | 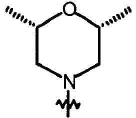 |
| **1ar** | 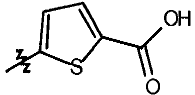 | 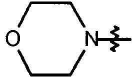 | 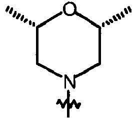 |
| **1as** | 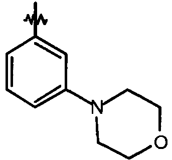 | 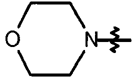 | 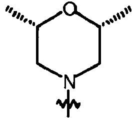 |
| **1at** | 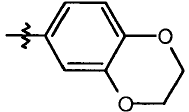 | 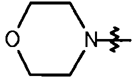 | 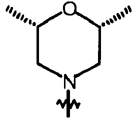 |
| **1au** | 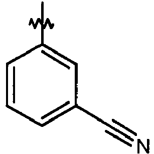 | 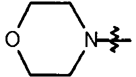 | 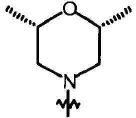 |
| **1av** | 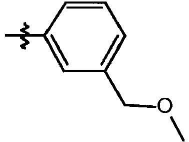 | 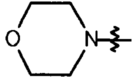 | 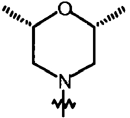 |
| **1aw** | 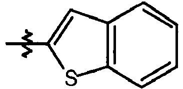 | 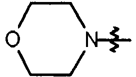 | 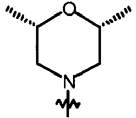 |
| **1ax** | 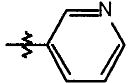 | 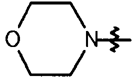 | 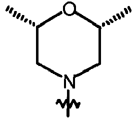 |
| **1ay** | 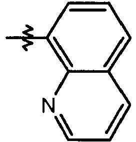 | 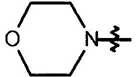 | 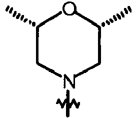 |
| **1az** | 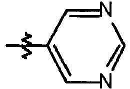 | 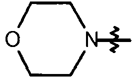 | 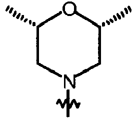 |
| **1ba** | 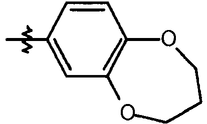 | 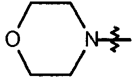 | 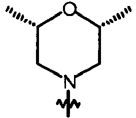 |
| **1bb** | 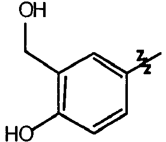 | 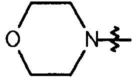 | 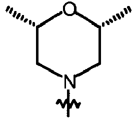 |
| **1bc** | 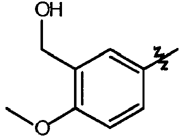 | 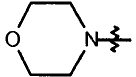 | 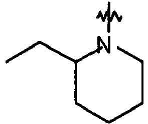 |
| **1bd** | 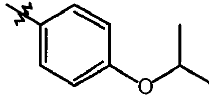 | 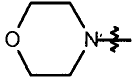 | 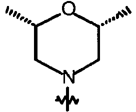 |
| **The** | 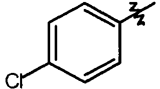 | 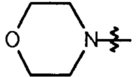 | 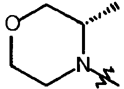 |
| **1bf** | 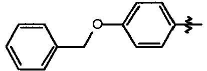 | 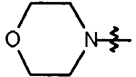 | 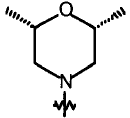 |
| **1bg** | 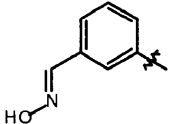 | 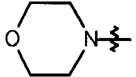 | 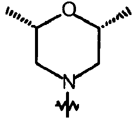 |
| **1bh** | 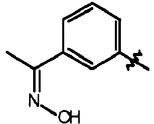 | 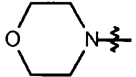 | 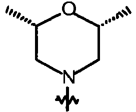 |
| **1bi** | 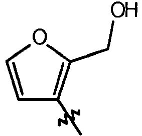 | 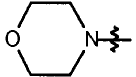 | 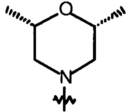 |
| **1bk** | 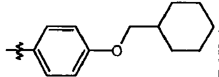 | 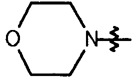 | 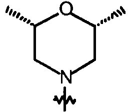 |
| **1bl** | 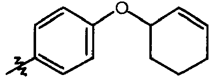 | 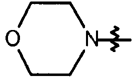 | 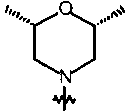 |
| **1bm** | 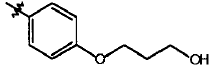 | 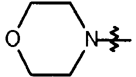 | 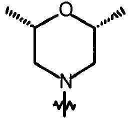 |
| **1bn** | 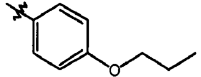 | 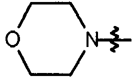 | 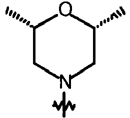 |
| **1bo** | 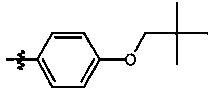 | 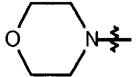 | 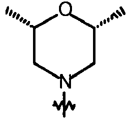 |
| **1bp** | 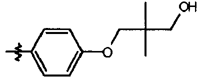 | 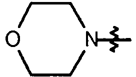 | 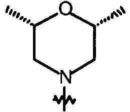 |
| **1bq** | 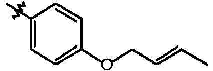 | 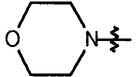 | 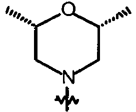 |
| **1br** | 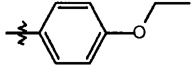 | 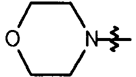 | 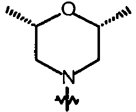 |
| **1bs** | 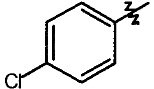 | 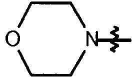 | 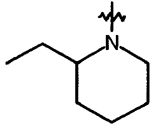 |
| **1bt** | 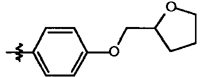 | 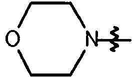 | 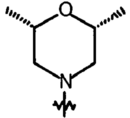 |
| **1bu** | 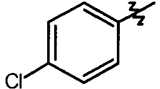 | 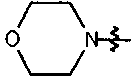 | 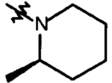 |
| **1bv** | 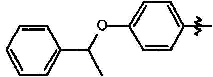 | 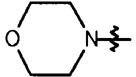 | 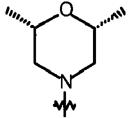 |
| **1bw** | 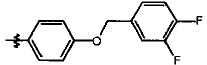 | 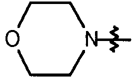 | 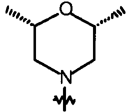 |
| **1bx** | 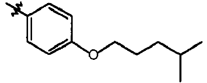 | 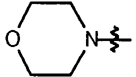 | 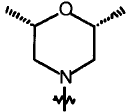 |
| | |
|---|---|
| **8a** | 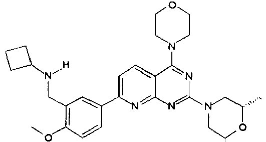 |
| **8b** | 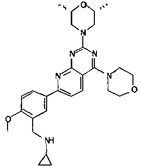 |
| **8c** | 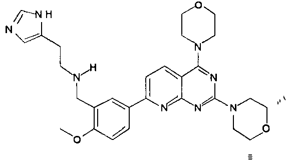 |
| **8d** | 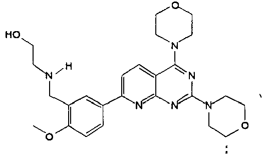 |
| **8e** | 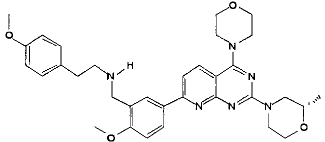 |
| **8f** | 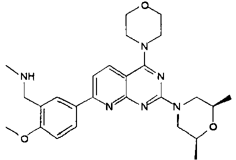 |
| **8g** | 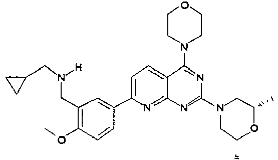 |
| **8h** | 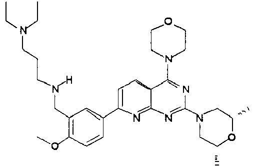 |
| **8i** | 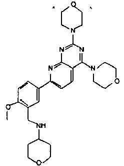 |
| **8j** | 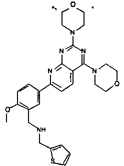 |
| **8k** | 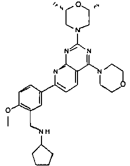 |
| **8l** | 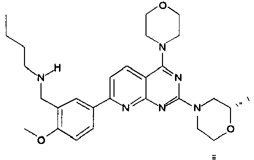 |
| **8m** | 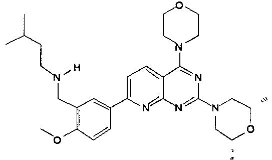 |
| **8n** | 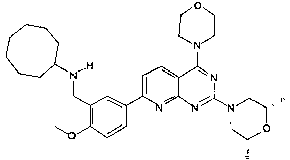 |
| **8o** | 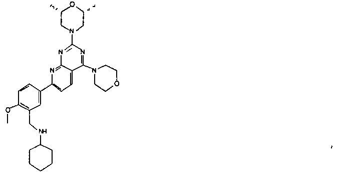 |
| **8p** | 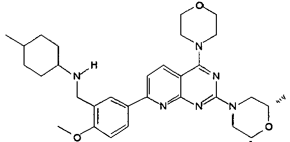 |
| **8q** | 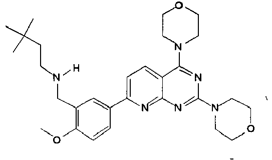 |
| **8r** | 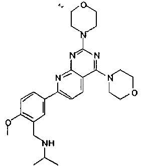 |
| **8s** | 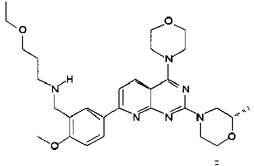 |
| **8t** | 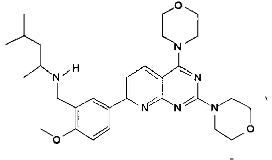 |
| **8u** | 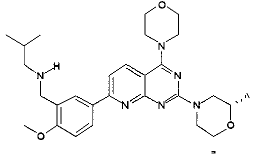 |
| **8v** | 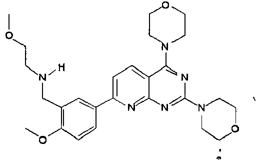 |
| **8w** | 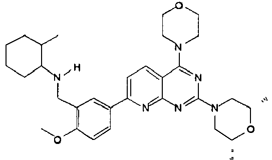 |
| **8x** | 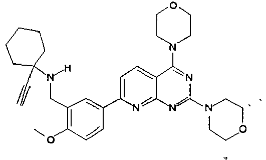 |
| **8y** | 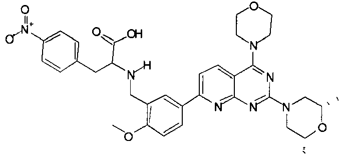 |
| **8z** | 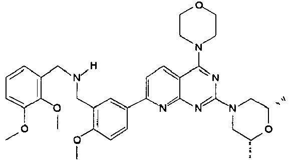 |
| **8aa** | 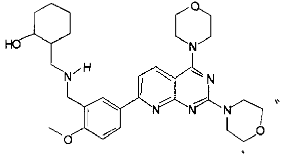 |
| **8ab** | 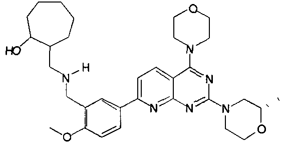 |
| **8ac** | 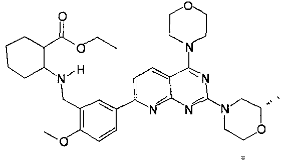 |
| **8ad** | 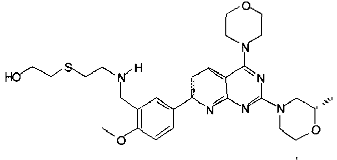 |
| **8ae** | 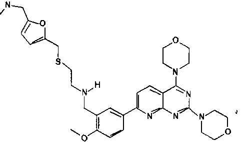 |
| **8af** | 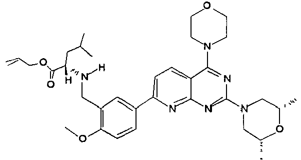 |
| **8ag** | 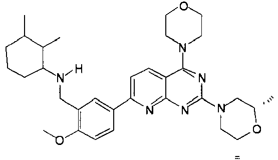 |
| **8ah** | 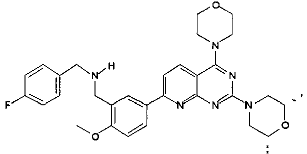 |
| **8ai** | 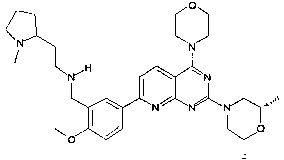 |
| **8aj** | 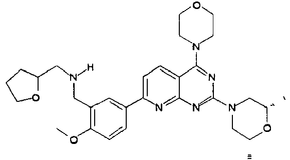 |
| **8ak** | 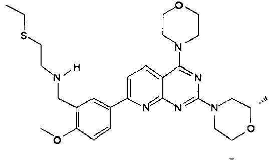 |
| **8al** | 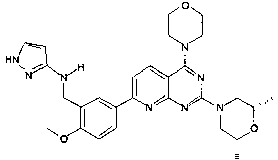 |
| **8am** | 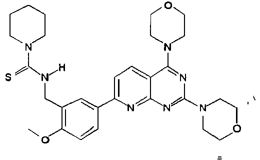 |
| **8an** | 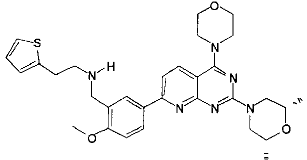 |
| **8ao** | 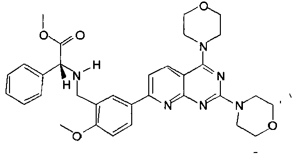 |
| **8ap** | 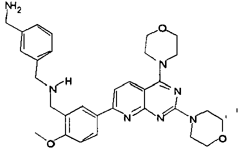 |
| **8aq** | 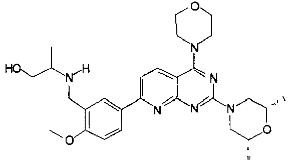 |
| **8ar** | 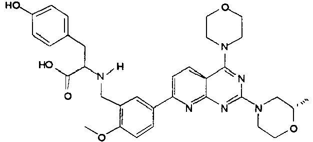 |
| **8as** | 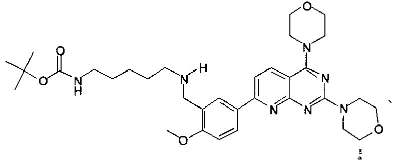 |
| **8at** | 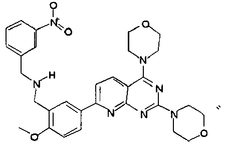 |
| **8au** | 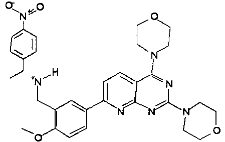 |
| **8av** | 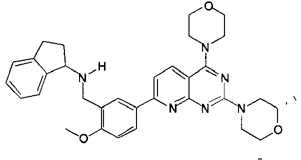 |
| **8aw** | 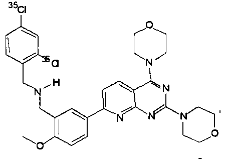 |
| **8ax** | 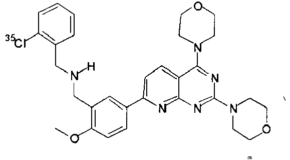 |
| **8ay** | 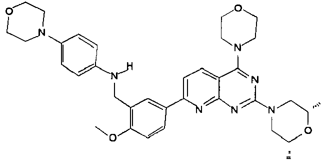 |
| **8az** | 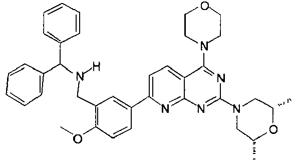 |
| **8ba** | 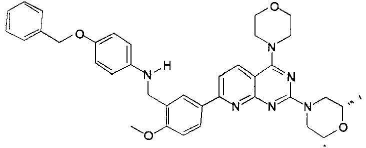 |
| **8bb** | 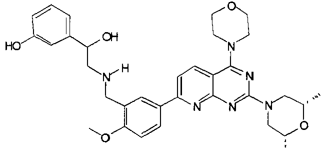 |
| **8bc** | 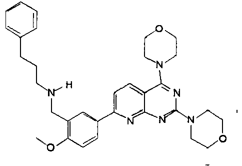 |
| **8bd** | 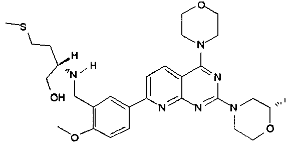 |
| **8be** | 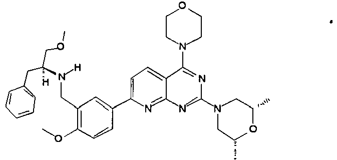 |
| **8bf** | 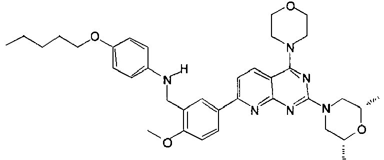 |
| **8bg** | 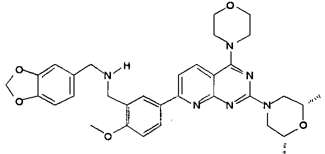 |
| | |
|---|---|
| **9a** | 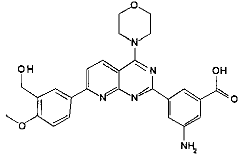 |
| **9b** | 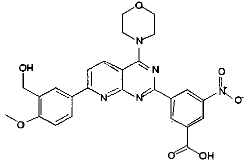 |
| **9c** | 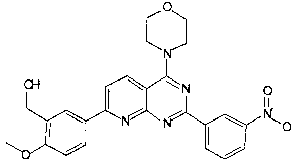 |
| **9d** | 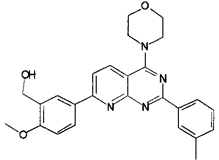 |
| **9e** | 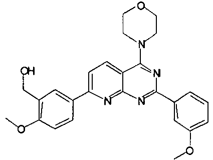 |
| **9f** | 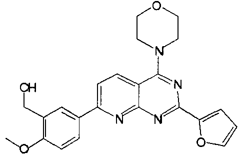 |
| **9g** | 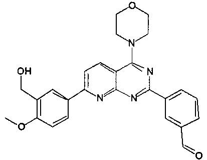 |
| **9h** | 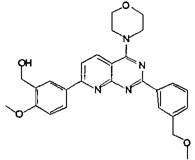 |
| **9i** | 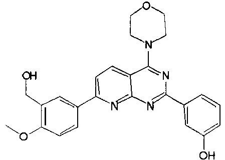 |
| **9j** | 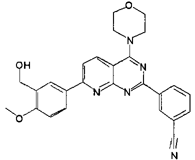 |
| **9k** | 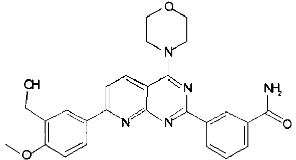 |
| **9l** | 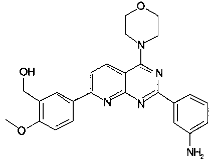 |
| **9m** | 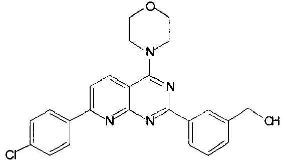 |
| **9n** | 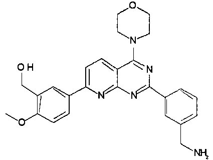 |
| **9o** | 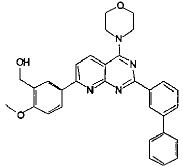 |
| **9p** | 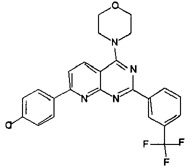 |
| **9q** | 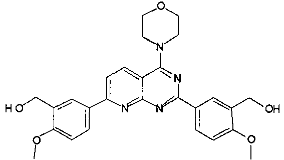 |
| **9r** | 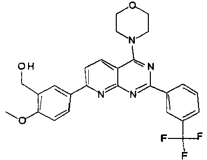 |
| **9s** | 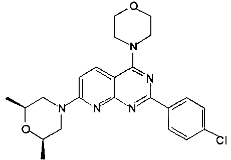 |
| **9t** | 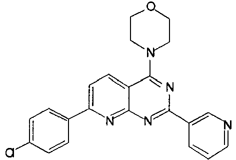 |
| **9u** | 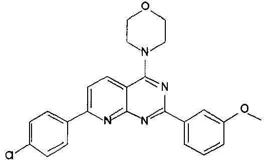 |
| **9v** | 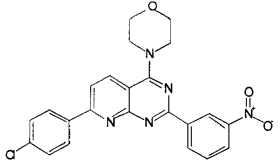 |
| **9w** | 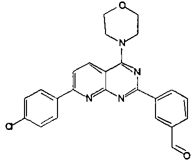 |
| **9x** | 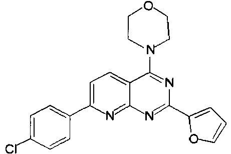 |
| **9y** | 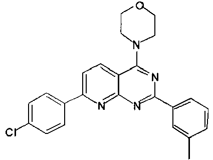 |
| **9z** | 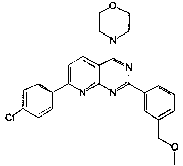 |
| **9aa** | 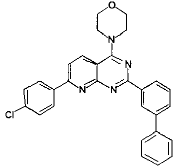 |
| **9ab** | 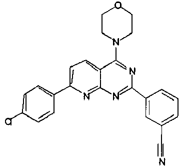 |
| **9ac** | 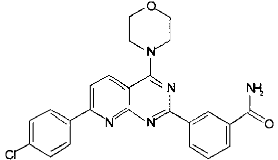 |
| **9ad** | 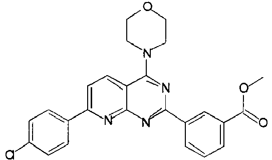 |
| **9ae** | 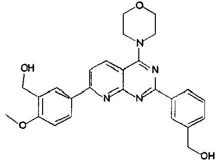 |
, or pharmaceutically acceptable salts thereof.

11. A pharmaceutical composition comprising a compound according to any one of claims 1 to 10, or a pharmaceutically acceptable salt thereof, and a pharmaceutically acceptable carrier or diluent.

12. A compound according to any one of claims 1 to 10, or a pharmaceutically acceptable salt thereof, for use as a medicament.

13. The use of a compound according to any one of claims 1 to 10, or a pharmaceutically acceptable salt thereof, without the provisos in the preparation of a medicament for treating a disease ameliorated by the inhibition of mTOR.

## Patentansprüche

1. Verbindung der Formel I: 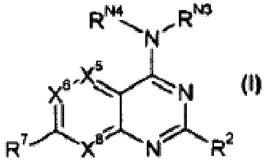 wobei:
X⁸ für N steht und X⁵ und X⁶ für CH stehen;
R⁷ für eine C₅₋₂₀-Arylgruppe, die gegebenenfalls durch eine oder mehrere unter Halogen, Hydroxyl, Nitro, Cyano, Carboxy und Thiol oder C₁₋₇-Alkyl, C₂₋₇-Alkenyl C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, steht;
R^{N3} und R^{N4} zusammen mit dem Stickstoff, an den sie gebunden sind, einen heterocyclischen Ring, der zwischen 3 und 8 Ringatome enthält und gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, bilden;
R² unter NR^{N5}R^{N6}, einer C₅₋₂₀-Heteroarylgruppe, die gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, und einer C₅₋₂₀-Arylgruppe, die gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, ausgewählt ist,
wobei R^{N5} und R^{N6} unabhängig voneinander unter H, einer C₁₋₇-Alkylgruppe, einer C₅₋₂₀-Heteroarylgruppe und einer C₅₋₂₀-Arylgruppe ausgewählt sind oder R^{N5} und R^{N6} zusammen mit dem Stickstoff, an den sie gebunden sind, einen heterocyclischen Ring, der zwischen 3 und 8 Ringatome enthält, bilden, wobei jedes C₁₋₇-Alkyl, jedes C₅₋₂₀-Heteroaryl, jedes C₅₋₂₀-Aryl oder jeder heterocyclische Ring gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist,
oder ein pharmazeutisch annehmbares Salz davon,
und wobei
"C₅₋₂₀-Aryl", wie hier verwendet, eine durch Entfernung eines Wasserstoffatoms von einem aromatischen Ringatom einer C₅₋₂₀-aromatischen Verbindung erhaltene einwertige Gruppierung betrifft, wobei die Verbindung einen Ring oder zwei oder mehr Ringe (z.B. anelliert) und 5 bis 20 Ringatome aufweist und wobei mindestens einer der Ringe ein aromatischer Ring ist und wobei die Ringatome ein oder mehrere Heteroatome einschließlich u.a. Sauerstoff, Stickstoff und Schwefel umfassen können; "Alkyl", wie hier verwendet, eine durch Entfernung eines Wasserstoffatoms von einem Kohlenstoffatom einer Kohlenwasserstoffverbindung mit 1 bis 20 Kohlenstoffatomen (sofern nicht anders angegeben), die aliphatisch oder alicyclisch und gesättigt oder ungesättigt (z.B. teilweise ungesättigt, vollständig ungesättigt) sein kann, erhaltene einwertige Gruppierung betrifft;
"Alkenyl", wie hier verwendet, eine Alkylgruppe mit einer oder mehreren Kohlenstoff-Kohlenstoff-Doppelbindungen umfasst;
"Alkinyl", wie hier verwendet, eine Alkylgruppe mit einer oder mehreren Kohlenstoff-Kohlenstoff-Dreifachbindungen umfasst;
"Cycloalkyl", wie hier verwendet, eine Alkylgruppe betrifft, die auch eine Cyclylgruppe ist; d.h. eine durch Entfernung eines Wasserstoffatoms von einem alicylischen Ringatom eines carbocyclischen Rings einer carbocyclischen Verbindung erhaltene einwertige Gruppierung, wobei der carbocyclische Ring gesättigt oder ungesättigt (z.B. teilweise ungesättigt, vollständig ungesättigt) sein kann, wobei die Gruppierung 3 bis 20 Kohlenstoffatome (sofern nicht anders angegeben) einschließlich 3 bis 20 Ringatomen aufweist; "Ether", wie hier verwendet, eine -OR-Gruppe betrifft, wobei R für eine C₁₋₇-Alkylgruppe, eine C₃₋₂₀-Heterocyclylgruppe oder eine C₅₋₂₀-Arylgruppe steht;
"Acyl", wie hier verwendet, eine -C(=O)R-Gruppe betrifft, wobei R für H, eine C₁-₇-Alkylgruppe, eine C₃₋₂₀-Heterocyclylgruppe oder eine C₅₋₂₀-Arylgruppe steht;
"Ester", wie hier verwendet, eine -C(=O)OR-Gruppe betrifft, wobei R für eine C₁₋₇-Alkylgruppe, eine C₃₋₂₀-Heterocyclylgruppe oder eine C₅₋₂₀-Arylgruppe steht;
"Amido", wie hier verwendet, eine -C(=O)NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅₋₂₀-Arylgruppe stehen oder R¹ und R² zusammen mit dem Stickstoffatom, an das sie gebunden sind, einen heterocyclischen Ring mit 4 bis 8 Ringatomen bilden;
"Amino", wie hier verwendet, eine -NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅₋₂₀-Arylgruppe stehen oder R¹ und R² zusammen mit dem Stickstoffatom, an das sie gebunden sind, einen heterocyclischen Ring mit 4 bis 8 Ringatomen bilden;
"Acylamido", wie hier verwendet, eine -NR¹C(=O)R²-Gruppe betrifft, wobei R¹ für Wasserstoff, eine C₁₋₇-Alkylgruppe, eine C₃₋₂₀-Heterocyclylgruppe oder eine C₅₋₂₀-Arylgruppe steht und R² für eine C₁₋₇-Alkylgruppe, eine C₃₋₂₀-Heterocyclylgruppe oder eine C₅₋₂₀-Arylgruppe steht oder R¹ und R² zusammen mit den Atomen, an die sie gebunden sind, eine Succinimidyl-, Maleinimidyl- und Phthalimidylgruppe bilden;
"Ureido", wie hier verwendet, eine -N(R¹)CONR²R³-Gruppe betrifft, wobei R² und R³ unabhängig voneinander für Wasserstoff, eine C₁₋₇-Alkylgruppe, eine C₃₋₂₀-Heterocyclylgruppe oder eine C₅₋₂₀-Arylgruppe stehen oder R² und R³ zusammen mit dem Stickstoffatom, an das sie gebunden sind, einen heterocyclischen Ring mit 4 bis 8 Ringatomen bilden, und R¹ für Wasserstoff, eine C₁₋₇-Alkylgruppe, eine C₃₋₂₀-Heterocyclylgruppe oder eine C₅₋₂₀-Arylgruppe steht;
"Acyloxy", wie hier verwendet, eine -OC(=O)R-Gruppe betrifft, wobei R für eine C₁₋₇-Alkylgruppe, eine C₃₋₂₀-Heterocyclylgruppe oder eine C₅₋₂₀-Arylgruppe steht;
"Thioether", wie hier verwendet, eine -SR-Gruppe betrifft, wobei R für eine C₁₋₇-Alkylgruppe, eine C₃₋₂₀-Heterocyclylgruppe oder eine C₅₋₂₀-Arylgruppe steht;
"Sulfoxid", wie hier verwendet, eine -S(=O)R-Gruppe betrifft, wobei R für eine C₁₋₇-Alkylgruppe, eine C₃₋₂₀-Heterocyclylgruppe oder eine C₅₋₂₀-Arylgruppe steht;
"Sulfonyl", wie hier verwendet, eine -S(=O)₂R-Gruppe betrifft, wobei R für eine C₁₋₇-Alkylgruppe, eine C₃₋₂₀-Heterocyclylgruppe oder eine C₅₋₂₀-Arylgruppe steht;
"Thioamido", wie hier verwendet, eine -C(=S)NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅₋₂₀-Arylgruppe stehen oder R¹ und R² zusammen mit dem Stickstoffatom, an das sie gebunden sind, einen heterocyclischen Ring mit 4 bis 8 Ringatomen bilden;
"Sulfonamino", wie hier verwendet, eine -NR¹S(=O)₂R-Gruppe betrifft, wobei R¹ für Wasserstoff, eine C₁₋₇-Alkylgruppe, eine C₃₋₂₀-Heterocyclylgruppe oder eine C₅₋₂₀-Arylgruppe steht und R für eine C₁₋₇-Alkylgruppe, eine C₃₋₂₀-Heterocyclylgruppe oder eine C₅₋₂₀-Arylgruppe steht; und
mit der Maßgabe, dass dann, wenn R² für unsubstituiertes Morpholino steht, R^{N3} und R^{N4} zusammen mit dem Stickstoffatom, an das sie gebunden sind, ein unsubstituiertes Morpholino bilden, R⁷ nicht für unsubstituiertes Phenyl steht, und
dann, wenn R² für unsubstituiertes Piperidinyl steht, R^{N3} und R^{N4} zusammen mit dem Stickstoffatom,
an das sie gebunden sind, ein unsubstituiertes Piperidinyl bilden, R⁷ nicht für unsubstituiertes Phenyl steht.

2. Verbindung nach Anspruch 1, wobei R⁷ für eine Phenylgruppe, die gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, steht, wobei die Ringheteroatome jeder C₅₋₂₀-Heteroarylgruppe oder C₃₋₂₀-Heterocyclylgruppe unter Stickstoff, Sauerstoff und Schwefel ausgewählt sind, und wobei
"C₅₋₂₀-Aryl", wie hier verwendet, eine durch Entfernung eines Wasserstoffatoms von einem aromatischen Ringatom einer C₅₋₂₀-aromatischen Verbindung erhaltene einwertige Gruppierung betrifft, wobei die Verbindung einen Ring oder zwei oder mehr Ringe (z.B. anelliert) und 5 bis 20 Ringatome aufweist und wobei mindestens einer der Ringe ein aromatischer Ring ist und wobei die Ringatome ein oder mehrere Heteroatome einschließlich u.a. Sauerstoff, Stickstoff und Schwefel umfassen können; "Alkyl", wie hier verwendet, eine durch Entfernung eines Wasserstoffatoms von einem Kohlenstoffatom einer Kohlenwasserstoffverbindung mit 1 bis 20 Kohlenstoffatomen (sofern nicht anders angegeben), die aliphatisch oder alicyclisch und gesättigt oder ungesättigt (z.B. teilweise ungesättigt, vollständig ungesättigt) sein kann, erhaltene einwertige Gruppierung betrifft;
"Alkenyl", wie hier verwendet, eine Alkylgruppe mit einer oder mehreren Kohlenstoff-Kohlenstoff-Doppelbindungen umfasst;
"Alkinyl", wie hier verwendet, eine Alkylgruppe mit einer oder mehreren Kohlenstoff-Kohlenstoff-Dreifachbindungen umfasst;
"Cycloalkyl", wie hier verwendet, eine Alkylgruppe betrifft, die auch eine Cyclylgruppe ist; d.h. eine durch Entfernung eines Wasserstoffatoms von einem alicylischen Ringatom eines carbocyclischen Rings einer carbocyclischen Verbindung erhaltene einwertige Gruppierung, wobei der carbocyclische Ring gesättigt oder ungesättigt (z.B. teilweise ungesättigt, vollständig ungesättigt) sein kann, wobei die Gruppierung 3 bis 20 Kohlenstoffatome (sofern nicht anders angegeben) einschließlich 3 bis 20 Ringatomen aufweist;
"Ether", wie hier verwendet, eine -OR-Gruppe betrifft, wobei R für eine C₁₋₇-Alkylgruppe, eine C₃₋₂₀-Heterocyclylgruppe oder eine C₅₋₂₀-Arylgruppe steht;
"Acyl", wie hier verwendet, eine -C(=O)R-Gruppe betrifft, wobei R für H, eine C₁₋₇-Alkylgruppe, eine C₃₋₂₀-Heterocyclylgruppe oder eine C₅₋₂₀-Arylgruppe steht;
"Ester", wie hier verwendet, eine -C(=O)OR-Gruppe betrifft, wobei R für eine C₁₋₇-Alkylgruppe, eine C₃₋₂₀-Heterocyclylgruppe oder eine C₅₋₂₀-Arylgruppe steht;
"Amido", wie hier verwendet, eine -C(=O)NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅₋₂₀-Arylgruppe stehen oder R¹ und R² zusammen mit dem Stickstoffatom, an das sie gebunden sind, einen heterocyclischen Ring mit 4 bis 8 Ringatomen bilden;
"Amino", wie hier verwendet, eine -NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅₋₂₀-Arylgruppe stehen oder R¹ und R² zusammen mit dem Stickstoffatom, an das sie gebunden sind, einen heterocyclischen Ring mit 4 bis 8 Ringatomen bilden;
"Acylamido", wie hier verwendet, eine -NR¹C(=O)R²-Gruppe betrifft, wobei R¹ für Wasserstoff, eine C₁₋₇-Alkylgruppe, eine C₃₋₂₀-Heterocyclylgruppe oder eine C₅₋₂₀-Arylgruppe steht und R² für eine C₁₋₇-Alkylgruppe, eine C₃₋₂₀-Heterocyclylgruppe oder eine C₅₋₂₀-Arylgruppe steht oder R¹ und R² zusammen mit den Atomen, an die sie gebunden sind, eine Succinimidyl-, Maleinimidyl- und Phthalimidylgruppe bilden;
"Ureido", wie hier verwendet, eine -N(R¹)CONR²R³-Gruppe betrifft, wobei R² und R³ unabhängig voneinander für Wasserstoff, eine C₁₋₇-Alkylgruppe, eine C₃₋₂₀-Heterocyclylgruppe oder eine C₅₋₂₀-Arylgruppe stehen oder R² und R³ zusammen mit dem Stickstoffatom, an das sie gebunden sind, einen heterocyclischen Ring mit 4 bis 8 Ringatomen bilden, und R¹ für Wasserstoff, eine C₁₋₇-Alkylgruppe, eine C₃₋₂₀-Heterocyclylgruppe oder eine C₅₋₂₀-Arylgruppe steht;
"Acyloxy", wie hier verwendet, eine -OC(=O)R-Gruppe betrifft, wobei R für eine C₁₋₇-Alkylgruppe, eine C₃₋₂₀-Heterocyclylgruppe oder eine C₅₋₂₀-Arylgruppe steht;
"Thioether", wie hier verwendet, eine -SR-Gruppe betrifft, wobei R für eine C₁-₇-Alkylgruppe, eine C₃₋₂₀-Heterocyclylgruppe oder eine C₅₋₂₀-Arylgruppe steht;
"Sulfoxid", wie hier verwendet, eine -S(=O)R-Gruppe betrifft, wobei R für eine C₁₋₇-Alkylgruppe, eine C₃₋₂₀-Heterocyclylgruppe oder eine C₅₋₂₀-Arylgruppe steht;
"Sulfonyl", wie hier verwendet, eine -S(=O)₂R-Gruppe betrifft, wobei R für eine C₁₋₇-Alkylgruppe, eine C₃₋₂₀-Heterocyclylgruppe oder eine C₅₋₂₀-Arylgruppe steht;
"Thioamido", wie hier verwendet, eine -C(=S)NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅₋₂₀-Arylgruppe stehen oder R¹ und R² zusammen mit dem Stickstoffatom, an das sie gebunden sind, einen heterocyclischen Ring mit 4 bis 8 Ringatomen bilden; und
"Sulfonamino", wie hier verwendet, eine -NR¹S(=O)₂R-Gruppe betrifft, wobei R¹ für Wasserstoff, eine C₁₋₇-Alkylgruppe, eine C₃₋₂₀-Heterocyclylgruppe oder eine C₅₋₂₀-Arylgruppe steht und R für eine C₁₋₇-Alkylgruppe, eine C₃₋₂₀-Heterocyclylgruppe oder eine C₅₋₂₀-Arylgruppe steht.

3. Verbindung nach Anspruch 2, wobei R⁷ für eine Phenylgruppe, die gegebenenfalls durch eine oder mehrere unter Chlor, Hydroxyl, Methyl, Methoxy, Ethoxy, i-Propoxy, Benzyloxy und Hydroxymethyl ausgewählte Gruppen substituiert ist, steht.

4. Verbindung nach Anspruch 2, wobei R⁷ für 4-Chlorphenyl, 4-Methylphenyl, 4-Methoxyphenyl, 3-Hydroxymethyl-4-methoxyphenyl, 3,5-Dimethoxy-4-hydroxyphenyl, 4-Hydroxyphenyl, 3-Hydroxyphenyl oder eine 3-Hydroxymethylphenylgruppe steht.

5. Verbindung nach Anspruch 1, wobei es sich bei der Verbindung um eine Verbindung der Formel I(A): 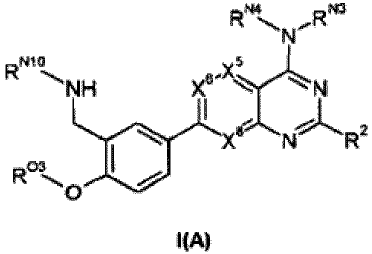 handelt, wobei:
X⁸ für N steht und X⁵ und X⁶ für CH stehen;
R^{N3} und R^{N4} zusammen mit dem Stickstoff, an den sie gebunden sind, einen heterocyclischen Ring, der zwischen 3 und 8 Ringatome enthält und gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, bilden;
R² unter NR^{N5}R^{N6}, einer C₅₋₂₀-Heteroarylgruppe, die gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, und einer C₅₋₂₀-Arylgruppe, die gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, ausgewählt ist,
wobei R^{N5} und R^{N6} unabhängig voneinander unter H, einer C₁₋₇-Alkylgruppe, einer C₅₋₂₀-Heteroarylgruppe und einer C₅₋₂₀-Arylgruppe ausgewählt sind oder R^{N5} und R^{N6} zusammen mit dem Stickstoff, an den sie gebunden sind, einen heterocyclischen Ring, der zwischen 3 und 8 Ringatome enthält, bilden, wobei jedes C₁₋₇-Alkyl, jedes C₅₋₂₀-Heteroaryl, jedes C₅₋₂₀-Aryl oder jeder heterocyclische Ring gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist,
R^{O3} unter Wasserstoff oder einer C₁₋₆-Alkylgruppe, die gegebenenfalls durch eine oder mehrere unter Halogen, Hydroxyl, Nitro, Cyano, Carboxy und Thiol oder C₁-₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, ausgewählt ist und
R^{N10} unter C(=O)R^{C2}, C(=S)R^{C3}, SO₂R^{S3}, einer C₅₋₂₀-Heteroarylgruppe, die gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, einer C₅₋₂₀-Arylgruppe, die gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, oder einer C₁₋₁₀-Alkylgruppe, die gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, ausgewählt ist, wobei R^{C2} und R^{C3} unter H, einer C₅₋₂₀-Arylgruppe, einer C₅₋₂₀-Heteroarylgruppe, einer C₁₋₇-Alkylgruppe oder NR^{N11}R^{N12} ausgewählt sind, wobei R^{N11} und R^{N12} unabhängig voneinander unter H, einer C₁₋₇-Alkylgruppe, einer C₅₋₂₀-Heteroarylgruppe und einer C₅₋₂₀-Arylgruppe ausgewählt sind oder R^{N11} und R^{N12} zusammen mit dem Stickstoff, an den sie gebunden sind, einen heterocyclischen Ring, der zwischen 3 und 8 Ringatome enthält, bilden, wobei jedes C₁₋₇-Alkyl, jedes C₅₋₂₀-Heteroaryl, jedes C₅₋₂₀-Aryl oder jeder heterocyclische Ring gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, und R^{S3} unter H, einer C₅₋₂₀-Arylgruppe, einer C₅₋₂₀-Heteroarylgruppe oder einer C₁₋₇-Alkylgruppe ausgewählt ist, wobei jedes C₁₋₇-Alkyl, jedes C₅₋₂₀-Heteroaryl oder jedes C₅₋₂₀-Aryl gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃-₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist,
oder ein pharmazeutisch annehmbares Salz davon,
und wobei
"C₅₋₂₀-Aryl", wie hier verwendet, eine durch Entfernung eines Wasserstoffatoms von einem aromatischen Ringatom einer C₅₋₂₀-aromatischen Verbindung erhaltene einwertige Gruppierung betrifft, wobei die Verbindung einen Ring oder zwei oder mehr Ringe (z.B. anelliert) und 5 bis 20 Ringatome aufweist und wobei mindestens einer der Ringe ein aromatischer Ring ist und wobei die Ringatome ein oder mehrere Heteroatome einschließlich u.a. Sauerstoff, Stickstoff und Schwefel umfassen können;
"Alkyl", wie hier verwendet, eine durch Entfernung eines Wasserstoffatoms von einem Kohlenstoffatom einer Kohlenwasserstoffverbindung mit 1 bis 20 Kohlenstoffatomen (sofern nicht anders angegeben), die aliphatisch oder alicyclisch und gesättigt oder ungesättigt (z.B. teilweise ungesättigt, vollständig ungesättigt) sein kann, erhaltene einwertige Gruppierung betrifft;
"Alkenyl", wie hier verwendet, eine Alkylgruppe mit einer oder mehreren Kohlenstoff-Kohlenstoff-Doppelbindungen umfasst;
"Alkinyl", wie hier verwendet, eine Alkylgruppe mit einer oder mehreren Kohlenstoff-Kohlenstoff-Dreifachbindungen umfasst;
"Cycloalkyl", wie hier verwendet, eine Alkylgruppe betrifft, die auch eine Cyclylgruppe ist; d.h. eine durch Entfernung eines Wasserstoffatoms von einem alicylischen Ringatom eines carbocyclischen Rings einer carbocyclischen Verbindung erhaltene einwertige Gruppierung, wobei der carbocyclische Ring gesättigt oder ungesättigt (z.B. teilweise ungesättigt, vollständig ungesättigt) sein kann, wobei die Gruppierung 3 bis 20 Kohlenstoffatome (sofern nicht anders angegeben) einschließlich 3 bis 20 Ringatomen aufweist; "Ether", wie hier verwendet, eine -OR-Gruppe betrifft, wobei R für eine C₁₋₇-Alkylgruppe, eine C₃₋₂₀-Heterocyclylgruppe oder eine C₅₋₂₀-Arylgruppe steht;
"Acyl", wie hier verwendet, eine -C(=O)R-Gruppe betrifft, wobei R für H, eine C₁₋₇-Alkylgruppe, eine C₃₋₂₀-Heterocyclylgruppe oder eine C₅₋₂₀-Arylgruppe steht;
"Ester", wie hier verwendet, eine -C(=O)OR-Gruppe betrifft, wobei R für eine C₁₋₇-Alkylgruppe, eine C₃₋₂₀-Heterocyclylgruppe oder eine C₅₋₂₀-Arylgruppe steht;
"Amido" , wie hier verwendet, eine -C (=O)NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅-₂₀-Arylgruppe stehen oder R¹ und R² zusammen mit dem Stickstoffatom, an das sie gebunden sind, einen heterocyclischen Ring mit 4 bis 8 Ringatomen bilden;
"Amino", wie hier verwendet, eine -NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅₋₂₀-Arylgruppe stehen oder R¹ und R² zusammen mit dem Stickstoffatom, an das sie gebunden sind, einen heterocyclischen Ring mit 4 bis 8 Ringatomen bilden;
"Acylamido", wie hier verwendet, eine -NR¹C (=O)R²-Gruppe betrifft, wobei R¹ für Wasserstoff, eine C₁₋₇-Alkylgruppe, eine C₃₋₂₀-Heterocyclylgruppe oder eine C₅₋₂₀-Arylgruppe steht und R² für eine C₁₋₇-Alkylgruppe, eine C₃₋₂₀-Heterocyclylgruppe oder eine C₅₋₂₀-Arylgruppe steht oder R¹ und R² zusammen mit den Atomen, an die sie gebunden sind, eine Succinimidyl-, Maleinimidyl- und Phthalimidylgruppe bilden;
"Ureido", wie hier verwendet, eine -N(R¹)CONR²R³-Gruppe betrifft, wobei R² und R³ unabhängig voneinander für Wasserstoff, eine C₁₋₇-Alkylgruppe, eine C₃₋₂₀-Heterocyclylgruppe oder eine C₅₋₂₀-Arylgruppe stehen oder R² und R³ zusammen mit dem Stickstoffatom, an das sie gebunden sind, einen heterocyclischen Ring mit 4 bis 8 Ringatomen bilden, und R¹ für Wasserstoff, eine C₁₋₇-Alkylgruppe, eine C₃₋₂₀-Heterocyclylgruppe oder eine C₅₋₂₀-Arylgruppe steht;
"Acyloxy", wie hier verwendet, eine -OC(=O)R-Gruppe betrifft, wobei R für eine C₁₋₇-Alkylgruppe, eine C₃₋₂₀-Heterocyclylgruppe oder eine C₅₋₂₀-Arylgruppe steht;
"Thioether", wie hier verwendet, eine -SR-Gruppe betrifft, wobei R für eine C₁₋₇-Alkylgruppe, eine C₃₋₂₀-Heterocyclylgruppe oder eine C₅₋₂₀-Arylgruppe steht;
"Sulfoxid", wie hier verwendet, eine -S(=O)R-Gruppe betrifft, wobei R für eine C₁₋₇-Alkylgruppe, eine C₃₋₂₀-Heterocyclylgruppe oder eine C₅₋₂₀-Arylgruppe steht;
"Sulfonyl", wie hier verwendet, eine -S(=O)₂R-Gruppe betrifft, wobei R für eine C₁₋₇-Alkylgruppe, eine C₃₋₂₀-Heterocyclylgruppe oder eine C₅₋₂₀-Arylgruppe steht;
"Thioamido", wie hier verwendet, eine -C(=S)NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅₋₂₀-Arylgruppe stehen oder R¹ und R² zusammen mit dem Stickstoffatom, an das sie gebunden sind, einen heterocyclischen Ring mit 4 bis 8 Ringatomen bilden;
"Sulfonamino", wie hier verwendet, eine -NR¹S(=O)₂R-Gruppe betrifft, wobei R¹ für Wasserstoff, eine C₁₋₇-Alkylgruppe, eine C₃₋₂₀-Heterocyclylgruppe oder eine C₅₋₂₀-Arylgruppe steht und R für eine C₁₋₇-Alkylgruppe, eine C₃₋₂₀-Heterocyclylgruppe oder eine C₅₋₂₀-Arylgruppe steht.

6. Verbindung nach einem der Ansprüche 1 bis 5, wobei R^{N3} und R^{N4} zusammen mit dem Stickstoff, an den sie gebunden sind, eine Morpholinogruppe bilden.

7. Verbindung nach einem der Ansprüche 1 bis 6, wobei R² für NR^{N5}R^{N6} steht, wobei R^{N5} und R^{N6} zusammen mit dem Stickstoff, an den sie gebunden sind, einen heterocyclischen Ring bilden, der zwischen 3 und 8 Ringatome enthält, und gegebenenfalls durch eine oder mehrere unter Halogen, Hydroxyl, Nitro, Cyano, Carboxy und Thiol oder C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino (jeweils gegebenenfalls substituiert durch eine oder mehrere unter Halogen, Hydroxyl, Nitro, Cyano, Carboxy, Thiol, C₁₋₇-Alkyl, C₂₋₇-Alkenyl, C₂₋₇-Alkinyl, C₃₋₇-Cycloalkyl, C₃₋₇-Cycloalkenyl, C₃₋₂₀-Heterocyclyl, C₅₋₂₀-Aryl, C₅₋₂₀-Heteroaryl, Ether, Acyl, Ester, Amido, Amino, Acylamido, Ureido, Acyloxy, Thioether, Sulfoxid, Sulfonyl, Thioamido und Sulfonamino ausgewählte Gruppen) ausgewählte Gruppen substituiert ist, und wobei die Ringheteroatome jeder C₅₋₂₀-Heteroarylgruppe, jeder C₃₋₂₀-Heterocyclylgruppe oder jedes heterocyclischen Rings mit 3 bis 8 Ringatomen unter Stickstoff, Sauerstoff und Schwefel ausgewählt sind, und
wobei
"C₅-₂₀-Aryl", wie hier verwendet, eine durch Entfernung eines Wasserstoffatoms von einem aromatischen Ringatom einer C₅₋₂₀-aromatischen Verbindung erhaltene einwertige Gruppierung betrifft, wobei die Verbindung einen Ring oder zwei oder mehr Ringe (z.B. anelliert) und 5 bis 20 Ringatome aufweist und wobei mindestens einer der Ringe ein aromatischer Ring ist und wobei die Ringatome ein oder mehrere Heteroatome einschließlich u.a. Sauerstoff, Stickstoff und Schwefel umfassen können; "Alkyl", wie hier verwendet, eine durch Entfernung eines Wasserstoffatoms von einem Kohlenstoffatom einer Kohlenwasserstoffverbindung mit 1 bis 20 Kohlenstoffatomen (sofern nicht anders angegeben), die aliphatisch oder alicyclisch und gesättigt oder ungesättigt (z.B. teilweise ungesättigt, vollständig ungesättigt) sein kann, erhaltene einwertige Gruppierung betrifft;
"Alkenyl", wie hier verwendet, eine Alkylgruppe mit einer oder mehreren Kohlenstoff-Kohlenstoff-Doppelbindungen umfasst;
"Alkinyl", wie hier verwendet, eine Alkylgruppe mit einer oder mehreren Kohlenstoff-Kohlenstoff-Dreifachbindungen umfasst;
"Cycloalkyl", wie hier verwendet, eine Alkylgruppe betrifft, die auch eine Cyclylgruppe ist; d.h. eine durch Entfernung eines Wasserstoffatoms von einem alicylischen Ringatom eines carbocyclischen Rings einer carbocyclischen Verbindung erhaltene einwertige Gruppierung, wobei der carbocyclische Ring gesättigt oder ungesättigt (z.B. teilweise ungesättigt, vollständig ungesättigt) sein kann, wobei die Gruppierung 3 bis 20 Kohlenstoffatome (sofern nicht anders angegeben) einschließlich 3 bis 20 Ringatomen aufweist; "Ether", wie hier verwendet, eine -OR-Gruppe betrifft, wobei R für eine C₁₋₇-Alkylgruppe, eine C₃₋₂₀-Heterocyclylgruppe oder eine C₅₋₂₀-Arylgruppe steht;
"Acyl", wie hier verwendet, eine -C(=O)R-Gruppe betrifft, wobei R für H, eine C₁₋₇-Alkylgruppe, eine C₃₋₂₀-Heterocyclylgruppe oder eine C₅₋₂₀-Arylgruppe steht;
"Ester", wie hier verwendet, eine -C(=O)OR-Gruppe betrifft, wobei R für eine C₁₋₇-Alkylgruppe, eine C₃₋₂₀-Heterocyclylgruppe oder eine C₅₋₂₀-Arylgruppe steht;
"Amido", wie hier verwendet, eine -C(=O)NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅₋₂₀-Arylgruppe stehen oder R¹ und R² zusammen mit dem Stickstoffatom, an das sie gebunden sind, einen heterocyclischen Ring mit 4 bis 8 Ringatomen bilden;
"Amino", wie hier verwendet, eine -NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅₋₂₀-Arylgruppe stehen oder R¹ und R² zusammen mit dem Stickstoffatom, an das sie gebunden sind, einen heterocyclischen Ring mit 4 bis 8 Ringatomen bilden;
"Acylamido", wie hier verwendet, eine -NR¹C(=O)R²-Gruppe betrifft, wobei R¹ für Wasserstoff, eine C₁₋₇-Alkylgruppe, eine C₃₋₂₀-Heterocyclylgruppe oder eine C₅₋₂₀-Arylgruppe steht und R² für eine C₁₋₇-Alkylgruppe, eine C₃₋₂₀-Heterocyclylgruppe oder eine C₅₋₂₀-Arylgruppe steht oder R¹ und R² zusammen mit den Atomen, an die sie gebunden sind, eine Succinimidyl-, Maleinimidyl- und Phthalimidylgruppe bilden;
"Ureido", wie hier verwendet, eine -N(R¹)CONR²R³-Gruppe betrifft, wobei R² und R³ unabhängig voneinander für Wasserstoff, eine C₁₋₇-Alkylgruppe, eine C₃₋₂₀-Heterocyclylgruppe oder eine C₅₋₂₀-Arylgruppe stehen oder R² und R³ zusammen mit dem Stickstoffatom, an das sie gebunden sind, einen heterocyclischen Ring mit 4 bis 8 Ringatomen bilden, und R¹ für Wasserstoff, eine C₁₋₇-Alkylgruppe, eine C₃₋₂₀-Heterocyclylgruppe oder eine C₅₋₂₀-Arylgruppe steht;
"Acyloxy", wie hier verwendet, eine -OC(=O)R-Gruppe betrifft, wobei R für eine C₁₋₇-Alkylgruppe, eine C₃₋₂₀-Heterocyclylgruppe oder eine C₅₋₂₀-Arylgruppe steht;
"Thioether", wie hier verwendet, eine -SR-Gruppe betrifft, wobei R für eine C₁₋₇-Alkylgruppe, eine C₃₋₂₀-Heterocyclylgruppe oder eine C₅₋₂₀-Arylgruppe steht;
"Sulfoxid", wie hier verwendet, eine -S(=O)R-Gruppe betrifft, wobei R für eine C₁₋₇-Alkylgruppe, eine C₃₋₂₀-Heterocyclylgruppe oder eine C₅₋₂₀-Arylgruppe steht;
"Sulfonyl", wie hier verwendet, eine -S(=O)₂R-Gruppe betrifft, wobei R für eine C₁₋₇-Alkylgruppe, eine C₃₋₂₀-Heterocyclylgruppe oder eine C₅₋₂₀-Arylgruppe steht;
"Thioamido", wie hier verwendet, eine -C(=S)NR¹R²-Gruppe betrifft, wobei R¹ und R² unabhängig voneinander für Wasserstoff, eine C₁₋₇-Alkylgruppe, eine C₃₋₂₀-Heterocyclylgruppe oder eine C₅₋₂₀-Arylgruppe stehen oder R¹ und R² zusammen mit dem Stickstoffatom, an das sie gebunden sind, einen heterocyclischen Ring mit 4 bis 8 Ringatomen bilden;
"Sulfonamino", wie hier verwendet, eine -NR¹S(=O)₂R-Gruppe betrifft, wobei R¹ für Wasserstoff, eine C₁₋₇-Alkylgruppe, eine C₃₋₂₀-Heterocyclylgruppe oder eine C₅₋₂₀-Arylgruppe steht und R für eine C₁₋₇-Alkylgruppe, eine C₃₋₂₀-Heterocyclylgruppe oder eine C₅₋₂₀-Arylgruppe steht.

8. Verbindung nach Anspruch 7, wobei R² unter Morpholino, Thiomorpholino, Piperidinyl, Piperazinyl, Homopiperazinyl und Pyrrolidinyl ausgewählt ist, wobei jedes Morpholino, Thiomorpholino, Piperidinyl, Piperazinyl, Homopiperazinyl und Pyrrolidinyl gegebenenfalls an Kohlenstoff durch eine oder mehrere Methylgruppen substituiert sein kann.

9. Verbindung nach Anspruch 8, wobei R² für 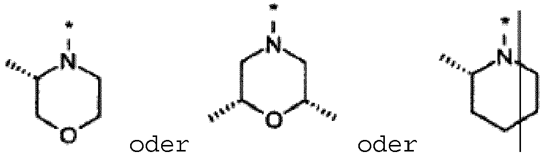 steht.

10. Verbindung nach Anspruch 1, ausgewählt unter einem der folgenden Beispiele:
| | **R⁷** | **NR³R⁴** | **R²** |
|---|---|---|---|
| **1a** | 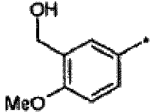 | 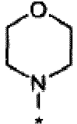 | 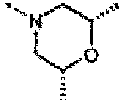 |
| **1b** | 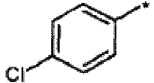 | 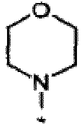 | 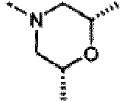 |
| **1c** | 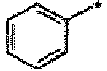 | 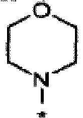 | 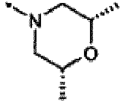 |
| **1d** | 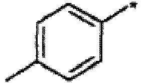 | 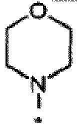 | 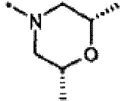 |
| **1e** | 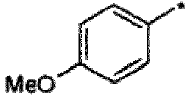 | 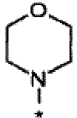 | 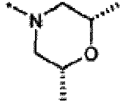 |
| **1f** | 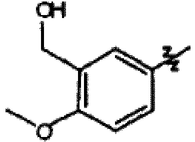 | 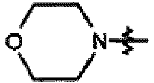 | 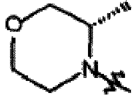 |
| **1g** | 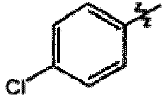 | 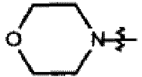 | 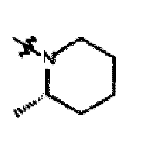 |
| **1h** | 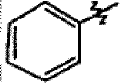 | 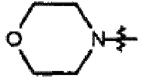 | 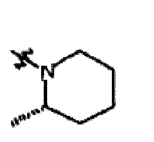 |
| **1i** | 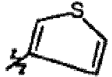 | 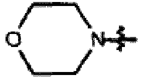 | 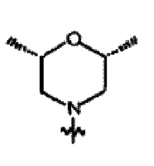 |
| **1j** | 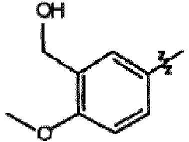 | 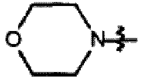 | 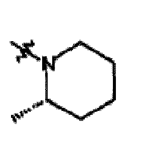 |
| **1k** | 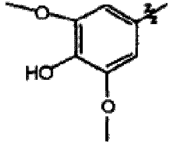 | 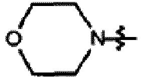 | 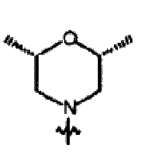 |
| **1l** | 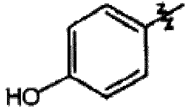 | 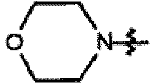 | 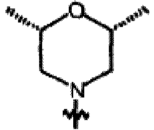 |
| **1m** | 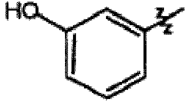 | 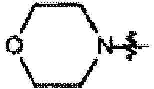 | 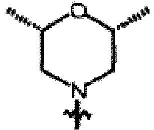 |
| **1n** | 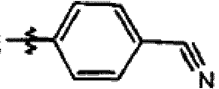 | 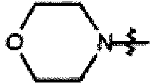 | 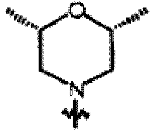 |
| **1o** | 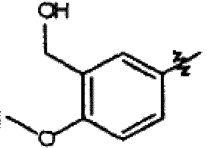 | 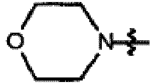 | 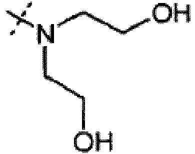 |
| **1p** | 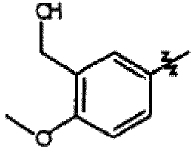 | 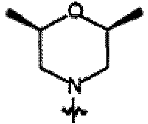 | 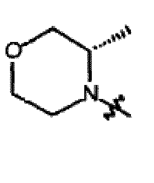 |
| **1q** | 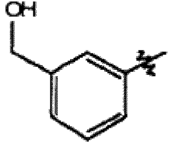 | 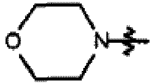 | 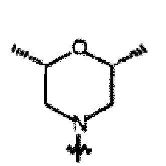 |
| **1r** | 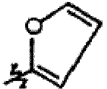 | 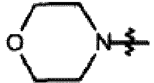 | 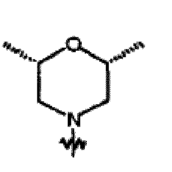 |
| **1s** | 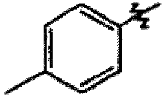 | 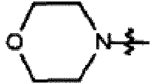 | 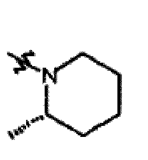 |
| **1t** | 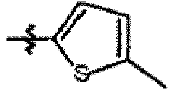 | 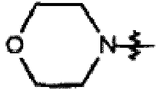 | 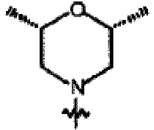 |
| **1u** | 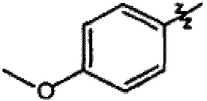 | 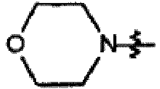 | 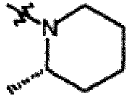 |
| **1v** | 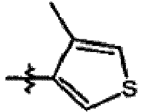 | 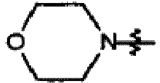 | 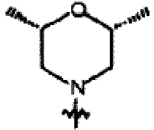 |
| **1w** | 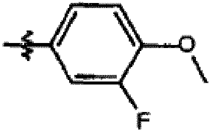 | 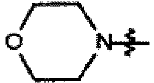 | 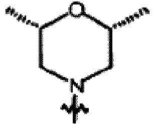 |
| **1x** | 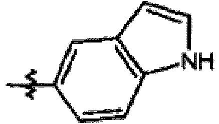 | 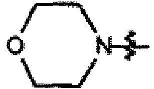 | 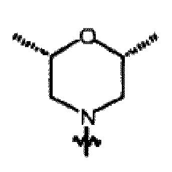 |
| **1y** | 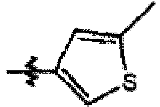 | 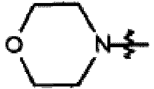 | 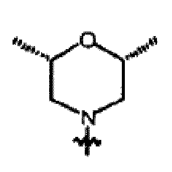 |
| **1z** | 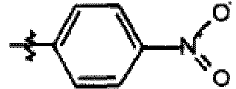 | 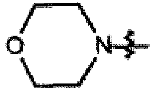 | 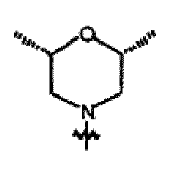 |
| **1aa** | 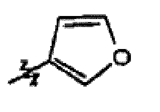 | 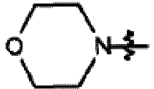 | 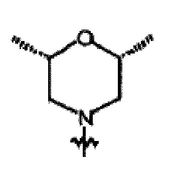 |
| **1ab** | 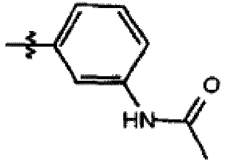 | 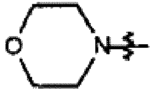 | 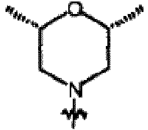 |
| **1ac** | 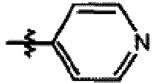 | 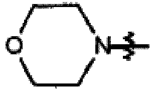 | 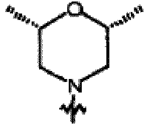 |
| **1ad** | 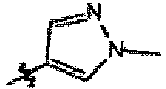 | 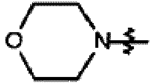 | 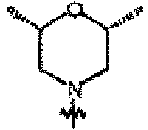 |
| **1ae** | 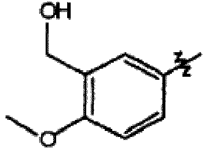 | 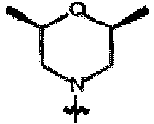 | 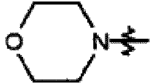 |
| **1af** | 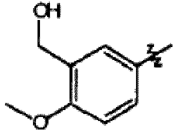 | 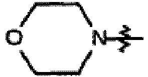 | 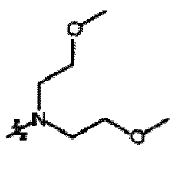 |
| **1ag** | 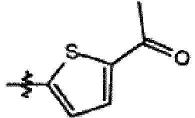 | 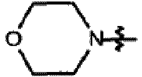 | 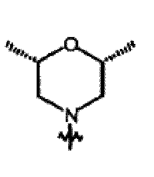 |
| **1ah** | 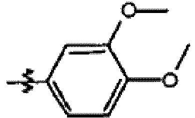 | 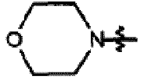 | 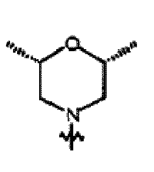 |
| **1ai** | 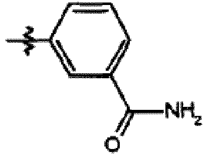 | 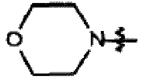 | 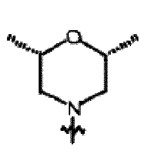 |
| **1aj** | 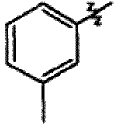 | 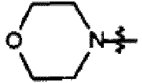 | 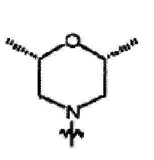 |
| **1ak** | 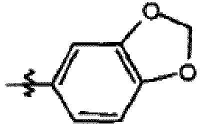 | 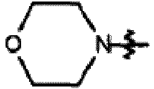 | 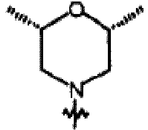 |
| **1al** | 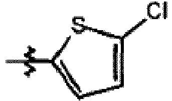 | 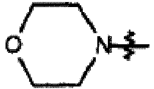 | 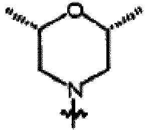 |
| **1am** | 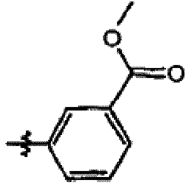 | 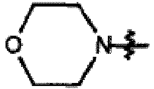 | 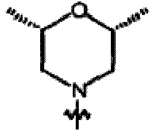 |
| **1an** | 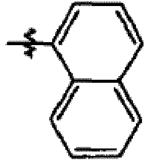 | 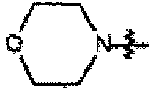 | 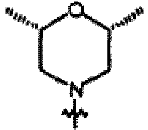 |
| **1ao** | 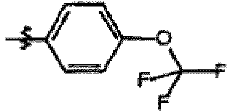 | 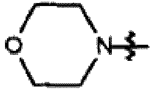 | 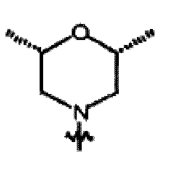 |
| **1ap** | 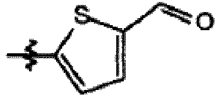 | 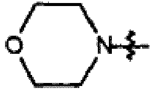 | 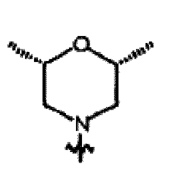 |
| **1aq** | 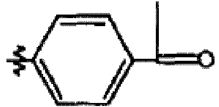 | 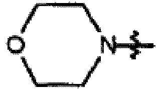 | 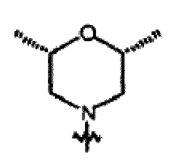 |
| **1ar** | 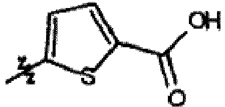 | 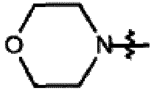 | 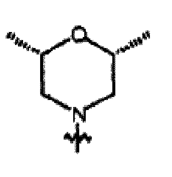 |
| **1as** | 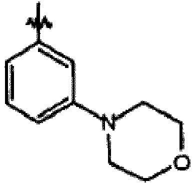 | 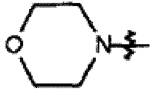 | 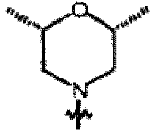 |
| **1at** | 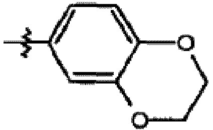 | 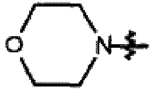 | 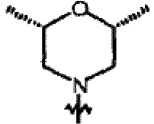 |
| **1au** | 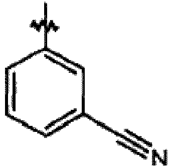 | 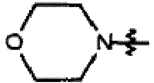 | 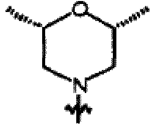 |
| **1av** | 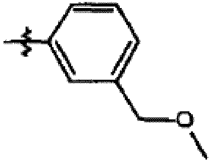 | 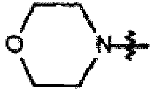 | 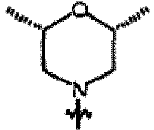 |
| **1aw** | 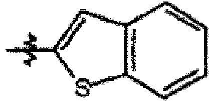 | 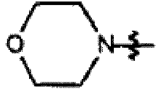 | 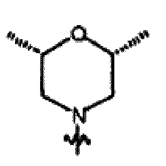 |
| **1ax** | 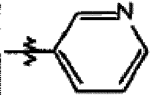 | 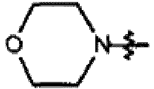 | 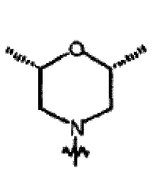 |
| **1ay** | 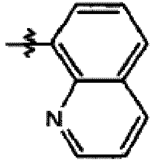 | 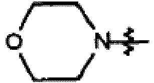 | 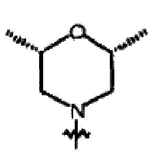 |
| **1az** | 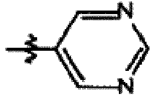 | 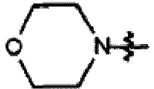 | 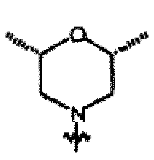 |
| **1ba** | 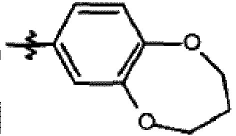 | 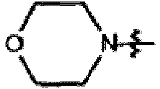 | 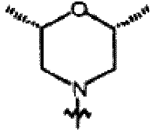 |
| **1bb** | 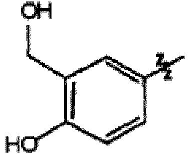 | 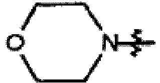 | 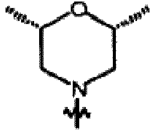 |
| **1bc** | 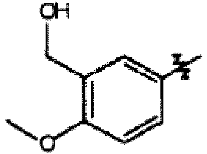 | 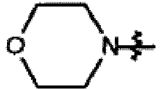 | 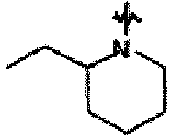 |
| **1bd** | 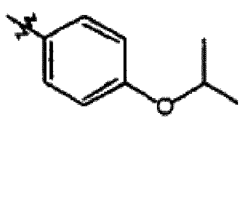 | 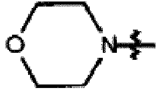 | 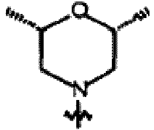 |
| **1be** | 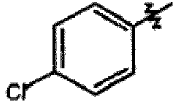 | 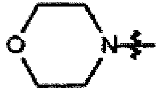 | 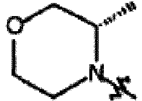 |
| **1bf** | 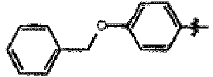 | 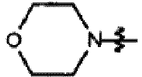 | 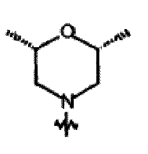 |
| **1bg** | 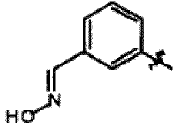 | 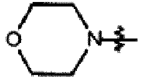 | 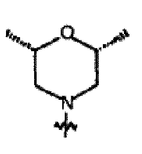 |
| **1bh** | 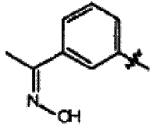 | 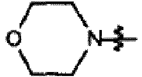 | 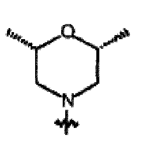 |
| **1bi** | 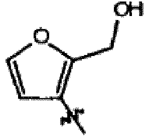 | 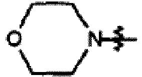 | 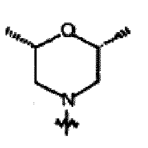 |
| **1bk** | 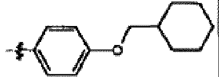 | 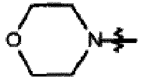 | 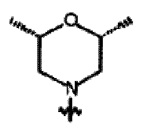 |
| **1bl** | 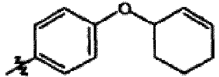 | 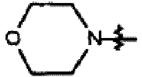 | 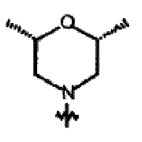 |
| **1bm** | 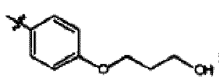 | 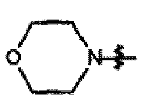 | 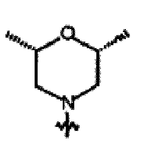 |
| **1bn** | 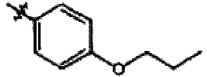 | 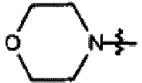 | 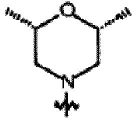 |
| **1bo** | 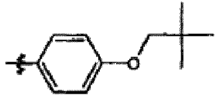 | 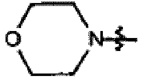 | 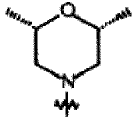 |
| **1bp** | 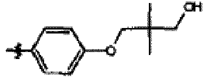 | 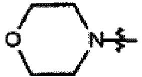 | 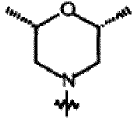 |
| **1bq** | 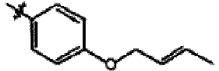 | 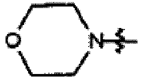 | 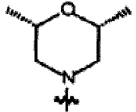 |
| **1br** | 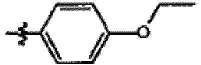 | 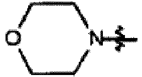 | 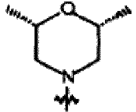 |
| **1bs** | 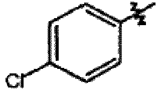 | 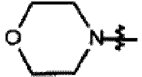 | 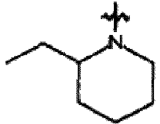 |
| **1bt** | 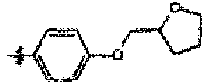 | 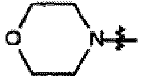 | 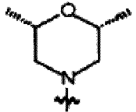 |
| **1bu** | 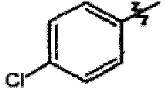 | 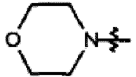 | 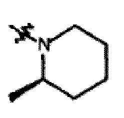 |
| **1bv** | 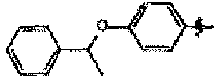 | 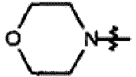 | 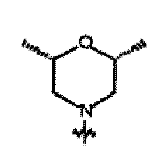 |
| **1bw** | 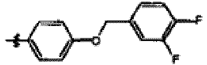 | 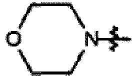 | 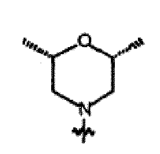 |
| **1bx** | 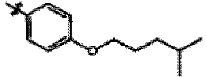 | 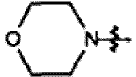 | 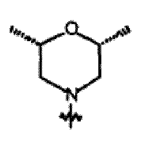 |
| | |
|---|---|
| **8a** | 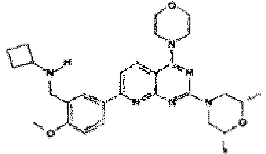 |
| **8b** | 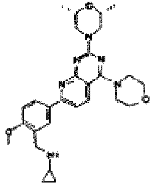 |
| **8c** | 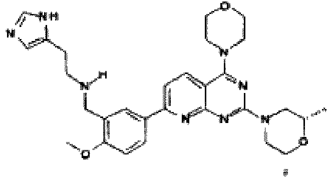 |
| **8d** | 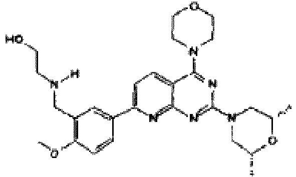 |
| **8e** | 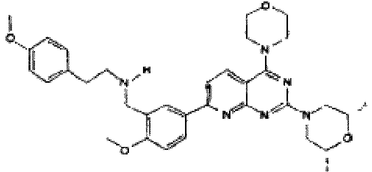 |
| **8f** | 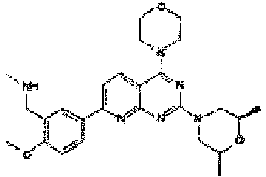 |
| **8g** | 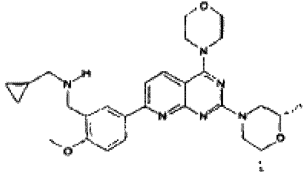 |
| **8h** | 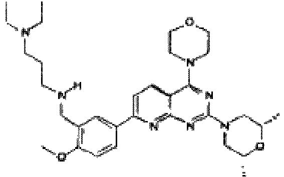 |
| **8i** | 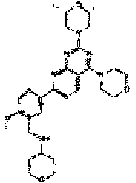 |
| **8j** | 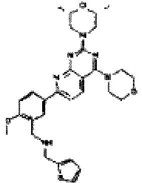 |
| **8k** | 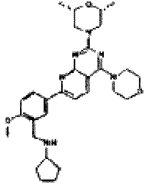 |
| **8l** | 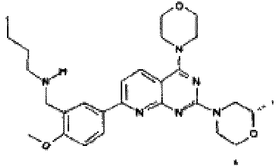 |
| **8m** | 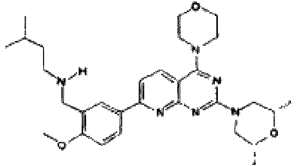 |
| **8n** | 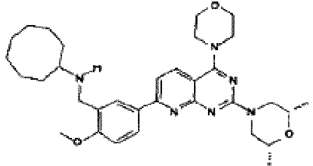 |
| **8o** | 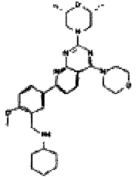 |
| **8p** | 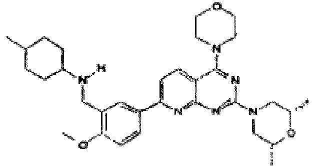 |
| **8q** | 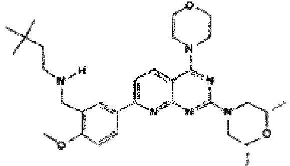 |
| **8r** | 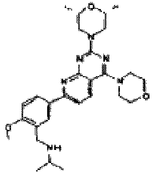 |
| **8s** | 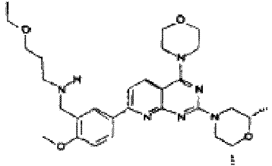 |
| **8t** | 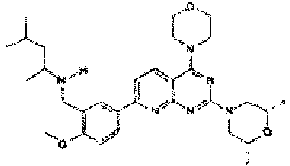 |
| **8u** | 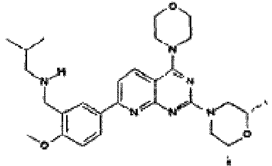 |
| **8v** | 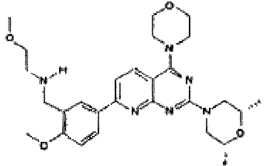 |
| **8w** | 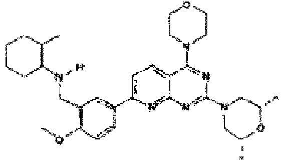 |
| **8x** | 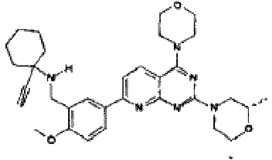 |
| **8y** | 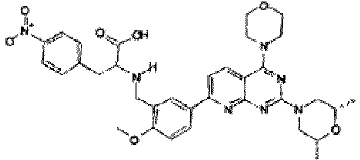 |
| **8z** | 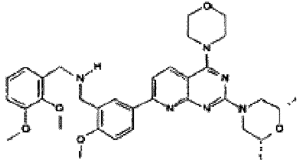 |
| **8aa** | 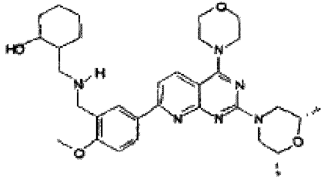 |
| **8ab** | 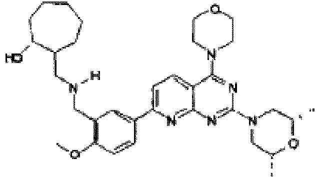 |
| **8ac** | 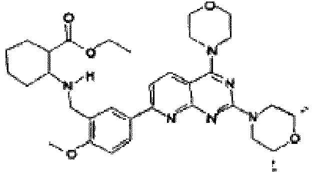 |
| **8ad** | 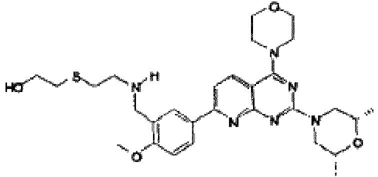 |
| **8ae** | 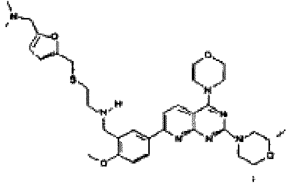 |
| **8af** | 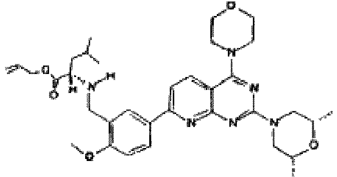 |
| **8ag** | 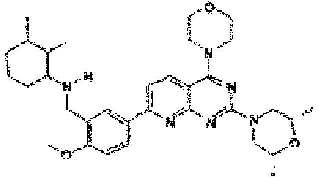 |
| **8ah** | 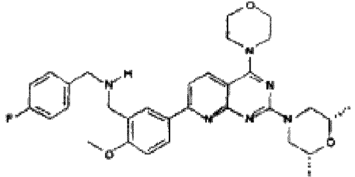 |
| **8ai** | 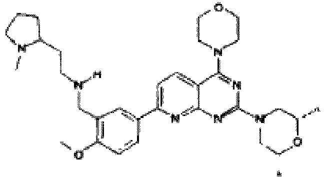 |
| **8aj** | 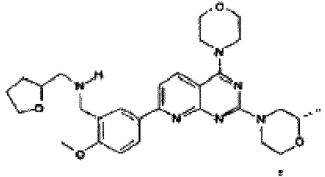 |
| **8ak** | 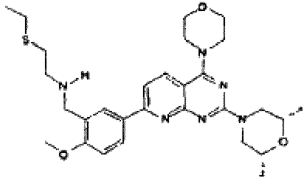 |
| **8al** | 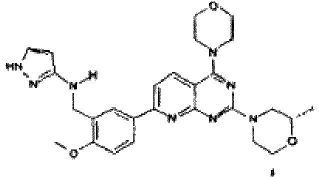 |
| **8am** | 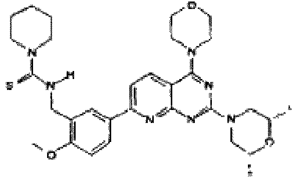 |
| **8an** | 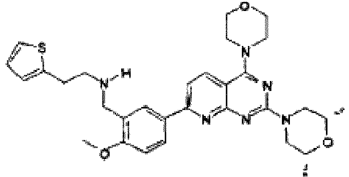 |
| **8ao** | 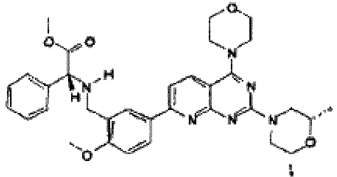 |
| **8ap** | 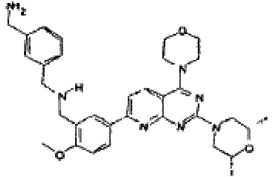 |
| **8aq** | 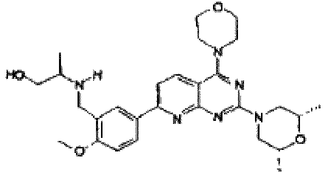 |
| **8ar** | 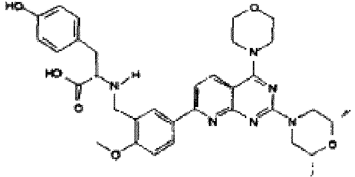 |
| **8as** | 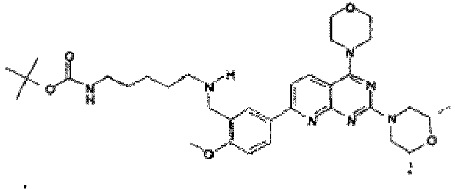 |
| **8at** | 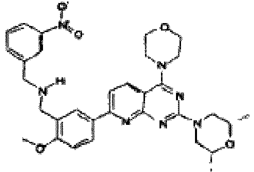 |
| **8au** | 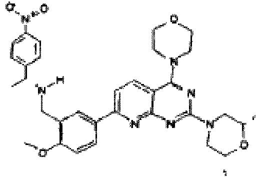 |
| **8av** | 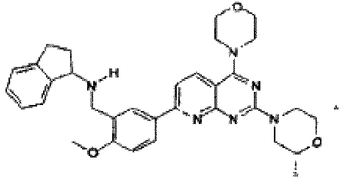 |
| **8aw** | 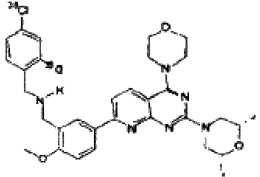 |
| **8ax** | 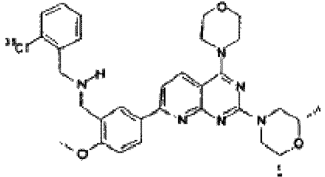 |
| **8ay** | 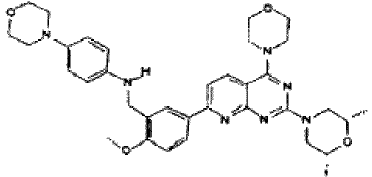 |
| **8az** | 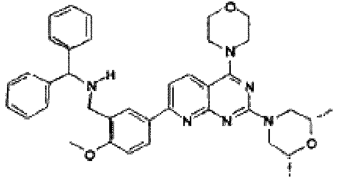 |
| **8ba** | 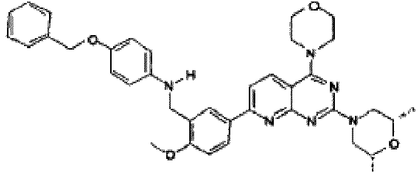 |
| **8bb** | 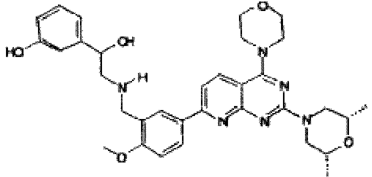 |
| **8bc** | 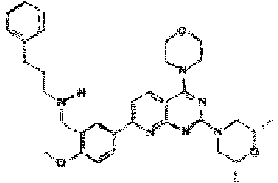 |
| **8bd** | 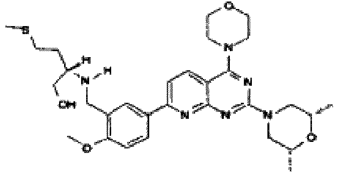 |
| **8be** | 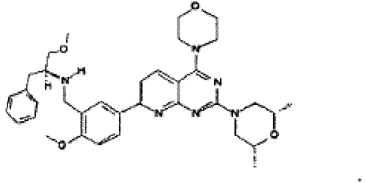 |
| **8bf** | 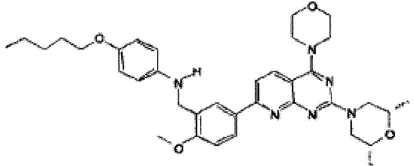 |
| **8bg** | 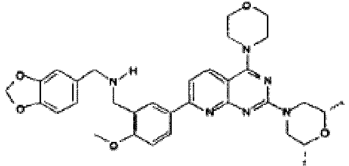 |
| | |
|---|---|
| **9a** | 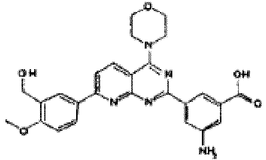 |
| **9b** | 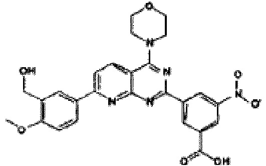 |
| **9c** | 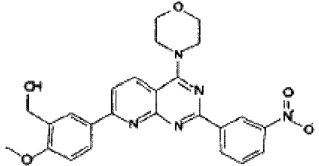 |
| **9d** | 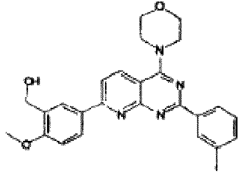 |
| **9e** | 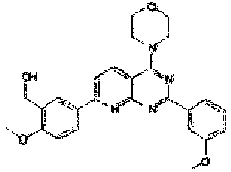 |
| **9f** | 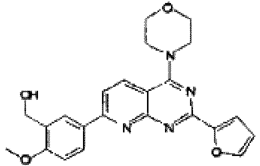 |
| **9g** | 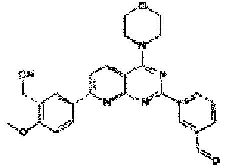 |
| **9h** | 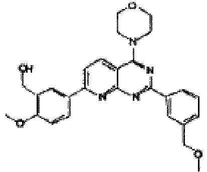 |
| **9i** | 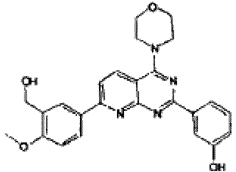 |
| **9j** | 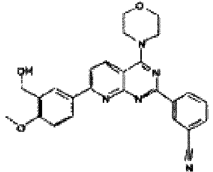 |
| **9k** | 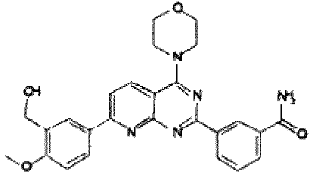 |
| **9l** | 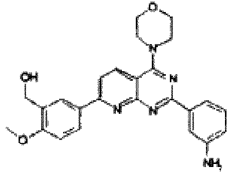 |
| **9m** | 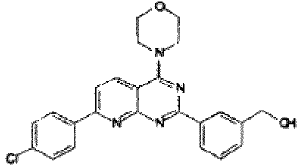 |
| **9n** | 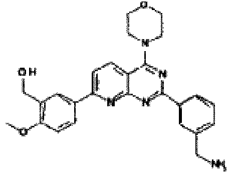 |
| **9o** | 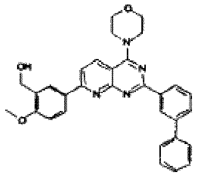 |
| **9p** | 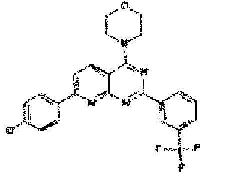 |
| **9q** | 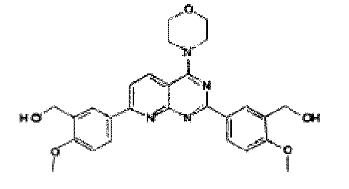 |
| **9r** | 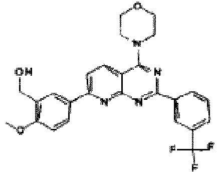 |
| **9s** | 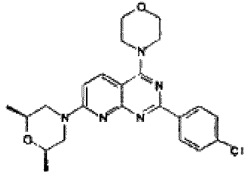 |
| **9t** | 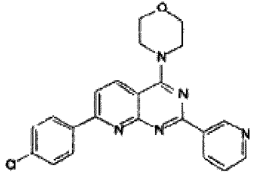 |
| **9u** | 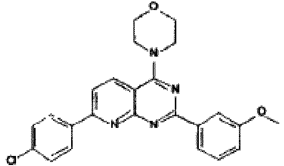 |
| **9v** | 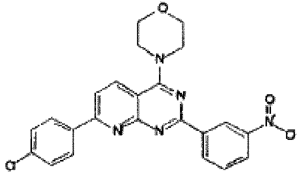 |
| **9w** | 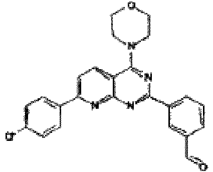 |
| **9x** | 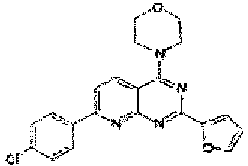 |
| **9y** | 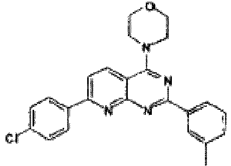 |
| **9z** | 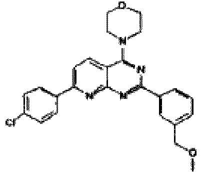 |
| **9aa** | 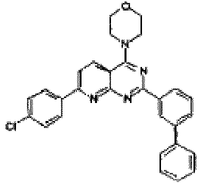 |
| **9ab** | 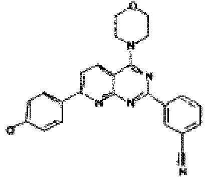 |
| **9ac** | 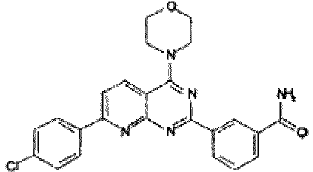 |
| **9ad** | 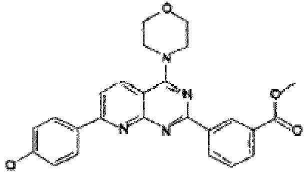 |
| **9ae** | 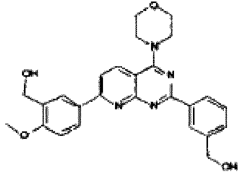 |
oder pharmazeutisch annehmbare Salze davon.

11. Pharmazeutische Zusammensetzung, umfassend eine Verbindung nach einem der Ansprüche 1 bis 10 oder ein pharmazeutisch annehmbares Salz davon und einen pharmazeutisch annehmbaren Träger oder ein pharmazeutisch annehmbares Verdünnungsmittel.

12. Verbindung nach einem der Ansprüche 1 bis 10 oder ein pharmazeutisch annehmbares Salz davon zur Verwendung als Arzneimittel.

13. Verwendung einer Verbindung nach einem der Ansprüche 1 bis 10 oder ein pharmazeutisch annehmbares Salz davon ohne die Maßgaben bei der Herstellung eines Arzneimittels zur Behandlung einer Erkrankung, die durch die Inhibierung von mTOR gebessert wird.

## Revendications

1. Composé de formule I : 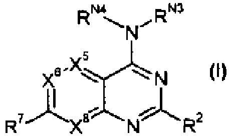 dans laquelle :
X⁸ est N, et X⁵ et X⁶ sont CH ;
R⁷ est un groupement C₅₋₂₀aryle éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino) ;
R^{N3} et R^{N4}, conjointement avec l'azote auquel ils sont liés, forment un cycle hétérocyclique contenant entre 3 et 8 atomes du cycle éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino) ;
R² est choisi parmi NR^{N5}R^{N6}, un groupement C₅₋₂₀hétéroaryle éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino), et un groupement C₅₋₂₀aryle éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino),
où R^{N5} et R^{N6} sont choisis indépendamment parmi H, un groupement C₁₋₇alkyle, un groupement C₅₋₂₀hétéroaryle, et un groupement C₅₋₂₀aryle, ou R^{N5} et R^{N6} conjointement avec l'azote auquel ils sont liés forment un cycle hétérocyclique contenant entre 3 et 8 atomes du cycle où chaque C₁₋₇alkyle, C₅₋₂₀hétéroaryle, C₅₋₂₀aryle ou cycle hétérocyclique est éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino),
ou un sel pharmaceutiquement acceptable de celui-ci,
et où
"C₅₋₂₀aryle", tel qu'utilisé dans la présente, désigne un motif monovalent obtenu par l'élimination d'un atome d'hydrogène d'un atome de cycle aromatique d'un composé aromatique en C₅₋₂₀, ledit composé ayant un cycle, ou deux cycles ou plus (par exemple condensés), et ayant de 5 à 20 atomes du cycle, et où au moins l'un desdits cycles est un cycle aromatique et où les atomes du cycle peuvent inclure un ou plusieurs hétéroatomes, y compris, mais sans s'y limiter, l'oxygène, l'azote et le soufre ; "alkyle", tel qu'utilisé dans la présente, désigne un motif monovalent obtenu par l'élimination d'un atome d'hydrogène d'un atome de carbone d'un composé hydrocarboné ayant de 1 à 20 atomes de carbone (sauf précisions contraires) qui peut être aliphatique ou alicyclique, et qui peut être saturé ou insaturé (par exemple partiellement insaturé, totalement insaturé) ;
"alcényle", tel qu'utilisé dans la présente, désigne un groupement alkyle ayant une ou plusieurs doubles liaisons carbone-carbone ; "alcynyle", tel qu'utilisé dans la présente, désigne un groupement alkyle ayant une ou plusieurs triples liaisons carbone-carbone ; "cycloalkyle", tel qu'utilisé dans la présente, désigne un groupement alkyle qui est également un groupement cyclyle ; c'est-à-dire un motif monovalent obtenu par l'élimination d'un atome d'hydrogène d'un atome de cycle alicyclique d'un cycle carbocyclique d'un composé carbocyclique, lequel cycle carbocyclique peut être saturé ou insaturé (par exemple partiellement insaturé, totalement insaturé), lequel motif a de 3 à 20 atomes de carbone (sauf précisions contraires), y compris de 3 à 20 atomes du cycle ;
"éther", tel qu'utilisé dans la présente, désigne un groupement -OR, dans lequel R est un groupement C₁₋₇alkyle, un groupement C₃₋₂₀hétérocyclyle ou un groupement C₅₋₂₀aryle ; "acyle", tel qu'utilisé dans la présente, désigne un groupement -C(=O)R, dans lequel R est H, un groupement C₁₋₇alkyle, un groupement C₃₋₂₀hétérocyclyle ou un groupement C₅₋₂₀aryle ; "ester", tel qu'utilisé dans la présente, désigne un groupement -C(=O)OR, dans lequel R est un groupement C₁₋₇alkyle, un groupement C₃₋₂₀hétérocyclyle ou un groupement C₅₋₂₀aryle ;
"amido", tel qu'utilisé dans la présente, désigne un groupement -C(=O)NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ;
"amino", tel qu'utilisé dans la présente, désigne un groupement -NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ;
"acylamido", tel qu'utilisé dans la présente, désigne un groupement -NR¹C(=O)R², dans lequel R¹ est hydrogène, un groupement C₁₋₇alkyle, un groupement C₃₋₂₀hétérocyclyle ou un groupement C₅₋₂₀aryle, R² est un groupement C₁₋₇alkyle, un groupement C₃₋₂₀hétérocyclyle ou un groupement C₅₋₂₀aryle, ou R¹ et R² peuvent, conjointement avec les atomes auxquels ils sont liés, former un groupement succinimidyle, maléimidyle et phtalimidyle ;
"uréido", tel qu'utilisé dans la présente, désigne un groupement -N(R¹)CONR²R³, dans lequel R² et R³ sont indépendamment hydrogène, un groupement C₁₋₇alkyle, un groupement C₃₋₂₀hétérocyclyle ou un groupement C₅₋₂₀aryle, ou R² et R³, pris conjointement avec l'atome d'azote auquel ils sont liés, forment un cycle hétérocyclique ayant de 4 à 8 atomes du cycle, et R¹ est hydrogène, un groupement C₁₋₇alkyle, un groupement C₃₋₂₀hétérocyclyle ou un groupement C₅₋₂₀aryle ;
"acyloxy", tel qu'utilisé dans la présente, désigne un groupement -OC(=O)R, dans lequel R est un groupement C₁₋₇alkyle, un groupement C₃₋₂₀hétérocyclyle ou un groupement C₅₋₂₀aryle ; "thioéther", tel qu'utilisé dans la présente, désigne un groupement -SR, dans lequel R est un groupement C₁₋₇alkyle, un groupement C₃₋₂₀hétérocyclyle ou un groupement C₅₋₂₀aryle ;
"sulfoxyde", tel qu'utilisé dans la présente, désigne un groupement -S(=O)R, dans lequel R est un groupement C₁₋₇alkyle, un groupement C₃₋₂₀hétérocyclyle ou un groupement C₅₋₂₀aryle ; "sulfonyle", tel qu'utilisé dans la présente, désigne un groupement -S(=O)₂R, dans lequel R est un groupement C₁₋₇alkyle, un groupement C₃₋₂₀hétérocyclyle ou un groupement C₅₋₂₀aryle ;
"thioamido", tel qu'utilisé dans la présente, désigne un groupement -C(=S)NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ;
"sulfonamino", tel qu'utilisé dans la présente, désigne un groupement -NR¹S(=O)₂R, dans lequel R¹ est hydrogène, un groupement C₁₋₇alkyle, un groupement C₃₋₂₀hétérocyclyle ou un groupement C₅₋₂₀aryle, et R est un groupement C₁₋₇alkyle, un groupement C₃₋₂₀hétérocyclyle ou un groupement C₅₋₂₀aryle ; et
à condition que lorsque R² est morpholino non substitué, R^{N3} et R^{N4} conjointement avec l'atome d'azote auquel ils sont liés forment un morpholino non substitué, R⁷ ne soit pas phényle non substitué, et
lorsque R² est pipéridinyle non substitué, R^{N3} et R^{N4} conjointement avec l'atome d'azote auquel ils sont liés forment un pipéridinyle non substitué, R⁷ ne soit pas phényle non substitué.

2. Composé selon la revendication 1, **caractérisé en ce que** R⁷ est un groupement phényle éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃-₇cycloalkyle, C₃₋₇cycloalcényle, C₃₋₂hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino), et **en ce que** les hétéroatomes du cycle de tout groupement C₅₋₂₀hétéroaryle ou C₃₋₂₀hétérocyclyle sont choisis parmi l'azote, l'oxygène et le soufre, et **en ce que**
"C₅₋₂₀aryle", tel qu'utilisé dans la présente, désigne un motif monovalent obtenu par l'élimination d'un atome d'hydrogène d'un atome de cycle aromatique d'un composé aromatique en C₅₋₂₀, ledit composé ayant un cycle, ou deux cycles ou plus (par exemple condensés), et ayant de 5 à 20 atomes du cycle, et où au moins l'un desdits cycles est un cycle aromatique et où les atomes du cycle peuvent inclure un ou plusieurs hétéroatomes, y compris, mais sans s'y limiter, l'oxygène, l'azote et le soufre ; "alkyle", tel qu'utilisé dans la présente, désigne un motif monovalent obtenu par l'élimination d'un atome d'hydrogène d'un atome de carbone d'un composé hydrocarboné ayant de 1 à 20 atomes de carbone (sauf précisions contraires) qui peut être aliphatique ou alicyclique, et qui peut être saturé ou insaturé (par exemple partiellement insaturé, totalement insaturé) ;
"alcényle", tel qu'utilisé dans la présente, désigne un groupement alkyle ayant une ou plusieurs doubles liaisons carbone-carbone ; "alcynyle", tel qu'utilisé dans la présente, désigne un groupement alkyle ayant une ou plusieurs triples liaisons carbone-carbone ; "cycloalkyle", tel qu'utilisé dans la présente, désigne un groupement alkyle qui est également un groupement cyclyle ; c'est-à-dire un motif monovalent obtenu par l'élimination d'un atome d'hydrogène d'un atome de cycle alicyclique d'un cycle carbocyclique d'un composé carbocyclique, lequel cycle carbocyclique peut être saturé ou insaturé (par exemple partiellement insaturé, totalement insaturé), lequel motif a de 3 à 20 atomes de carbone (sauf précisions contraires), y compris de 3 à 20 atomes du cycle ;
"éther", tel qu'utilisé dans la présente, désigne un groupement -OR, dans lequel R est un groupement C₁₋₇alkyle, un groupement C₃₋₂₀hétérocyclyle ou un groupement C₅₋₂₀aryle ; "acyle", tel qu'utilisé dans la présente, désigne un groupement -C(=O)R, dans lequel R est H, un groupement C₁₋₇alkyle, un groupement C₃₋₂₀hétérocyclyle ou un groupement C₅₋₂₀aryle ; "ester", tel qu'utilisé dans la présente, désigne un groupement -C(=O)OR, dans lequel R est un groupement C₁₋₇alkyle, un groupement C₃₋₂₀hétérocyclyle ou un groupement C₅₋₂₀aryle ;
"amido", tel qu'utilisé dans la présente, désigne un groupement -C(=O)NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ;
"amino", tel qu'utilisé dans la présente, désigne un groupement -NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ;
"acylamido", tel qu'utilisé dans la présente, désigne un groupement -NR¹C(=O)R², dans lequel R¹ est hydrogène, un groupement C₁₋₇alkyle, un groupement C₃₋₂₀hétérocyclyle ou un groupement C₅₋₂₀aryle, R² est un groupement C₁₋₇alkyle, un groupement C₃₋₂₀hétérocyclyle ou un groupement C₅₋₂₀aryle, ou R¹ et R² peuvent, conjointement avec les atomes auxquels ils sont liés, former un groupement succinimidyle, maléimidyle et phtalimidyle ;
"uréido", tel qu'utilisé dans la présente, désigne un groupement -N(R¹)CONR²R³, dans lequel R² et R³ sont indépendamment hydrogène, un groupement C₁₋₇alkyle, un groupement C₃₋₂₀hétérocyclyle ou un groupement C₅₋₂₀aryle, ou R² et R³, pris conjointement avec l'atome d'azote auquel ils sont liés, forment un cycle hétérocyclique ayant de 4 à 8 atomes du cycle, et R¹ est hydrogène, un groupement C₁₋₇alkyle, un groupement C₃₋₂₀hétérocyclyle ou un groupement C₅₋₂₀aryle ;
"acyloxy", tel qu'utilisé dans la présente, désigne un groupement -OC(=O)R, dans lequel R est un groupement C₁₋₇alkyle, un groupement C₃₋₂₀hétérocyclyle ou un groupement C₅₋₂₀aryle ; "thioéther", tel qu'utilisé dans la présente, désigne un groupement -SR, dans lequel R est un groupement C₁₋₇alkyle, un groupement C₃₋₂₀hétérocyclyle ou un groupement C₅₋₂₀aryle ;
"sulfoxyde", tel qu'utilisé dans la présente, désigne un groupement -S(=O)R, dans lequel R est un groupement C₁₋₇alkyle, un groupement C₃₋₂₀hétérocyclyle ou un groupement C₅₋₂₀aryle ;
"sulfonyle", tel qu'utilisé dans la présente, désigne un groupement -S(=O)₂R, dans lequel R est un groupement C₁₋₇alkyle, un groupement C₃₋₂₀hétérocyclyle ou un groupement C₅₋₂₀aryle ;
"thioamido", tel qu'utilisé dans la présente, désigne un groupement -C(=S)NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ; et
"sulfonamino", tel qu'utilisé dans la présente, désigne un groupement -NR¹S(=O)₂R, dans lequel R¹ est hydrogène, un groupement C₁₋₇alkyle, un groupement C₃₋₂₀hétérocyclyle ou un groupement C₅₋₂₀aryle, et R est un groupement C₁₋₇alkyle, un groupement C₃₋₂₀hétérocyclyle ou un groupement C₅₋₂₀aryle.

3. Composé selon la revendication 2, **caractérisé en ce que** R⁷ est un groupement phényle éventuellement substitué par un ou plusieurs groupements choisis parmi chloro, hydroxyle, méthyle, méthoxy, éthoxy, i-propoxy, benzyloxy et hydroxyméthyle.

4. Composé selon la revendication 2, **caractérisé en ce que** R⁷ est 4-chlorophényle, 4-méthylphényle, 4-méthoxyphényle, 3-hydroxyméthyl-4-méthoxyphényle, 3,5-diméthoxy-4-hydroxyphényle, 4-hydroxyphényle, 3-hydroxyphényle ou un groupement 3-hydroxyméthylphényle.

5. Composé selon la revendication 1, **caractérisé en ce que** le composé est un composé de formule I(A): 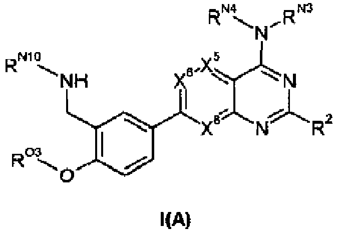 dans laquelle :
X⁸ est N, et X⁵ et X⁶ sont CH ;
R^{N3} et R^{N4}, conjointement avec l'azote auquel ils sont liés, forment un cycle hétérocyclique contenant entre 3 et 8 atomes du cycle éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino) ;
R² est choisi parmi NR^{N5}R^{N6}, un groupement C₅₋₂₀hétéroaryle éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino), et un groupement C₅₋₂₀aryle éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino),
où R^{N5} et R^{N6} sont choisis indépendamment parmi H, un groupement C₁₋₇alkyle, un groupement C₅₋₂₀₂₀hétéroaryle, et un groupement C₅₋₂₀aryle, ou R^{N5} et R^{N6} conjointement avec l'azote auquel ils sont liés forment un cycle hétérocyclique contenant entre 3 et 8 atomes du cycle où chaque C₁₋₇alkyle, C₅₋₂₀hétéroaryle, C₅₋₂₀aryle ou cycle hétérocyclique est éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino) ;
R^{O3} est choisi parmi hydrogène ou un groupement C₁₋₆alkyle, éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino) ; et
R^{N10} est choisi parmi C(=O)R^{C2}, C(=S)R^{C3}, SO₂R^{S3}, un groupement C₅₋₂₀hétéroaryle éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino), un groupement C₅₋₂₀aryle éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino), ou un groupement C₁₋₁₀alkyle éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino) où R^{C2} et R^{C3} sont choisis parmi H, un groupement C₅₋₂₀aryle, un groupement C₅₋₂₀hétéroaryle, un groupement C₁₋₇alkyle ou NR^{N11}R^{N12} où R^{N11} et R^{N12} sont choisis indépendamment parmi H, un groupement C₁₋₇alkyle, un groupement C₅₋₂₀hétéroaryle, un groupement C₅₋₂₀aryle ou R^{N11} et
R^{N12} Conjointement avec l'azote auquel ils sont liés forment un cycle hétérocyclique contenant entre 3 et 8 atomes du cycle, où chaque C₁₋₇alkyle, C₅₋₂₀hétéroaryle, C₅₋₂₀aryle ou cycle hétérocyclique est éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino) ; et R^{S3} est choisi parmi H, un groupement C₅₋₂₀aryle, un groupement C₅₋₂₀hétéroaryle, ou un groupement C₁₋₇alkyle où chaque C₁₋₇alkyle, C₅₋₂₀hétéroaryle ou C₅₋₂₀aryle est éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino),
ou un sel pharmaceutiquement acceptable de celui-ci,
et où
"C₅₋₂₀aryle", tel qu'utilisé dans la présente, désigne un motif monovalent obtenu par l'élimination d'un atome d'hydrogène d'un atome de cycle aromatique d'un composé aromatique en C₅₋₂₀, ledit composé ayant un cycle, ou deux cycles ou plus (par exemple condensés), et ayant de 5 à 20 atomes du cycle, et où au moins l'un desdits cycles est un cycle aromatique et où les atomes du cycle peuvent inclure un ou plusieurs hétéroatomes, y compris, mais sans s'y limiter, l'oxygène, l'azote et le soufre ;
"alkyle", tel qu'utilisé dans la présente, désigne un motif monovalent obtenu par l'élimination d'un atome d'hydrogène d'un atome de carbone d'un composé hydrocarboné ayant de 1 à 20 atomes de carbone (sauf précisions contraires) qui peut être aliphatique ou alicyclique, et qui peut être saturé ou insaturé (par exemple partiellement insaturé, totalement insaturé) ;
"alcényle", tel qu'utilisé dans la présente, désigne un groupement alkyle ayant une ou plusieurs doubles liaisons carbone-carbone ;
"alcynyle", tel qu'utilisé dans la présente, désigne un groupement alkyle ayant une ou plusieurs triples liaisons carbone-carbone ;
"cycloalkyle", tel qu'utilisé dans la présente, désigne un groupement alkyle qui est également un groupement cyclyle ; c'est-à-dire un motif monovalent obtenu par l'élimination d'un atome d'hydrogène d'un atome de cycle alicyclique d'un cycle carbocyclique d'un composé carbocyclique, lequel cycle carbocyclique peut être saturé ou insaturé (par exemple partiellement insaturé, totalement insaturé), lequel motif a de 3 à 20 atomes de carbone (sauf précisions contraires), y compris de 3 à 20 atomes du cycle ;
"éther", tel qu'utilisé dans la présente, désigne un groupement -OR, dans lequel R est un groupement C₁₋₇alkyle, un groupement C₃₋₂₀hétérocyclyle ou un groupement C₅₋₂₀aryle ;
"acyle", tel qu'utilisé dans la présente, désigne un groupement -C(=O)R, dans lequel R est H, un groupement C₁₋₇alkyle, un groupement C₃₋₂₀hétérocyclyle ou un groupement C₅₋₂₀aryle ;
"ester", tel qu'utilisé dans la présente, désigne un groupement -C(=O)OR, dans lequel R est un groupement C₁₋₇alkyle, un groupement C₃₋₂₀hétérocyclyle ou un groupement C₅₋₂₀aryle ;
"amido", tel qu'utilisé dans la présente, désigne un groupement -C(=O)NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ;
"amino", tel qu'utilisé dans la présente, désigne un groupement -NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ;
"acylamido", tel qu'utilisé dans la présente, désigne un groupement -NR¹C(=O)R², dans lequel R¹ est hydrogène, un groupement C₁₋₇alkyle, un groupement C₃₋₂₀hétérocyclyle ou un groupement C₅₋₂₀aryle, R² est un groupement C₁₋₇alkyle, un groupement C₃₋₂₀hétérocyclyle ou un groupement C₅₋₂₀aryle, ou R¹ et R² peuvent, conjointement avec les atomes auxquels ils sont liés, former un groupement succinimidyle, maléimidyle et phtalimidyle ;
"uréido", tel qu'utilisé dans la présente, désigne un groupement -N(R¹)CONR²R³, dans lequel R² et R³ sont indépendamment hydrogène, un groupement C₁₋₇alkyle, un groupement C₃₋₂₀hétérocyclyle ou un groupement C₅₋₂₀aryle, ou R² et R³, pris conjointement avec l'atome d'azote auquel ils sont liés, forment un cycle hétérocyclique ayant de 4 à 8 atomes du cycle, et R¹ est hydrogène, un groupement C₁₋₇alkyle, un groupement C₃₋₂₀hétérocyclyle ou un groupement C₅₋₂₀aryle ;
"acyloxy", tel qu'utilisé dans la présente, désigne un groupement -OC(=O)R, dans lequel R est un groupement C₁₋₇alkyle, un groupement C₃₋₂₀hétérocyclyle ou un groupement C₅₋₂₀aryle ;
"thioéther", tel qu'utilisé dans la présente, désigne un groupement -SR, dans lequel R est un groupement C₁₋₇alkyle, un groupement C₃₋₂₀hétérocyclyle ou un groupement C₅₋₂₀aryle ;
"sulfoxyde", tel qu'utilisé dans la présente, désigne un groupement -S(=O)R, dans lequel R est un groupement C₁₋₇alkyle, un groupement C₃₋₂₀hétérocyclyle ou un groupement C₅₋₂₀aryle ;
"sulfonyle", tel qu'utilisé dans la présente, désigne un groupement -S(=O)₂R, dans lequel R est un groupement C₁₋₇alkyle, un groupement C₃₋₂₀hétérocyclyle ou un groupement C₅₋₂₀aryle ;
"thioamido", tel qu'utilisé dans la présente, désigne un groupement -C(=S)NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ; et
"sulfonamino", tel qu'utilisé dans la présente, désigne un groupement -NR¹S(=O)₂R, dans lequel R¹ est hydrogène, un groupement C₁₋₇alkyle, un groupement C₃₋₂₀hétérocyclyle ou un groupement C₅₋₂₀aryle, et R est un groupement C₁₋₇alkyle, un groupement C₃₋₂₀hétérocyclyle ou un groupement C₅₋₂₀aryle.

6. Composé selon l'une quelconque des revendications 1 à 5, **caractérisé en ce que** R^{N3} et R^{N4} conjointement avec l'azote auquel ils sont liés forment un groupement morpholino.

7. Composé selon l'une quelconque des revendications 1 à 6, **caractérisé en ce que** R² est NR^{N5}R^{N6}, dans lequel R^{N5} et R^{N6} conjointement avec l'azote auquel ils sont liés forment un cycle hétérocyclique contenant entre 3 et 8 atomes du cycle, qui peut être éventuellement substitué par un ou plusieurs groupements choisis parmi halogéno, hydroxyle, nitro, cyano, carboxy, et thiol, ou C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino (chacun éventuellement substitué par un ou plusieurs groupements choisis parmi halogéno, hydroxyle, nitro, cyano, carboxy, thiol, C₁₋₇alkyle, C₂₋₇alcényle, C₂₋₇alcynyle, C₃₋₇cycloalkyle, C₃₋₇cycloalcényle, C₃₋₂₀hétérocyclyle, C₅₋₂₀aryle, C₅₋₂₀hétéroaryle, éther, acyle, ester, amido, amino, acylamido, uréido, acyloxy, thioéther, sulfoxyde, sulfonyle, thioamido et sulfonamino), et **en ce que** les hétéroatomes du cycle de tout groupement C₅₋₂₀hétéroaryle, C₃₋₂₀hétérocyclyle ou cycle hétérocyclique contenant entre 3 et 8 atomes du cycle sont choisis parmi l'azote, l'oxygène et le soufre, et
**en ce que**
"C₅₋₂₀aryle", tel qu'utilisé dans la présente, désigne un motif monovalent obtenu par l'élimination d'un atome d'hydrogène d'un atome de cycle aromatique d'un composé aromatique en C₅₋₂₀, ledit composé ayant un cycle, ou deux cycles ou plus (par exemple condensés), et ayant de 5 à 20 atomes du cycle, et où au moins l'un desdits cycles est un cycle aromatique et où les atomes du cycle peuvent inclure un ou plusieurs hétéroatomes, y compris, mais sans s'y limiter, l'oxygène, l'azote et le soufre ;
"alkyle", tel qu'utilisé dans la présente, désigne un motif monovalent obtenu par l'élimination d'un atome d'hydrogène d'un atome de carbone d'un composé hydrocarboné ayant de 1 à 20 atomes de carbone (sauf précisions contraires) qui peut être aliphatique ou alicyclique, et qui peut être saturé ou insaturé (par exemple partiellement insaturé, totalement insaturé) ;
"alcényle", tel qu'utilisé dans la présente, désigne un groupement alkyle ayant une ou plusieurs doubles liaisons carbone-carbone ; "alcynyle", tel qu'utilisé dans la présente, désigne un groupement alkyle ayant une ou plusieurs triples liaisons carbone-carbone ;
"cycloalkyle", tel qu'utilisé dans la présente, désigne un groupement alkyle qui est également un groupement cyclyle ; c'est-à-dire un motif monovalent obtenu par l'élimination d'un atome d'hydrogène d'un atome de cycle alicyclique d'un cycle carbocyclique d'un composé carbocyclique, lequel cycle carbocyclique peut être saturé ou insaturé (par exemple partiellement insaturé, totalement insaturé), lequel motif a de 3 à 20 atomes de carbone (sauf précisions contraires), y compris de 3 à 20 atomes du cycle ;
"éther", tel qu'utilisé dans la présente, désigne un groupement -OR, dans lequel R est un groupement C₁₋₇alkyle, un groupement C₃₋₂₀hétérocyclyle ou un groupement C₅₋₂₀aryle ;
"acyle", tel qu'utilisé dans la présente, désigne un groupement -C(=O)R, dans lequel R est H, un groupement C₁₋₇alkyle, un groupement C₃₋₂₀hétérocyclyle ou un groupement C₅₋₂₀aryle ; "ester", tel qu'utilisé dans la présente, désigne un groupement -C(=O)OR, dans lequel R est un groupement C₁₋₇alkyle, un groupement C₃₋₂₀hétérocyclyle ou un groupement C₅₋₂₀aryle ;
"amido", tel qu'utilisé dans la présente, désigne un groupement -C(=O)NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ;
"amino", tel qu'utilisé dans la présente, désigne un groupement -NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ;
"acylamido", tel qu'utilisé dans la présente, désigne un groupement -NR^{1C} (=O) R², dans lequel R¹ est hydrogène, un groupement C₁₋₇alkyle, un groupement C₃₋₂₀hétérocyclyle ou un groupement C₅₋₂₀aryle, R² est un groupement C₁₋₇alkyle, un groupement C₃₋₂₀hétérocyclyle ou un groupement C₅₋₂₀aryle, ou R¹ et R² peuvent, conjointement avec les atomes auxquels ils sont liés, former un groupement succinimidyle, maléimidyle et phtalimidyle ;
"uréido", tel qu'utilisé dans la présente, désigne un groupement -N(R¹)CONR²R³, dans lequel R² et R³ sont indépendamment hydrogène, un groupement C₁₋₇alkyle, un groupement C₃₋₂₀hétérocyclyle ou un groupement C₅₋₂₀aryle, ou R² et R³, pris conjointement avec l'atome d'azote auquel ils sont liés, forment un cycle hétérocyclique ayant de 4 à 8 atomes du cycle, et R¹ est hydrogène, un groupement C₁₋₇alkyle, un groupement C₃₋₂₀hétérocyclyle ou un groupement C₅₋₂₀aryle ;
"acyloxy", tel qu'utilisé dans la présente, désigne un groupement -OC(=O)R, dans lequel R est un groupement C₁₋₇alkyle, un groupement C₃₋₂₀hétérocyclyle ou un groupement C₅₋₂₀aryle ; "thioéther", tel qu'utilisé dans la présente, désigne un groupement -SR, dans lequel R est un groupement C₁₋₇alkyle, un groupement C₃₋₂₀hétérocyclyle ou un groupement C₅₋₂₀aryle ;
"sulfoxyde", tel qu'utilisé dans la présente, désigne un groupement -S(=O)R, dans lequel R est un groupement C₁₋₇alkyle, un groupement C₃₋₂₀hétérocyclyle ou un groupement C₅₋₂₀aryle ;
"sulfonyle", tel qu'utilisé dans la présente, désigne un groupement -S(=O)₂R, dans lequel R est un groupement C₁₋₇alkyle, un groupement C₃₋₂₀hétérocyclyle ou un groupement C₅₋₂₀aryle ;
"thioamido", tel qu'utilisé dans la présente, désigne un groupement -C(=S)NR¹R², dans lequel R¹ et R² sont indépendamment hydrogène, un groupement C₁₋₇alkyle, un groupement C₃₋₂₀hétérocyclyle ou un groupement C₅₋₂₀aryle, ou R¹ et R², pris ensemble avec l'atome d'azote auquel ils sont liés, forment un cycle hétérocyclique ayant de 4 à 8 atomes du cycle ; et "sulfonamino", tel qu'utilisé dans la présente, désigne un groupement -NR¹S(=O)₂R, dans lequel R¹ est hydrogène, un groupement C₁₋₇alkyle, un groupement C₃₋₂₀hétérocyclyle ou un groupement C₅₋₂₀aryle, et R est un groupement C₁₋₇alkyle, un groupement C₃₋₂₀hétérocyclyle ou un groupement C₅₋₂₀aryle.

8. Composé selon la revendication 7, **caractérisé en ce que** R² est choisi parmi morpholino, thiomorpholino, pipéridinyle, pipérazinyle, homopipérazinyle et pyrrolidinyle, chaque morpholino, thiomorpholino, pipéridinyle, pipérazinyle, homopipérazinyle et pyrrolidinyle pouvant être éventuellement substitué sur le carbone par un ou plusieurs groupements méthyle.

9. Composé selon la revendication 8, **caractérisé en ce que** R² est

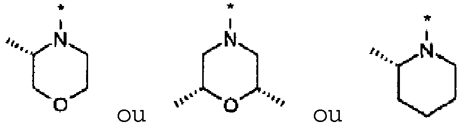

10. Composé selon la revendication 1, choisi parmi l'un quelconque des exemples suivants
| | **R⁷** | **NR³R⁴** | **R²** |
|---|---|---|---|
| **1a** | 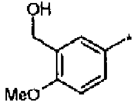 | 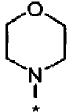 | 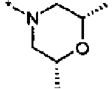 |
| **1b** | 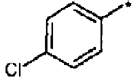 | 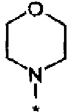 | 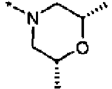 |
| **1c** | 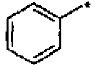 | 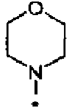 | 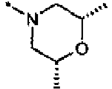 |
| **1d** | 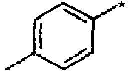 | 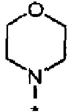 | 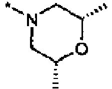 |
| **1e** | 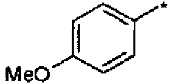 | 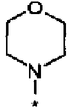 | 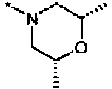 |
| **1f** | 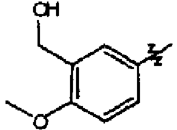 | 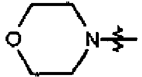 | 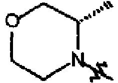 |
| **1g** | 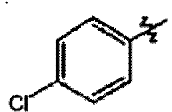 | 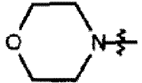 | 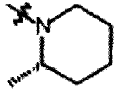 |
| **1h** | 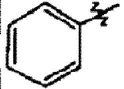 | 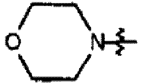 | 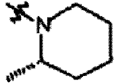 |
| **1**i | 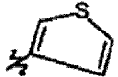 | 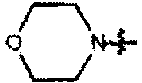 | 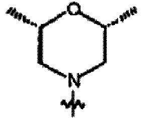 |
| **1j** | 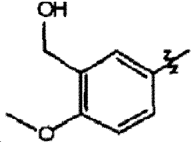 | 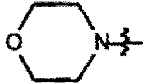 | 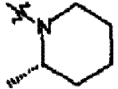 |
| **1k** | 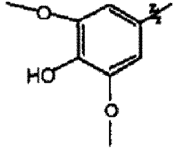 | 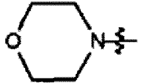 | 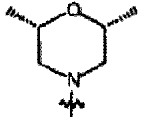 |
| **1l** | 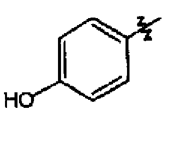 | 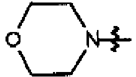 | 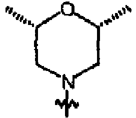 |
| **1m** | 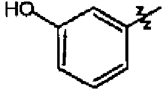 | 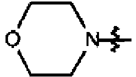 | 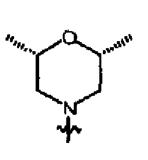 |
| **1n** | 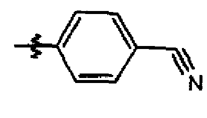 | 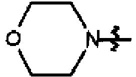 | 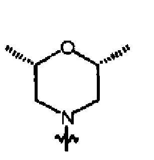 |
| **1o** | 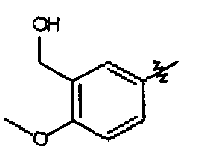 | 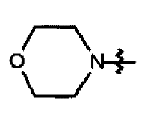 | 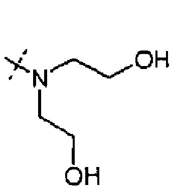 |
| **1p** | 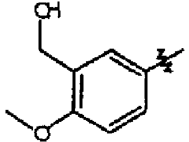 | 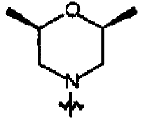 | 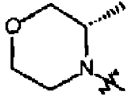 |
| **1q** | 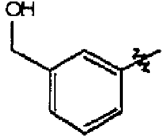 | 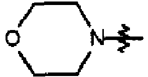 | 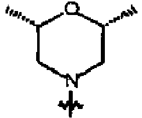 |
| **1r** | 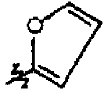 | 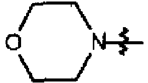 | 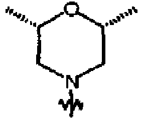 |
| **1s** | 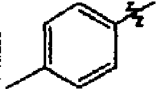 | 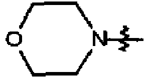 | 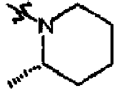 |
| **1t** | 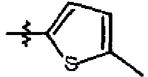 | 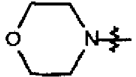 | 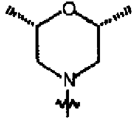 |
| **1u** | 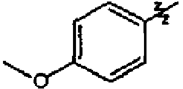 | 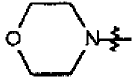 | 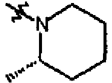 |
| **1v** | 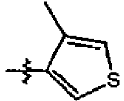 | 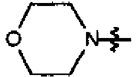 | 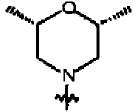 |
| **1w** | 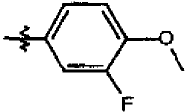 | 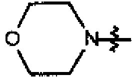 | 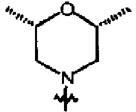 |
| **1x** | 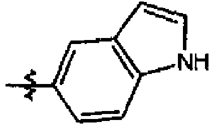 | 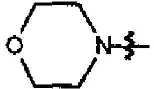 | 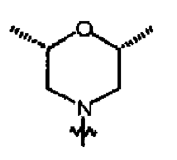 |
| **1y** | 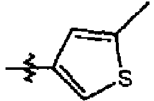 | 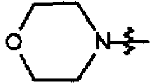 | 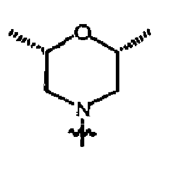 |
| **1z** | 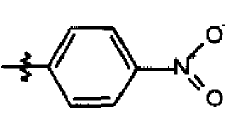 | 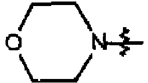 | 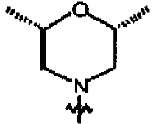 |
| **1aa** | 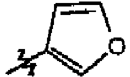 | 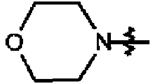 | 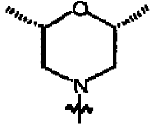 |
| **1ab** | 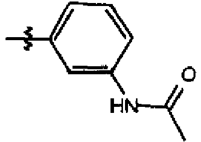 | 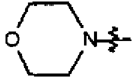 | 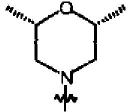 |
| **1ac** | 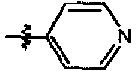 | 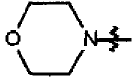 | 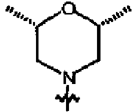 |
| **1ad** | 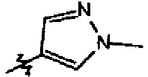 | 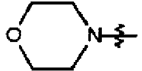 | 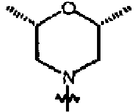 |
| **1ae** | 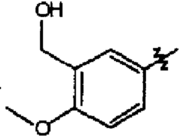 | 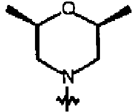 | 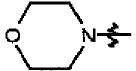 |
| **1af** | 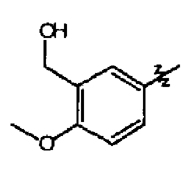 | 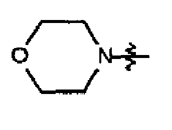 | 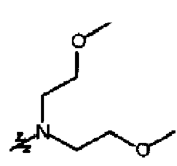 |
| **1ag** | 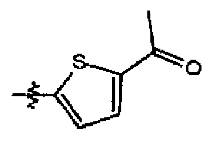 | 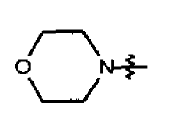 | 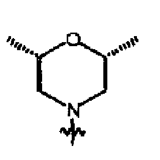 |
| **1ah** | 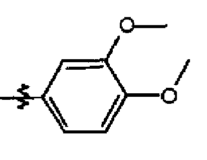 | 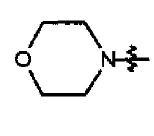 | 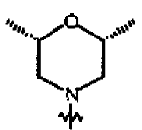 |
| **1ai** | 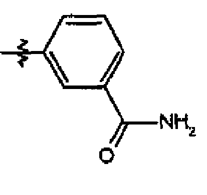 | 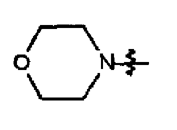 | 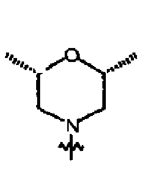 |
| **1aj** | 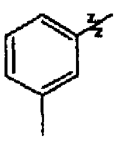 | 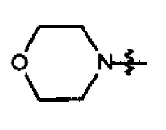 | 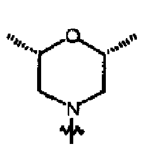 |
| **1ak** | 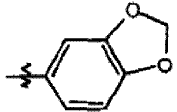 | 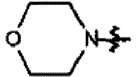 | 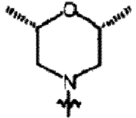 |
| **1al** | 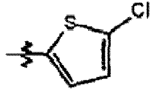 | 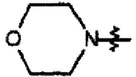 | 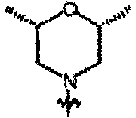 |
| **1am** | 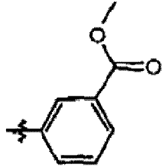 | 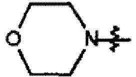 | 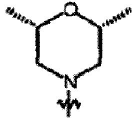 |
| **1an** | 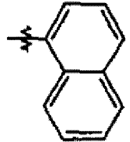 | 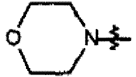 | 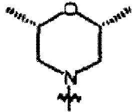 |
| **1ao** | 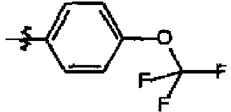 | 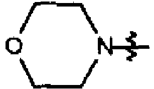 | 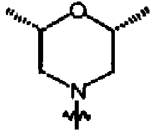 |
| **1ap** | 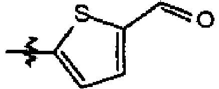 | 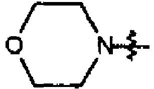 | 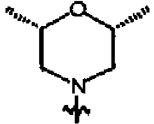 |
| **1aq** | 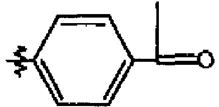 | 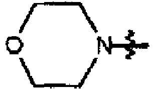 | 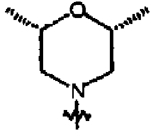 |
| **1ar** | 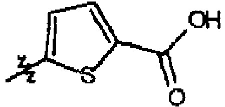 | 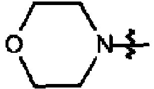 | 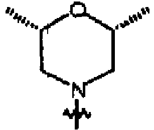 |
| **1as** | 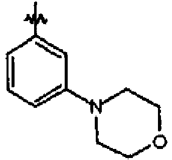 | 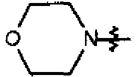 | 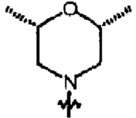 |
| **1at** | 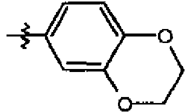 | 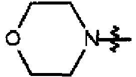 | 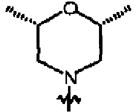 |
| **1au** | 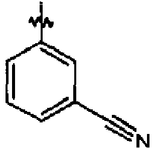 | 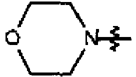 | 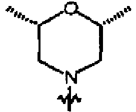 |
| **1av** | 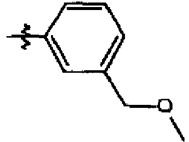 | 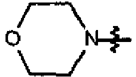 | 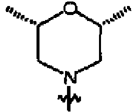 |
| **1aw** | 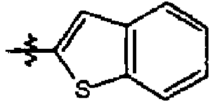 | 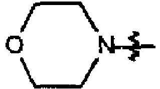 | 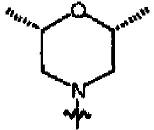 |
| **1a** | 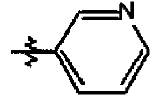 | 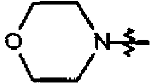 | 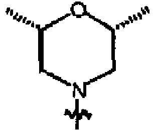 |
| **1ay** | 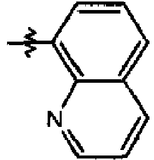 | 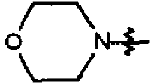 | 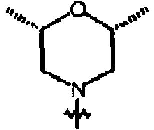 |
| **1az** | 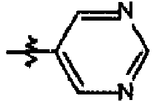 | 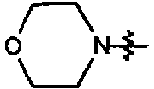 | 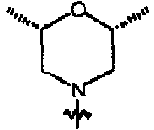 |
| **1ba** | 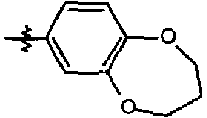 | 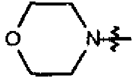 | 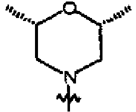 |
| **1bb** | 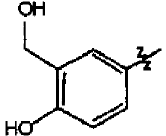 | 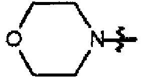 | 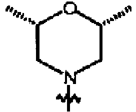 |
| **1bc** | 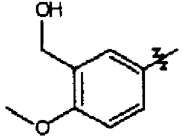 | 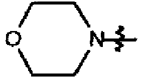 | 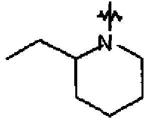 |
| **1bd** | 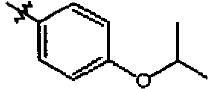 | 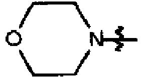 | 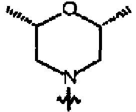 |
| **1be** | 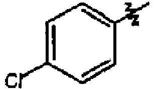 | 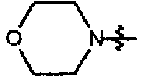 | 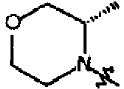 |
| **1bf** | 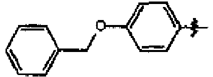 | 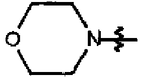 | 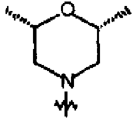 |
| **1bg** | 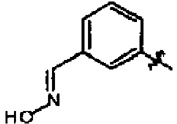 | 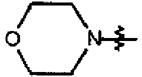 | 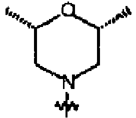 |
| **1bh** | 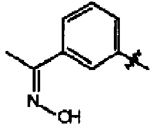 | 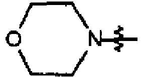 | 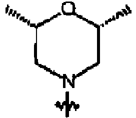 |
| **1bi** | 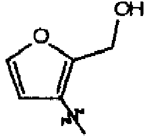 | 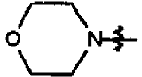 | 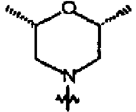 |
| **1bk** | 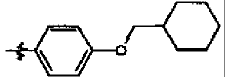 | 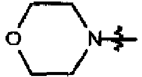 | 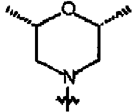 |
| **1bl** | 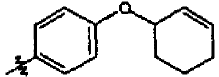 | 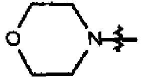 | 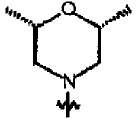 |
| **1bm** | 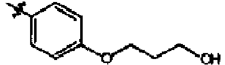 | 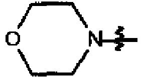 | 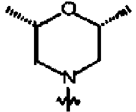 |
| **1bn** | 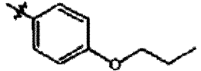 | 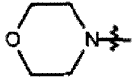 | 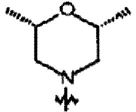 |
| **1bo** | 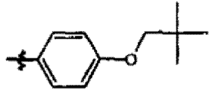 | 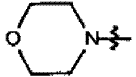 | 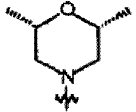 |
| **1bp** | 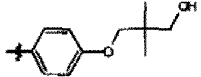 | 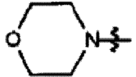 | 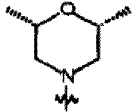 |
| **1bq** | 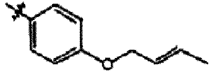 | 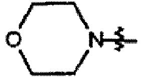 | 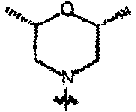 |
| **1br** | 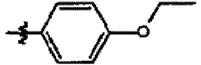 | 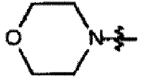 | 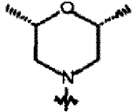 |
| **1bs** | 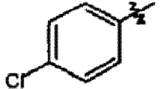 | 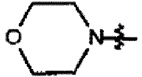 | 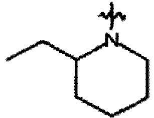 |
| **1bt** | 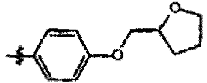 | 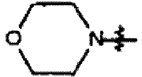 | 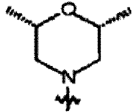 |
| **1bu** | 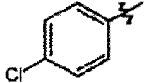 | 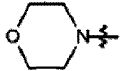 | 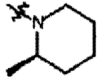 |
| **1bv** | 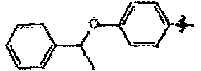 | 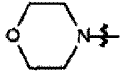 | 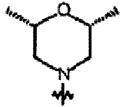 |
| **1bw** 1 | 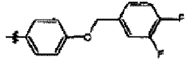 | 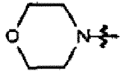 | 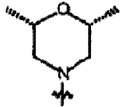 |
| **1bx** | 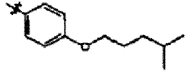 | 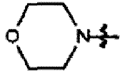 | 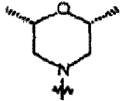 |
| | |
|---|---|
| **8a** | 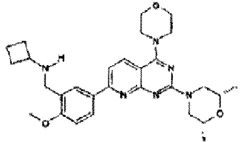 |
| **8b** | 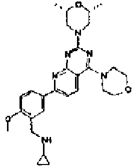 |
| **8c** | 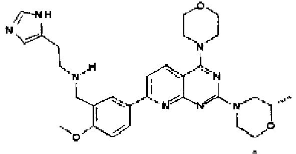 |
| **8d** | 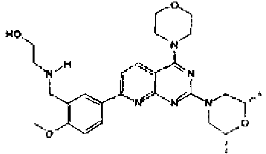 |
| **8e** | 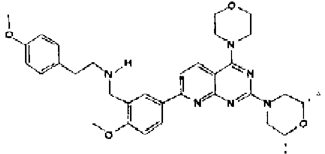 |
| **8f** | 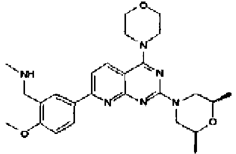 |
| **8g** | 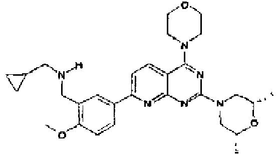 |
| **8h** | 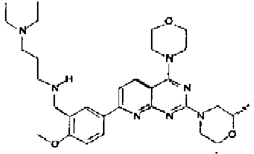 |
| **8i** | 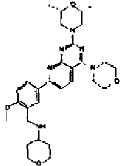 |
| **8j** | 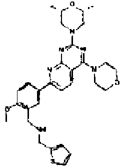 |
| **8k** | 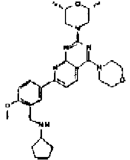 |
| **8l** | 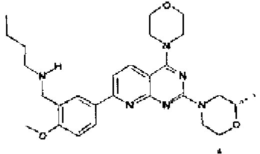 |
| **8m** | 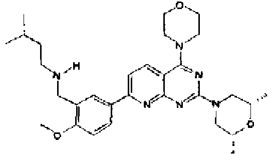 |
| **8n** | 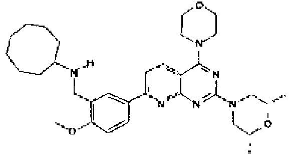 |
| **8o** | 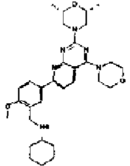 |
| **8p** | 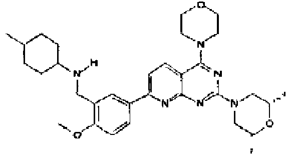 |
| **8q** | 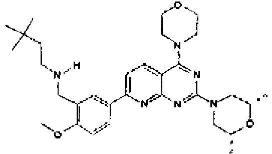 |
| **8r** | 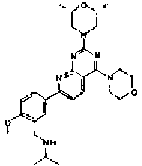 |
| **8s** | 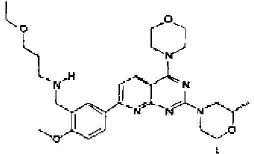 |
| **8t** | 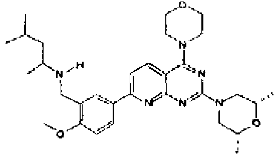 |
| **8u** | 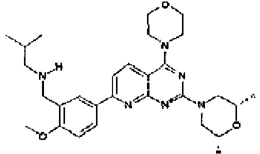 |
| **8v** | 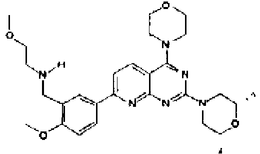 |
| **8w** | 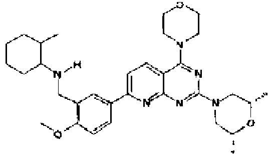 |
| **8x** | 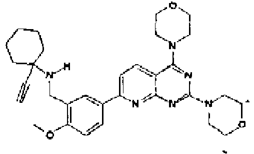 |
| **8y** | 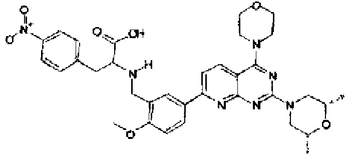 |
| **8z** | 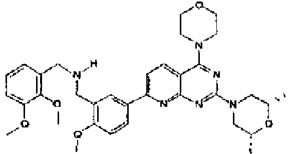 |
| **8aa** | 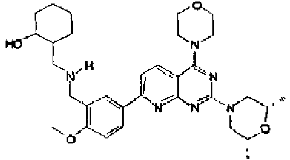 |
| **8ab** | 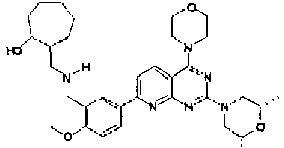 |
| **8ac** | 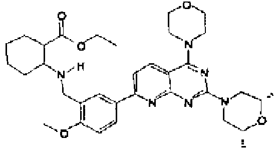 |
| **8ad** | 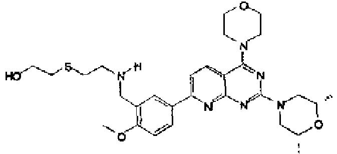 |
| **8ae** | 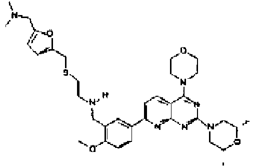 |
| **8af** | 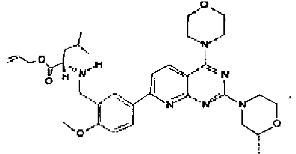 |
| **8ag** | 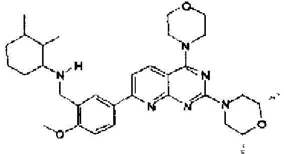 |
| **8ah** | 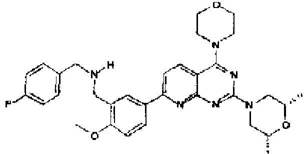 |
| **8ai** | 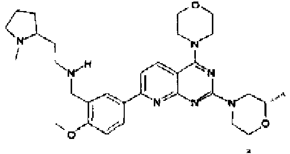 |
| **8aj** | 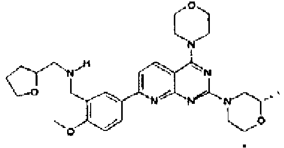 |
| **8ak** | 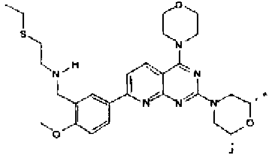 |
| **8al** | 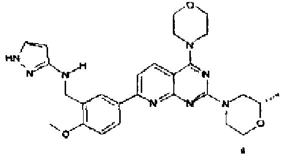 |
| **8am** | 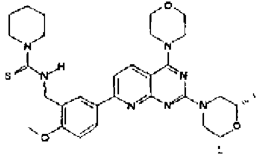 |
| **8an** | 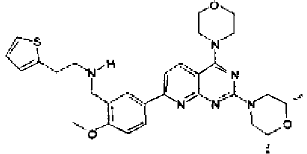 |
| **8ao** | 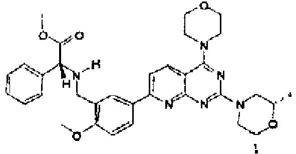 |
| **8ap** | 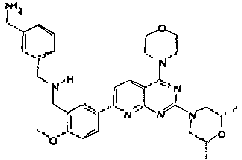 |
| **8aq** | 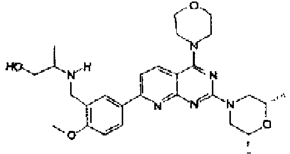 |
| **8ar** | 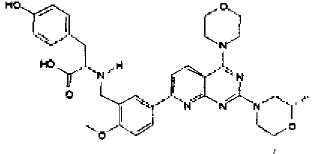 |
| **8as** | 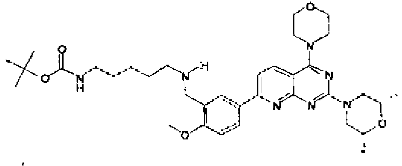 |
| **8at** | 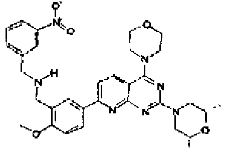 |
| **8au** | 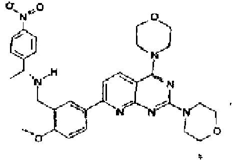 |
| **8av** | 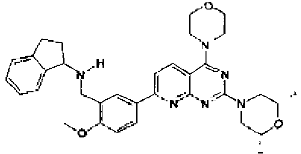 |
| **8aw** | 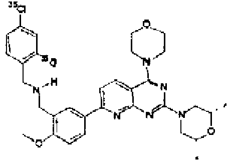 |
| **8ax** | 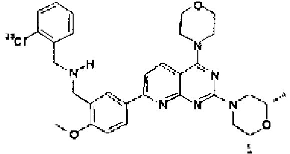 |
| **8ay** | 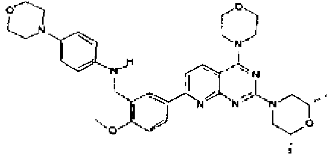 |
| **8az** | 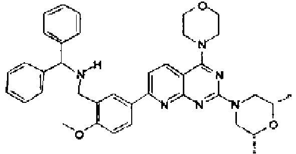 |
| **8ba** | 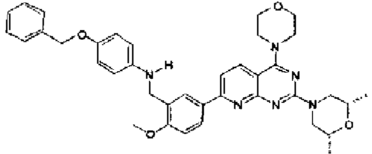 |
| **8bb** | 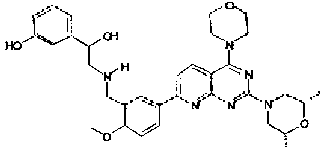 |
| **8bc** | 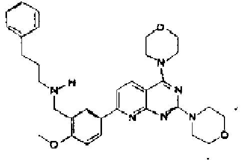 |
| **8bd** | 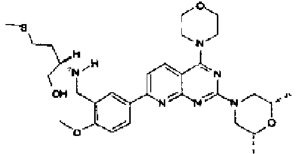 |
| **8be** | 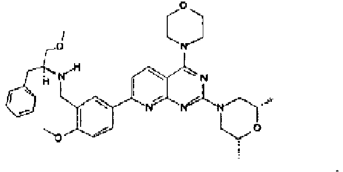 |
| **8bf** | 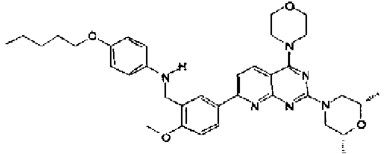 |
| **8bg** | 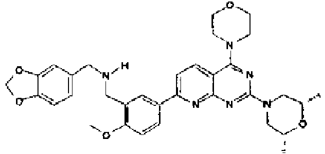 |
| | |
|---|---|
| **9a** | 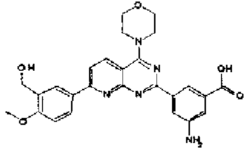 |
| **9b** | 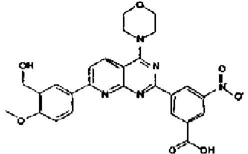 |
| **9c** | 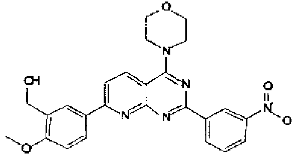 |
| **9d** | 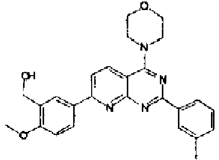 |
| **9e** | 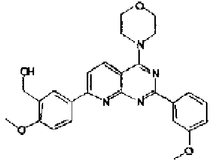 |
| **9f** | 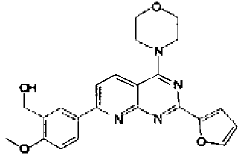 |
| **9g** | 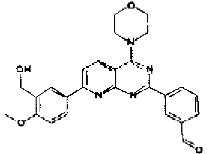 |
| **9h** | 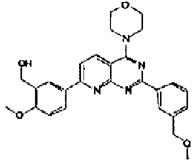 |
| **9i** | 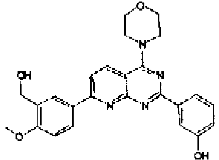 |
| **9j** | 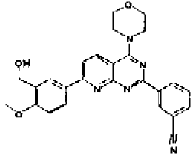 |
| **9k** | 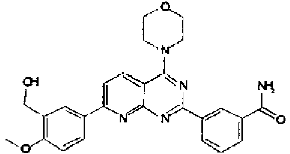 |
| **9l** | 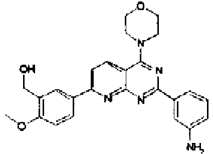 |
| **9m** | 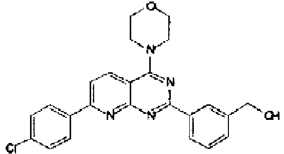 |
| **9n** | 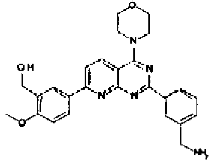 |
| **9o** | 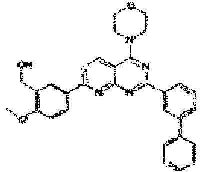 |
| **9p** | 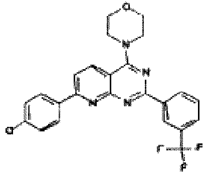 |
| **9q** | 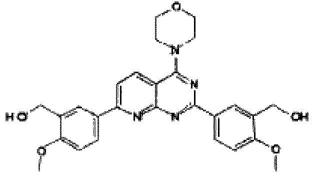 |
| **9r** | 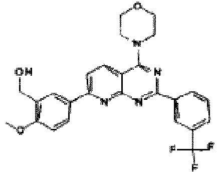 |
| **9s** | 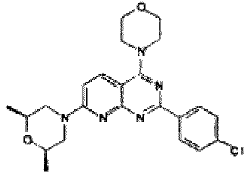 |
| **9t** | 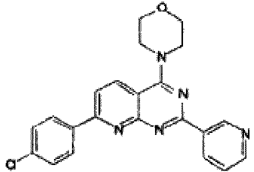 |
| **9u** | 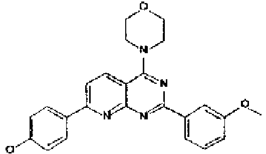 |
| **9v** | 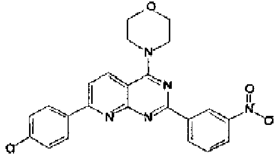 |
| **9w** | 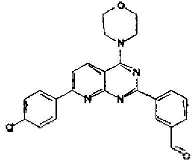 |
| **9x** | 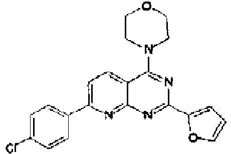 |
| **9y** | 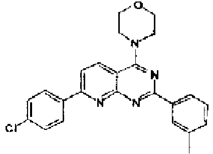 |
| **9z** | 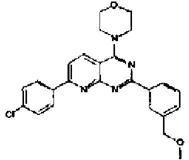 |
| **9aa** | 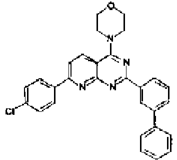 |
| **9ab** | 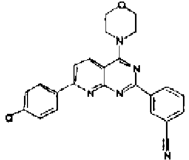 |
| **9ac** | 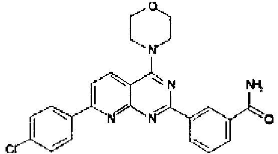 |
| **9ad** | 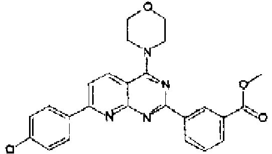 |
| **9ae** | 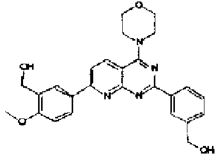 |
, ou les sels pharmaceutiquement acceptables de ceux-ci.

11. Composition pharmaceutique comprenant un composé selon l'une quelconque des revendications 1 à 10, ou un sel pharmaceutiquement acceptable de celui-ci, et un support ou diluant pharmaceutiquement acceptable.

12. Composé selon l'une quelconque des revendications 1 à 10, ou un sel pharmaceutiquement acceptable de celui-ci, destiné à être utilisé comme médicament.

13. Utilisation d'un composé selon l'une quelconque des revendications 1 à 10, ou un sel pharmaceutiquement acceptable de celui-ci, sans les conditions dans la préparation d'un médicament destiné au traitement d'une maladie améliorée par l'inhibition de mTOR.
